(19) Europäisches Patentamt
European Patent Office
Office européen des brevets

(11) EP 1 647 580 B1

# (12) FASCICULE DE BREVET EUROPEEN

(45) Date de publication et mention de la délivrance du brevet:
**16.02.2011 Bulletin 2011/07**

(51) Int Cl.:
*C09B 35/32 (2006.01)* *C07C 321/16 (2006.01)*
*C07D 251/50 (2006.01)* *C07D 251/52 (2006.01)*

(21) Numéro de dépôt: **05292159.0**

(22) Date de dépôt: **13.10.2005**

(54) **Composition de teinture comprenant un colorant disulfure particulier et procédé de coloration des fibres kératiniques humaines à partir de ce colorant**

Färbezusammensetzung enthaltend einen Disulfid Farbstoff und Verfahren zur färben von keratinfasern mit dieser Färbezusammensetzung

Dying composition comprising a particular disulfide dye and process of dying keratinous fibres with this dye.

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **14.10.2004 FR 0410864**

(43) Date de publication de la demande:
**19.04.2006 Bulletin 2006/16**

(73) Titulaire: **L'Oréal**
**75008 Paris (FR)**

(72) Inventeurs:
- **Daubresse, Nicolas**
  **78170 La Celles St Cloud (FR)**
- **Genain, Gilles**
  **75116 Paris (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 0 271 322** **FR-A- 1 156 407**
**FR-A- 2 822 696** **FR-A- 2 825 624**

- **LEWIS D M ET AL: "THE ROLE OF VINYLSULPHONYL REACTIVE DYES IN PREVENTION OF WOOL DAMAGE" 1 octobre 1991 (1991-10-01), JOURNAL OF THE SOCIETY OF DYERS AND COLOURISTS, SOCIETY OF DYERS AND COLOURISTS. BRADFORD, GB, PAGE(S) 357-362 , XP000244313 ISSN: 0037-9859 * le document en entier ***

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 647 580 B1

Printed by Jouve, 75001 PARIS (FR)

## Description

L'invention a pour objet une composition de teinture comprenant un colorant disulfure particulier ainsi qu'un procédé de coloration des fibres kératiniques en particulier humaines, et notamment les cheveux à partir de cette composition. L'invention a aussi pour objet des colorants disulfure nouveaux.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Il est aussi connu d'obtenir des colorations permanentes avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

Ce procédé de coloration d'oxydation consiste à appliquer sur les fibres kératiniques, des bases ou un mélange de bases et de coupleurs avec de l'eau oxygénée à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements.

Les systèmes de coloration d'oxydation permettent d'obtenir des colorations de fond relativement tenaces au shampooing mais ne permettent pas d'obtenir des nuances chromatiques.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres capillaires. Sont ainsi décrits certains colorants portant des fonctions sels de Bunte et isothiuroniums, ou d'autres groupements protecteurs de thiols. Cependant, l'obtention de la forme réactive du colorant nécessite en général l'utilisation de milieux fortement basiques. De plus, les fonctions thiols sont généralement générées en excès ce qui rend nécessaire une étape de post neutralisation à la suite de la coloration.

D'autres colorants disulfure connus pour la coloration des fibres kératiniques sont des dérivés disulfure de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un prétraitement réducteur du cheveu. Cependant, ces colorants peuvent occasionner des virages de couleur lors de l'application.

Par ailleurs, il est connu dans l'article « The role of the vinylsulphonyl reactive dyes in prevention of wool damage », J. Soc. Dyers Colourists, vol. 107, octobre 1991, p. 357-362 de colorer la laine à chaud (100°C) par un colorant disulfure, le groupement disulfure servant à générer un groupement vinylsulphonyle, afin de réduire la dégradation des fibres de laine.

Le but de la présente invention est de fournir de nouveaux systèmes de coloration des fibres kératiniques, notamment humaines, en particulier les cheveux, qui ne présentent pas les inconvénients des colorants directs existants. En particulier, un des buts de la présente invention est de fournir des systèmes de coloration directe permettant d'obtenir des colorations chromatiques, très tenaces, notamment face à des shampooings successifs.

Ce but est atteint avec la présente invention qui a pour objet un procédé de coloration des fibres kératiniques humaines consistant à appliquer sur les fibres, une
composition tinctoriale, comprenant dans un milieu cosmétique approprié, au moins un colorant disulfure choisi parmi les colorants de formules (I), (II), (III) ou (IV) suivantes :

$$A-(X)_p-C_{sat}-S\text{-}S-C_{sat}-(X)_p-A \qquad (I)$$

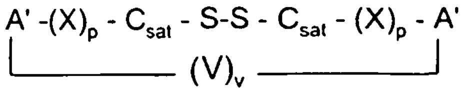

(II)

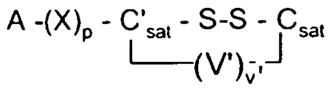

(III)

$$A-(X)_p-C_{sat}-S\text{-}S-C_{sat}-(X)_p-D \qquad (IV)$$

leurs sels, isomères solvates tels que hydrates, formules dans lesquelles :

- A, et A', identiques ou différents, représentent un radical contenant au moins un chromophore cationique ou non;
- V et V', identiques ou différents représentent un groupe pontant
- v et v', identiques ou différents représentent 0 ou 1 ;
- X, identiques ou différents, représentent une chaîne hydrocarbonée en $C_1$-$C_{30}$, linéaire ou ramifiée, saturée ou insaturée, éventuellement interrompue et/ ou éventuellement terminée à l'une ou deux de ses extrémités par un ou plusieurs groupes divalents ou leur combinaisons choisis parmi :

  - -N(R)-, $-N^+(R)(R)-$, -O-, -S-, -CO-, $-SO_2-$ avec R, identiques ou différents, choisi parmi un hydrogène, un radical alkyle en C1-C4, hydroxyalkyle, aminoalkyle
  - un radical (hétéro)cyclique aromatique ou non, saturé ou insaturé, condensé ou non comprenant éventuellement un ou plusieurs hétéroatomes identiques ou non, éventuellement substitué ;

- le coefficient p est égal à 0 ou 1 ;
- $C_{sat}$, $C'_{sat}$, identiques ou différents représentent une chaîne alkylène en $C_1$-$C_{18}$, linéaire ou ramifiée, éventuellement substituée, éventuellement cyclique ;
- D correspond à un radical choisi parmi les radicaux hydroxy, hydroxyalkyle, alcoxy, carboxyliques, carboxylates, amino, alkylamino, dialkylamino.

Le procédé de l'invention permet d'accéder à des couleurs chromatiques ou à des couleurs de fond très tenaces aux shampooings, aux agressions courantes (soleil, transpiration), et aux autres traitements capillaires.

L'invention a aussi pour objet une composition tinctoriale comprenant, dans un milieu cosmétique approprié pour la teinture des fibres kératiniques notamment humaines, au moins un colorant disulfure choisi parmi les colorants de formules (I), (II), (III) ou (IV).

Un autre objet de l'invention est l'utilisation d'au moins un colorant disulfure de formule (I), (II), (III) ou (IV), pour la teinture des fibres kératiniques, notamment humaines, en particulier les cheveux.

L'invention a enfin pour objet les colorants disulfure de formules (I), (II) (III) ou (IV), en tant que tels, leurs sels, leurs isomères et leurs solvates.

Au sens de la présente invention, et à moins qu'une indication différente ne soit donnée :

- les radicaux aryles ou hétéroaryles ou la partie aryle ou hétéroaryle d'un radical peuvent être substitués par au moins un substituant porté par un atome de carbone, choisi parmi :

  - un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
  - un atome d'halogène tel que chlore, fluor ou brome ;
  - un groupement hydroxyle ;

- un radical alcoxy en $C_1$-$C_2$ un radical (poly)-hydroxyalcoxy en $C_2$-$C_4$ ;
- un radical amino ; un radical amino substitué par un ou deux radicaux alkyle, identiques ou différents, en $C_1$-$C_4$ éventuellement porteurs d'au moins un groupement hydroxyle ou amino, par deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- un radical acylamino (-NR-COR') dans lequel le radical R est un atome d'hydrogène, un radical alkyle en $C_1$-$C_4$ éventuellement porteur d'au moins un groupement hydroxyle et le radical R' est un radical alkyle en $C_1$-$C_2$ un radical carbamoyle ($(R)_2N$-CO-) dans lequel les radicaux R, identiques ou non, représentent un atome d'hydrogène, un radical alkyle en $C_1$-$C_4$ éventuellement porteur d'au moins

un groupement hydroxyle ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxyle et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxyle,

- un radical carboxylique sous forme acide ou salifiée (de préférence avec un métal alcalin ou un ammonium, substitué ou non) ;
- un radical nitro ;
- un groupement nitrile (CN);
- un groupement trifluorométhyle($CF_3$) ;

  - la partie cyclique ou hétérocyclique d'un radical non aromatique peut être substituée par au moins un substituant porté par un atome de carbone choisi parmi les groupements :

- hydroxyle,
- alcoxy en $C_1$-$C_4$, (poly)hydroxyalcoxy en $C_2$-$C_4$,
- alkylcarbonylamino ((RCO-NR'-) dans lequel le radical R' est un atome d'hydrogène, un radical alkyle en $C_1$-$C_4$ éventuellement porteur d'au moins un groupement hydroxyle et le radical R est un radical alkyle en $C_1$-$C_2$, amino substitué par deux groupements alkyle identiques ou différents en $C_1$-$C_4$ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;

  - une chaîne hydrocarbonée est insaturée lorsqu'elle comporte une ou plusieurs liaisons doubles et/ou une ou plusieurs liaisons triples ;
  - un radical hétéroaromatique ou hétéroaryle, correspond à un radical aromatique dans lequel au moins l'un des atomes de carbone est remplacé par un hétéroatome, choisi parmi l'azote, l'oxygène ou le soufre.

De plus, sauf indication contraire, les bornes délimitant l'étendue d'une plage de valeurs sont comprises dans cette plage de valeurs.

Selon la présente invention, on entend par « chromophore » un radical issu d'un colorant, c'est-à-dire un radical issu d'une molécule absorbant dans le domaine visible du rayonnement (entre 400 et 800 nm).

Les radicaux A et A' des formules (I), (II), (III) et (IV) peuvent contenir un ou plusieurs chromophores, identiques ou différents.

Au sens de la présente invention, les chromophores sont dits différents lorsqu'ils diffèrent par leur structure chimique. De tels chromophores peuvent être des chromophores issus de familles différentes ou d'une même famille à la condition de présenter des structures chimiques différentes. Par exemple, les chromophores peuvent être choisis dans la famille des colorants azoïques mais différer par la structure chimique des radicaux le constituant ou par la position respective de ces radicaux.

A titre de chromophores utiles dans la présente invention, on peut citer les radicaux issus des colorants acridines, acridones, anthranthrones, anthrapyrimidines, anthraquinones, azines, azoïques, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (comme les azacarbocyanines, diazacarbocyanines, diazahémicyanines, hémicyanines, tétraazacarbocyanines), diazines, dicétopyrrolopyrroles, dioxazines, diphénylamines, diphénylméthanes, dithiazines, flavonoïdes tels que flavanthrones et flavones, fluorindines, formazans, hydrazones, en particulier arylhydrazones, hydroxycétones, indamines, indanthrones, indigoides et pseudo-indigoïdes, indophénols, indoanilines, isoindolines, isoindolinones, isoviolanthrones, lactones, méthines, naphthalimides, naphthanilides, naphtholactames, naphthoquinones, nitro, notamment les nitro(hétéro)aromatiques, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.

Parmi les chromophores nitro utilisables selon l'invention, on peut citer de manière non limitative les radicaux issus des colorants suivants:

- 1,4-diamino-2-nitrobenzène
- 1-amino-2 nitro,-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-βγ-dihydroxypropyloxy-3-méthylamino-4-nitrobenzène
- 1-β-hydroxyéthylamino-4-β,γ-dihydroxypropyloxy-2-nitrobenzène
- 1-βγ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-p-hydroxyéthylamino-3-nitrobenzène.

Parmi les chromophores azoïques utilisables selon l'invention, on peut citer les radicaux issus des colorants azoïques cationiques décrits dans les demandes de brevets WO 95/15144, WO 95/01772 et EP-714954.

On peut également citer parmi les chromophores azoïques ceux décrits dans le Colour Index International 3$^{e}$ édition, et notamment les composés suivants :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23

- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les chromophores quinoniques, ceux mentionnés dans le Colour Index International précité conviennent, et parmi ceux-ci, on peut citer entre autres, les radicaux issus des colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99

ainsi que les composés suivants :

-1-N-méthylmorpholiniumpropylamino-4-hydroxyanthraquinone
-1-Aminopropylamino-4-méthylaminoanthraquinone
-1-Aminopropylaminoanthraquinone
-5-β-hydroxyéthyl-1,4-diaminoanthraquinone
-2-Aminoéthylaminoanthraquinone
-1,4-Bis-(βγ-dihydroxypropylamino)-anthraquinone.

Parmi les chromophores aziniques, conviennent ceux listés dans le Colour Index International et par exemple les radicaux issus des colorants suivants :

- Basic Blue 17
- Basic Red 2.

Parmi les chromophores triarylméthaniques utilisables selon l'invention, on peut citer, outre ceux listés dans le Colour Index, les radicaux issus des colorants suivants :

- Basic Green 1
- Acid blue 9
- Basic Violet 3
- Basic Violet 14
- Basic Blue 7
- Acid Violet 49
- Basic Blue 26
- Acid Blue 7

Parmi les chromophores indoaminiques utilisables selon l'invention, on peut citer les radicaux issus des colorants suivants :

-2-β-hydroxyéthylamino-5-[bis-(β-4'-hydroxyéthyl)amino]anilino-1,4-benzoquinone
-2-β-hydroxyéthylamino-5-(2'-méthoxy-4'-amino)anilino-1,4-benzoquinone
-3-N(2'-Chloro-4'-hydroxy)phényl-acétylamino-6-méthoxy-1,4-benzoquinone imine
-3-N(3'-Chloro-4'-méthylamino)phényl-uréido-6-méthyl-1,4-benzoquinone imine
-3-[4'-N-(Ethyl,carbamylméthyl)-amino]-phényl-uréido-6-méthyl-1,4-benzoquinone imine.

On peut aussi citer les chromophores décrits dans les documents US 5888252, EP 1133975, WO 03/029359, EP 860636, WO 95/01772, WO 95/15144, EP 714954. On peut aussi citer ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitre de l'encyclopédie "ULLMANN's ENCYCLOPEDIA of Industrial chemistry" 7th édition, Wiley and sons.

De préférence, les chromophores sont choisis parmi ceux issus de colorants de type azoïque, anthraquinone et hydrazone.

Selon une variante, A et/ ou A' des formules (I), (II), (III) ou (IV) contiennent au moins un radical cationique porté par ou inclus dans au moins un des chromophores.

De préférence, le radical cationique est un ammonium quaternaire.

Ces radicaux cationiques sont par exemple un radical alkylammonium, acridinium, benzimidazolium, benzobistriazolium, benzopyrazolium, benzopyridazinium, benzoquinolium, benzothiazolium, benzotriazolium, benzoxazolium, bi-pyridinium, bis-tetrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phénazinium, phénooxazolium, pyrazinium, pyrazolium, pyrazoyltriazolium, pyridinium, pyridinoimidazolium, pyrrolium, quinolium, tétrazolium, thiadiazolium, thiazolium, thiazolopyridinium, thiazoylimidazolium, thiopyrylium, triazolium, xanthylium.

Des exemples de chromophores cationiques utiles dans la présente invention ont été cités précédemment. D'autres exemples sont donnés dans les demandes de brevet WO 95/01772, WO 95/15144, EP 714954, EP 318294, WO 03/029359,

Selon un mode de réalisation particulier, les radicaux A, A' dans les formules (I), (II), (III) ou (IV) comprennent au moins un chromophore azoïque cationique, décrit par exemple dans EP 850636, FR 2788433, EP 920856, WO 9948465, FR 2757385, EP 850637, EP 918053, WO 9744004, FR 2570946, FR 2285851, DE 2538363, FR 2189006, FR 1560664, FR 1540423, FR 1567219, FR 1516943, FR 1221122, DE 4220388, DE 4137005, WO 0166646, US 5708151, WO 9501772, WO 515144, GB 1195386, US 3524842, US 5879413, EP 1062940, EP 1133976, GB 738585, DE 2527638, FR 2275462, GB 1974-27645, Acta Histochem. (1978), 61(1), 48-52 ; Tsitologiya (1968), 10(3), 403-5 ; Zh. Obshch. Khim. (1970), 40(1), 195-202 ; Ann. Chim. (Rome) (1975), 65(5-6), 305-14 ; Journal of the Chinese Chemical Society (Taipei) (1998), 45(1), 209-211 ; Rev. Roum. Chim. (1988), 33(4), 377-83; Text. Res. J. (1984), 54(2), 105-7; Chim. Ind. (Milan) (1974), 56(9), 600-3; Khim. Tekhnol. (1979), 22(5), 548-53 ; Ger. Monatsh. Chem. (1975), 106(3), 643-8 ; MRL Bull. Res. Dev. (1992), 6(2), 21-7 ; Lihua Jianyan, Huaxue Fence (1993), 29(4), 233-4 ; Dyes Pigm. (1992), 19(1), 69-79 ; Dyes Pigm. (1989), 11(3), 163-72.

Comme indiqué auparavant, dans les formules (I), (II), (III) et (IV), $C_{sat}$ et $C'_{sat}$, indépendamment l'un de l'autre, représentent une chaîne alkylène en $C_1$-$C_{18}$, linéaire ou ramifiée, éventuellement substituée, éventuellement cyclique. A titre de substituant, on peut citer les groupements carboxylate, ester ou amide, présents de préférence sur le carbone en position béta ou gamma des atomes de soufre.

De préférence, dans le cas des formules (I), (II) et (IV), $C_{sat}$ représente une chaîne $-(CH_2)_n-$ avec n entier, compris entre 1 et 8.

De préférence, dans le cas de la formule (III), $C_{sat}$ représente un radical $-(CH_2)_n-$, $C'_{sat}$ représente un radical $-(CH_2)_n-CH-$ n ayant la même signification que précédemment

Conformément à un mode de réalisation particulier de l'invention, dans les formules (I), (II), (III) ou (IV) précitées, lorsque p est égal à1, X représente la séquence suivante :

$$-(T)_t-(Y)_y-(Z)_z-$$

ladite séquence étant reliée dans les formules (I), (II), (III) ou (IV), comme suit :

- $C_{sat}$ (ou $C'_{sat}$)$-(T)_t-(Y)_y-(Z)_z-$(A ou A') ; dans iaquelle
  **T** représente un ou plusieurs radicaux ou leurs combinaisons choisis parmi $-SO_2-$, -O-, -S-, -N(R)-, -N+(R)(R) -CO-, R représentant un atome d'hydrogène, un radical alkyle en $C_1$-$C_4$, ou hydroxyalkyle en $C_1$-$C_4$;
  le coefficient **t** vaut 0 ou 1 ;
  **Y** représente :

  - un radical choisi parmi $-(CH_2)_2-SO_2-$ ; $-CH_2-CHR-CO-NR'-$ avec R, R', identiques ou non, représentant un atome d'hydrogène, un radical alkyle en $C_1$-$C_4$.
  - un groupement de formule (a), (a') ou (a") :

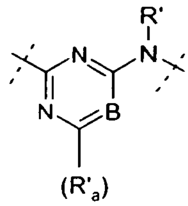 (a) 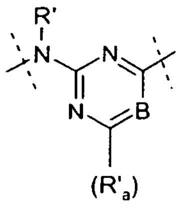 (a') 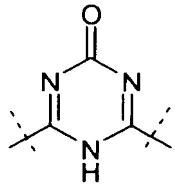 (a'')

dans laquelle

- B représente -N-, $-CR_a$, avec $R_a$ représentant un atome d'hydrogène, un atome d'halogène choisi parmi le chlore ou le fluor, un groupement nitro, un groupement pyridinium éventuellement substitué;
- R' a la même définition que précédemment
- $R'_a$ représente :
  - un atome d'hydrogène
  - un atome de chlore ou un atome de fluor
  - un groupement pyridinium éventuellement substitué par au moins un groupement $R_c$, $R_c$ prouvant être un groupement alkyle en $C_1$-$C_4$, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C1-C18, éventuellement porteurs d'au moins un hydroxyle) ; un groupement ester $-COOR_d$ avec $R_d$ représentant un radical alkyle en $C_1$-$C_4$; un groupement amide $-CON(R_d)_2$ avec $R_d$ identiques ou non, représentant un atome d'hydrogène ou un radical alkyle en $C_1$-$C_4$ ;
  - un groupement hydroxyle
  - un groupement amino, alkylamino ou dialkylamino, les groupements alkyles identiques ou différents, en C1-C18, étant linéaires ou ramifiés, éventuellement interrompus par un hétéroatome choisi parmi N, O, éventuellement substitués par un ou plusieurs groupements hydroxyles
  - un groupement NHNHCOR où R représente un groupement alkyle linéaire ou ramifié en C1-C10

- un groupement de formule (b) suivante :

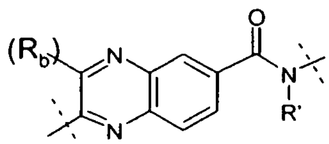 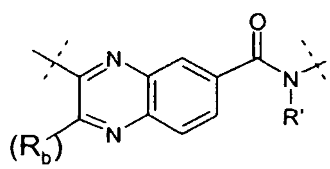

dans laquelle

- R' a la même définition que précédemment
- $R_b$ représente
  - un atome de chlore
  - un groupement amino, alkylamino ou dialkylamino, les groupements alkyles identiques ou différents, en $C_1$-$C_{18}$, étant linéaires ou ramifiés, éventuellement interrompus par un hétéroatome choisi parmi N, O, S, éventuellement substitués par un ou plusieurs hydroxyles
  - un hétérocycle azoté, saturé ou insaturé, pouvant être substitué
  - un groupement arylamino, dans lequel de préférence le radical aryle est en $C_6$;

**y** vaut 0 ou 1 ;
**Z** représente

- $-(CH_2)_m-$ avec m entier compris entre 1 et 8
- $-(CH_2CH_2O)_q-$ ou $-(OCH_2CH_2)_q-$ dans lesquelles q est un entier compris entre 1 et 15

- un radical aryle, alkylaryle ou arylalkyle dont le radical alkyle est en $C_1$-$C_4$ et le radical aryle est de préférence en $C_6$, étant éventuellement substitué par au moins un groupement $SO_3M$ avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle

**z** vaut 0 ou 1.

Avantageusement, Y représente l'un des groupements ci-dessous,

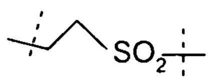
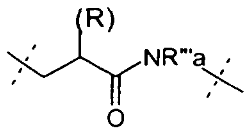
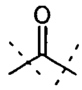
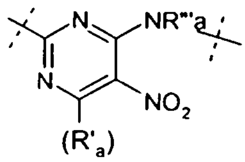
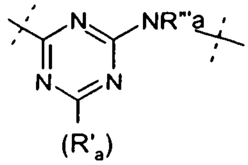

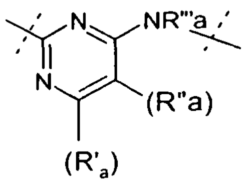
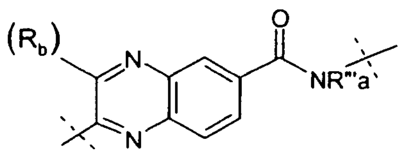
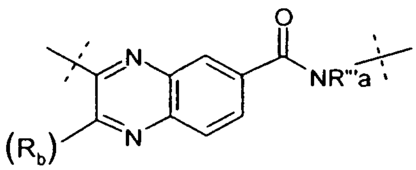

dans lesquels les radicaux R, $R'_a$ et $R_b$ sont définis comme précédemment ; $R''_a$, a la même définition que $R'_a$, indépendamment les uns des autres ; $R'''_a$ représente un atome d'hydrogène ou un radical alkyle.

De préférence, Y représente les groupements suivants :

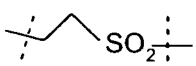
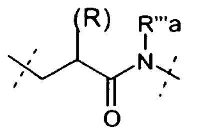
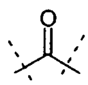

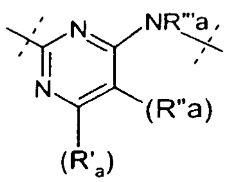
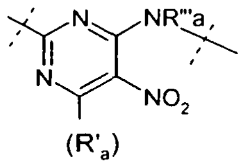

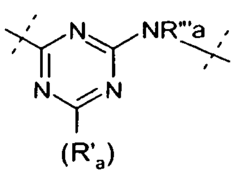
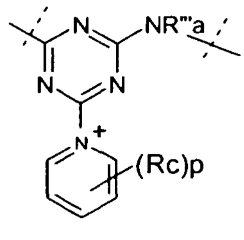

dans lesquels

- R représente un atome d'hydrogène ou un radical méthyle
- $R'_a$, $R''_a$, identiques ou non, représentent un atome de chlore, de fluor ou d'hydrogène

- $R_c$ représente un radical alkyle en $C_1$-$C_4$ ; un groupement carboxylique -COOM avec M représentant un atome d'hydrogène, un métal alcalin, un groupement ammonium substitué ou non ; un groupement ester -$COOR_d$ avec $R_d$ représentant un radical alkyle en $C_1$-$C_2$, ou un groupement amide -$CON(R_d)_2$ avec $R_d$, identiques ou non, représentant un atome d'hydrogène, un radical alkyle en $C_1$-$C_2$
- $R'''_a$ représentant un atome d'hydrogène, un radical alkyle avantageusement en $C_1$-$C_2$ et p est compris entre 0 et 2.

Par ailleurs, selon un mode de réalisation particulier de l'invention, Z représente :

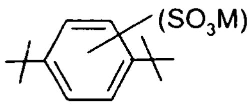
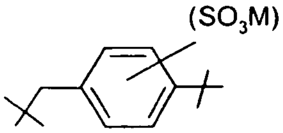
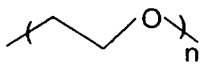
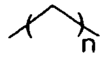

Comme indiqué auparavant, dans la formule (II), V représente un groupe pontant les deux radicaux A' identiques ou différents et v peut être égal à 0 ou 1.

Dans cette variante, le groupe V pontant les deux chromophores A' représente un radical alkyle en $C_1$-$C_8$, éventuellement terminé à l'une ou ses deux extrémités par un groupement choisi parmi amine, amide ou ester.

Conformément à un mode de réalisation particulier de l'invention, le colorant disulfure est tel que v est égal à 0.

A titre d'exemples particuliers, le colorant disulfure est choisi parmi :

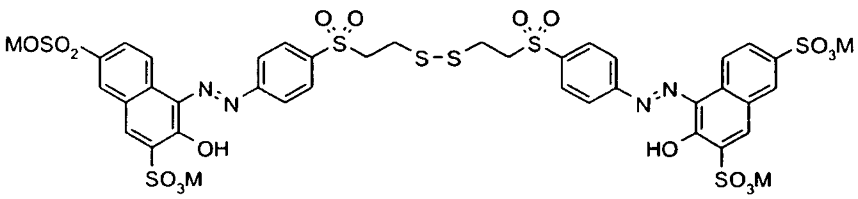

avec M représentant un atome d'hydrogène, un métal alcalin ou un groupement ammonium ou un groupement ammonium substitué par un ou plusieurs radicaux alkyle, identiques ou non, linéaires ou ramifiés, en $C_1$-$C_{10}$ éventuellement porteurs d'au moins un hydroxyle, ainsi que les composés suivants, sous forme acide, basique ou neutralisée :

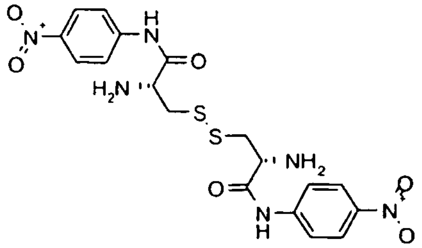
(1)

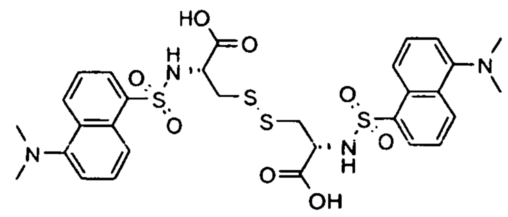

(2)

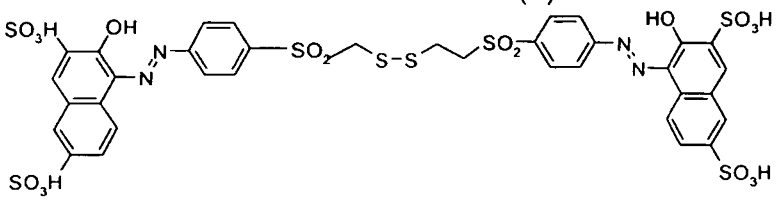

(3)

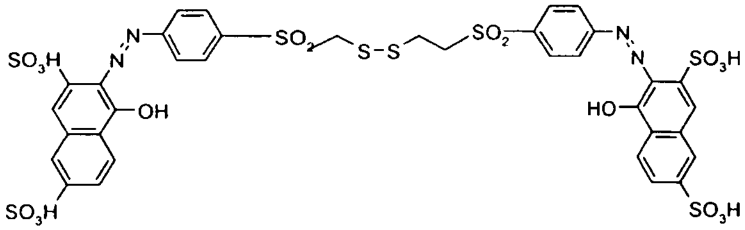

(4)

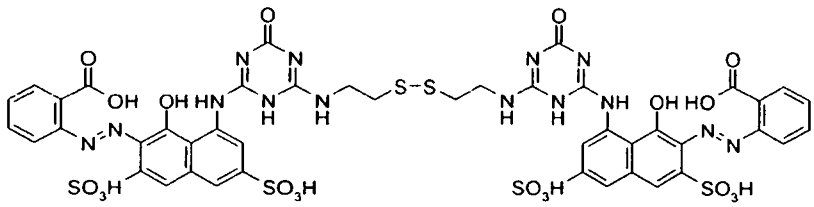

(5)

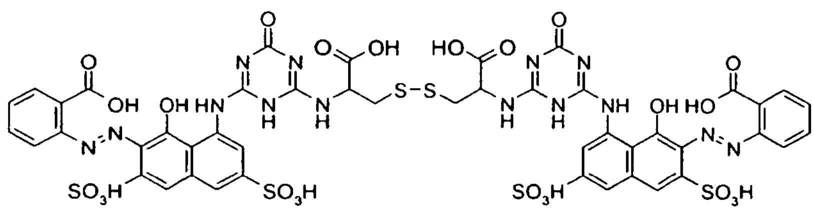

(6)

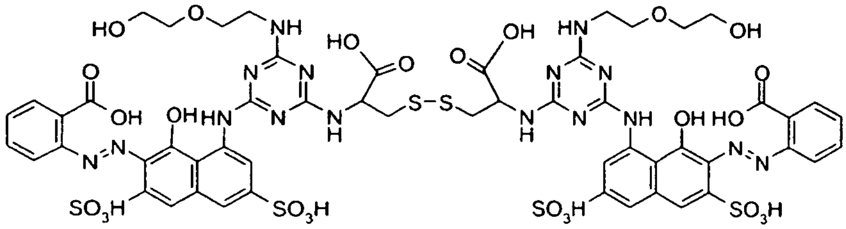

(7))

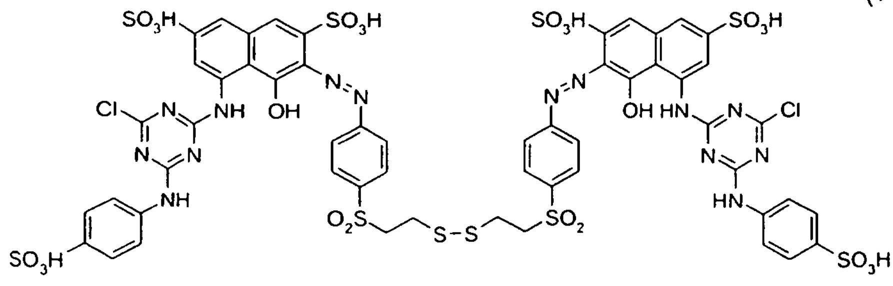

(8))

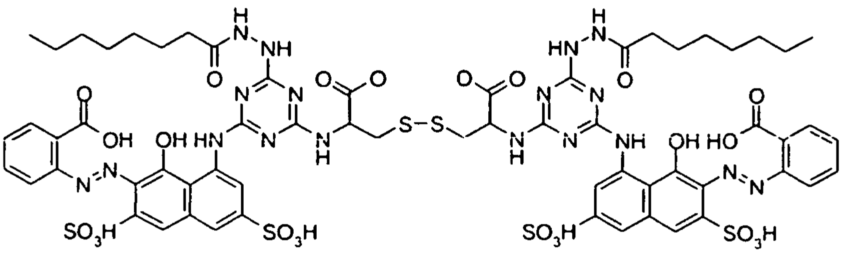

(9)

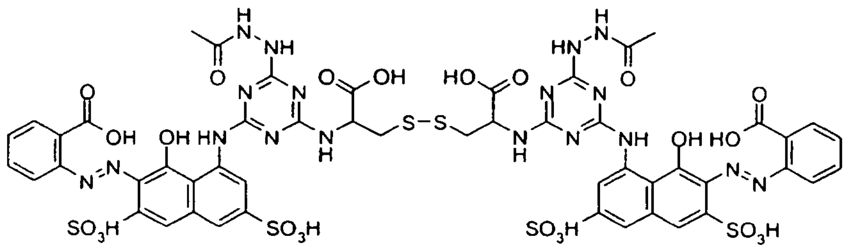

(10)

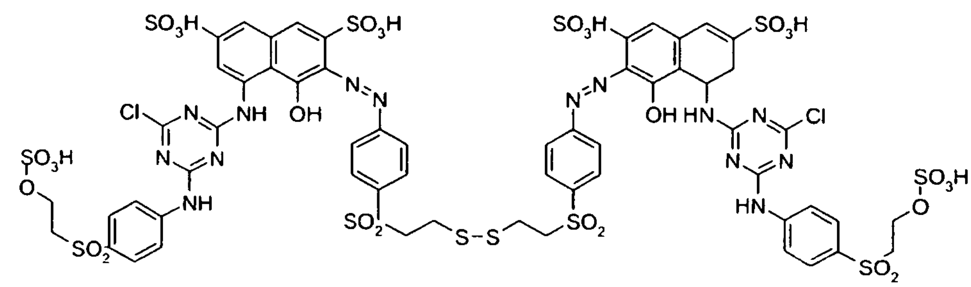

(11)

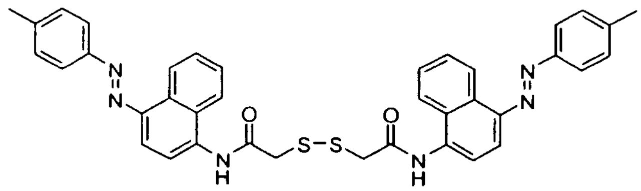

(12)

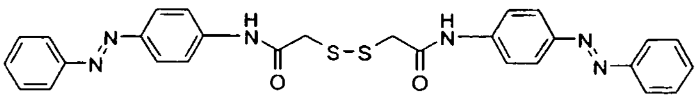

(13)

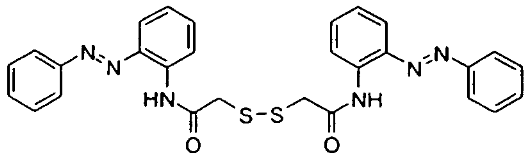

(14)

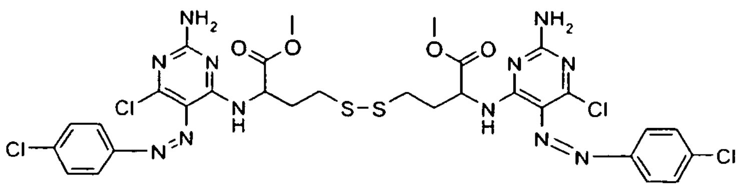

(15)

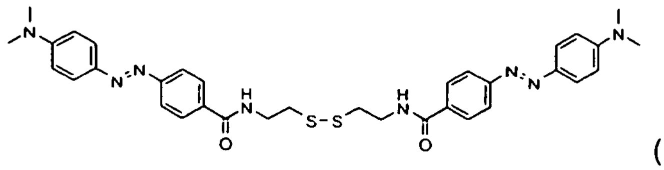

(16)

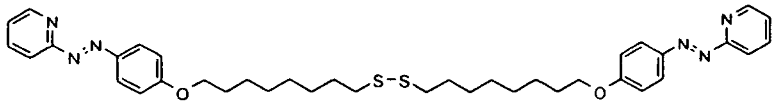

(17)

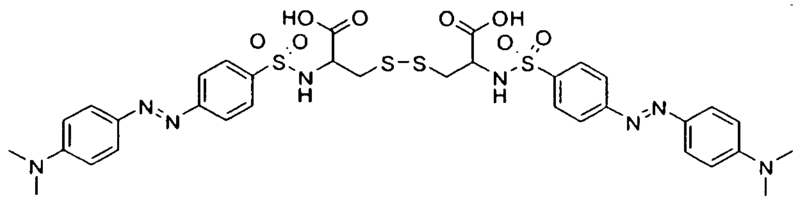

(18)

Ces composés particuliers, indépendamment de tout choix de forme libre ou salifiée, ainsi que leur mode de préparation sont connus de la technique.

A titre d'exemples particuliers on peut également citer les composés suivants, nouveaux et dont une préparation est décrite dans la présente invention

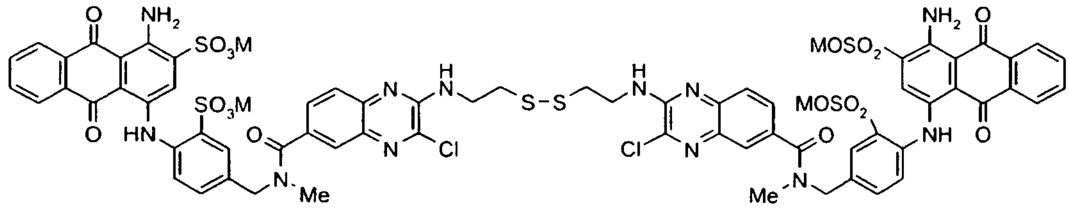

(19)

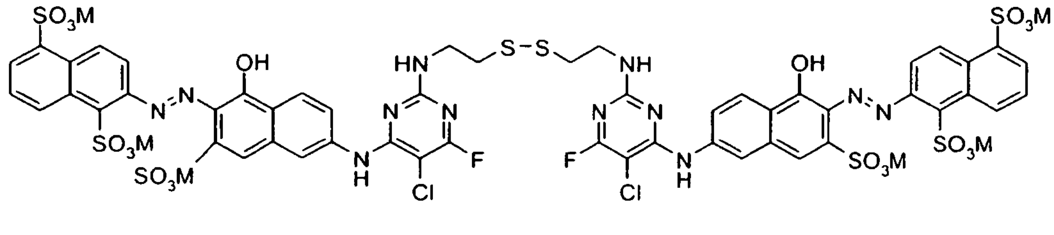

(20)

M ayant la même signification que précédemment.

Conformément à un mode préféré de l'invention, les deux radicaux contenant au moins un chromophore A et A' contiennent au moins un radical cationique porté ou inclus dans ou sur un chromophore, à titre d'exemple on peut citer les composés de formule suivante, leurs sels, hydrates ou solvates :

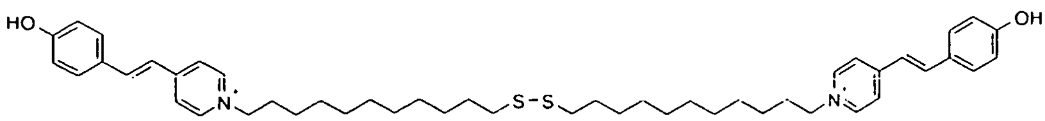

(21)

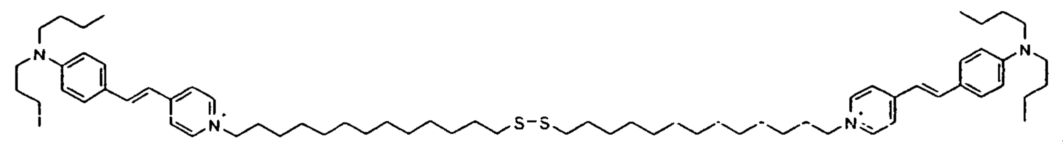

(22)

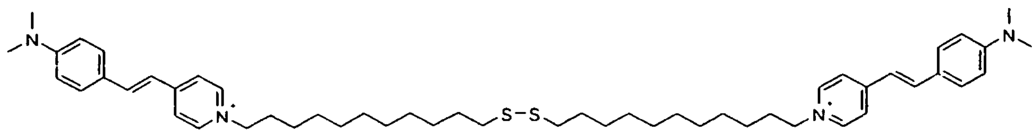

(23)

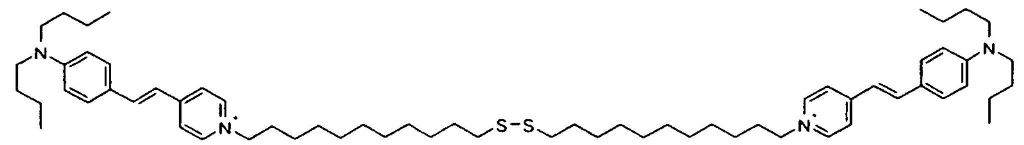

(24)

Ces quatre composés, indépendamment de tout choix de forme libre ou salifiée, ainsi que leur mode de préparation sont connus de la technique.

Selon une autre variante préférée de l'invention, le colorant disulfure est un colorant cationique comprenant au moins un radical ammonium quaternaire et tel que, dans la formule (I) avec p égal à 1 :

**A** représente W-N=N-Ar- ou -W-N=N-Ar, avec W représentant un hétérocycle aromatique ou non, condensé ou non, comprenant un ammonium quaternaire ; Ar représente un radical aryle en $C_5$, $C_6$, ou un bicycle aromatique de type naphtyle, éventuellement substitués par un ou plusieurs atomes d'halogène, de préférence chlore, fluor ; par un ou plusieurs groupements alkyle, de préférence en $C_1$-$C_4$ ; par un ou plusieurs groupements hydroxyle ; par un ou plusieurs groupements alcoxy, par un ou plusieurs groupements hydroxyalkyle, par un ou plusieurs groupements amino ou (di)alkylamino, de préférence avec la partie alkyle en $C_1$-$C_4$

Selon une variante préférée p=1, y=z=0, t=1 et T représente -N(R)-, de préférence en position para sur Ar par rapport à la fonction azoïque.

De préférence, W est un imidazolium, pyridinium, benzimidazolium, pyrazolium, benzothiazolium, éventuellement substitués par un ou plusieurs radicaux alkyles, identiques ou non, en $C_1$-$C_4$.

Parmi les colorants disulfure préférés de la présente invention, on peut citer notamment les composés suivants :

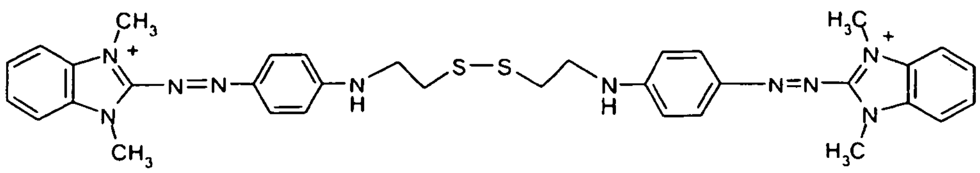

2M'

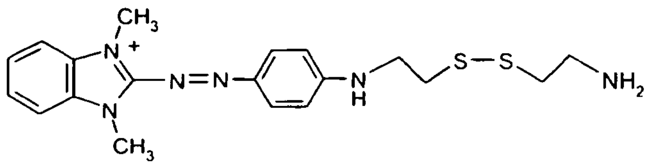

M'

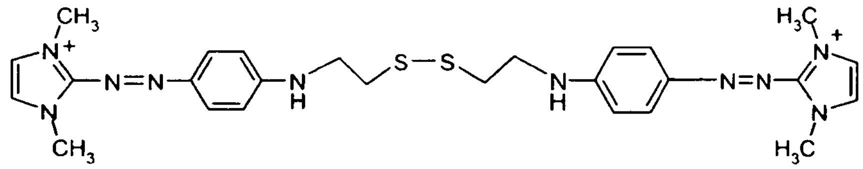

2M'

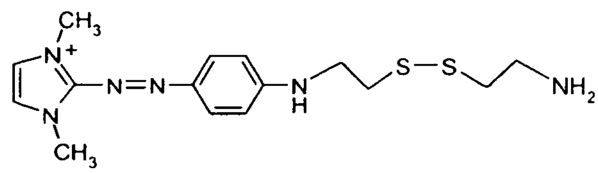

M'

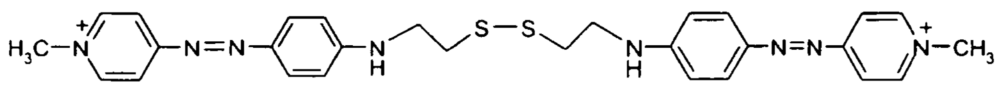

2M'

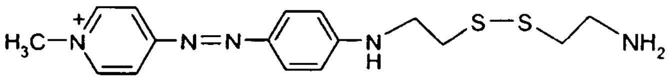

M'

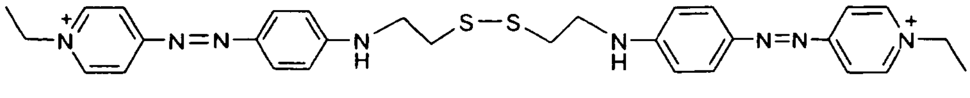

2M'

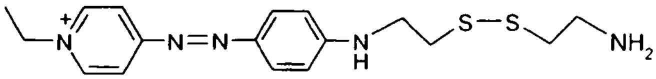

M'

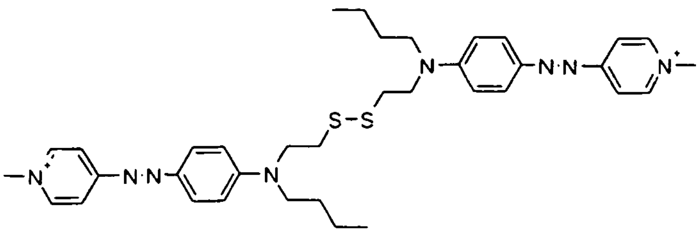

2M'

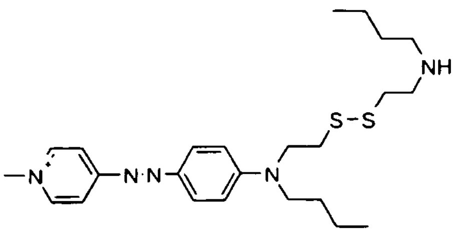

M'

avec M' représentant un sel d'acide organique ou minéral.

Plus particulièrement, ledit sel d'acide organique ou minéral est choisi parmi les chlorhydrates, les bromhydrates, les sulfates, parmi lesquels le méthylsulfate et l'éthylsulfate, les citrates, les succinates, les tartrates, les lactates, les acétates, les méthosulfates, les tosylates, les benzènesulfonates, les phosphates et les acétates.

Les colorants disulfure peuvent être préparés selon des méthodes connues de l'homme de l'art.

Selon une première possibilité, on peut faire réagir un composé disulfure comprenant deux fonctions amine, de préférence primaire ou secondaire avec une quantité suffisante d'un "chromophore réactif' ou d'un composé comprenant un tel "chromophore réactif', en d'autres termes comprenant une fonction électrophile.

Parmi les "chromophores réactifs", on peut citer les colorants réactifs répertoriés comme tels dans Colour Index et comportant notamment une fonction vinylsulfone, sulfatoéthylsulfone, mono- di- chlorotriazine, mono- di-chloro pyrimidine, difluoro chloro pyrimidine, dichloroquinoxaline, bromo-vinylsulfone.

Conviennent aussi, en tant que chromophores réactifs, les composés chromophores comprenant au moins un groupement susceptible de réagir avec une fonction amine pour donner un groupement sulfamide ($-SO_2-N-$), amide (-

CO-N-). Par exemple, on peut mentionner les groupes $-SO_3M'$, -COO M' (avec M' représentant un atome d'hydrogène, un métal alcalin, comme le sodium, le potassium, un groupement ammonium, un groupement ammonium substitué par un ou plusieurs groupement alkyle, identiques ou non, linéaires ou ramifiés, en C1-C10, éventuellement porteurs d'au moins un hydroxyle), que l'on peut activer préalablement, selon des méthodes connues, respectivement en groupement $-SO_2Cl$, -COCl.

Il est ainsi envisageable de mettre en oeuvre, à titre de chromophore réactif, les colorants acides du Colour Index répertoriés comme tels.

On pourra se référer notamment à l'ouvrage Advanced Organic Chemistry, March, 4ème Ed, pour avoir plus de détails sur les conditions opératoires mises en oeuvre.

Toujours dans le cadre de cette première possibilité, on peut mettre en oeuvre des chromophores comprenant un groupement labile directement lié ou non au chromophore et susceptible d'être substitué par un groupement amine, tel que Cl, Br, F, O-alkyle (par exemple O-Me), O-aryle, O-alkylaryle (par exemple O-benzyle).

Les colorants disulfure peuvent aussi être obtenus, dans le cadre de cette possibilité, en utilisant des chromophores possédant une fonction acrylate (-OCO-C=C-) sur laquelle on effectue une réaction d'addition.

Conformément à une autre possibilité, les colorants disulfures peuvent être obtenus en faisant réagir un composé disulfure avec un composé portant deux fonctions acide carboxylique activées selon les méthodes classiques (par exemple réaction avec un carbodiimide ou avec le chlorure de thionyle). Le produit résultant est ensuite mis à réagir avec un chromophore porteur d'une fonction nucléophile, par exemple de type amine primaire ou secondaire, ou de type alcool aliphatique ou aromatique comme le phénol.

Là encore, on pourra se référer à l'ouvrage Advanced Organic Chemistry, March, 4ème Ed, pour avoir plus de détails sur les conditions opératoires mises en oeuvre.

Conformément à une troisième possibilité, les colorants disulfure peuvent être obtenus par réaction d'un composé comprenant un groupement disulfure et deux groupements hydroxyle activés préalablement en groupements partants (par exemple mésylate, tosylate) avec un chromophore portant une fonction nucléophile, avantageusement de type amine primaire,secondaire ou tertiaire, hétéroaromatique ou non, par exemple de type pyridine, imidazole, benzimidazole.

Conformément à une quatrième possibilité, les colorants disulfure peuvent être obtenus par oxydation ménagée de colorants portant une fonction SH.

Conformément à une cinquième possibilité, et notamment pour la préparation des composés répondant aux formules (III) et (IV), les colorants disulfures peuvent être obtenus par une variante des possibilités une, deux ou trois décrites ci-dessus, en utilisant une quantité molaire de réactif disulfure supérieure ou égale à la quantité molaire de réactif contenant le groupement chromophore.

La préparation des colorants disulfure répondant à la formule (I) est en revanche facilitée par l'emploi d'une quantité molaire de reactif contenant le groupement chromophore de préférence supérieure ou égale à deux fois la quantité de reactif disulfure.

Conformément à une sixième possibilité, et notamment pour la préparation des composés répondant à la formule (I) et dont les deux groupements A, d'une part, X d'autre part, sont différents, les composés disulfures peuvent être obtenus à partir de composés disulfures répondant à la formule (IV)

La composition tinctoriale utile dans le procédé de l'invention peut contenir un ou plusieurs colorants disulfure de formules (I), (II), (III) ou (IV).

La composition contient en général une quantité de colorant disulfure comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs différents des colorants disulfure de formules (I), (II), (III) ou (IV). Ces colorants directs sont par exemple choisis parmi les colorants directs listés auparavant, et notamment parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants de type tétraazapentaméthiniques utilisables, on peut citer les composés suivants figurant dans le tableau ci-dessous, An représentant en général un anion organique ou minéral comme défini précédemment :

| | |
|---|---|
| 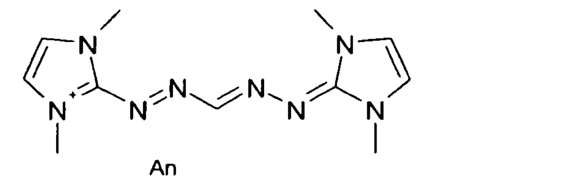 | 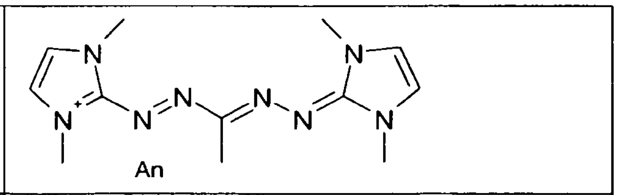 |
| 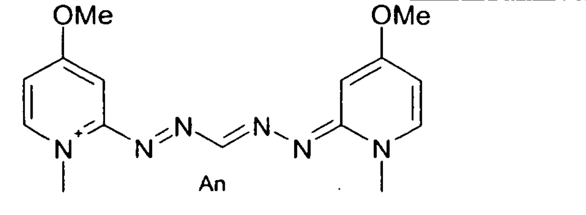 | 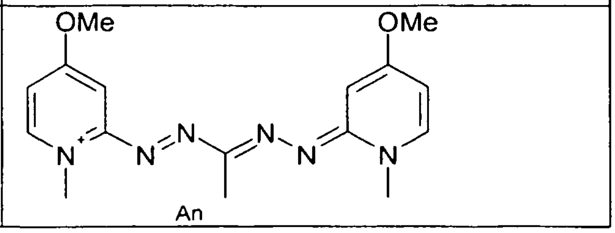 |

| | |
|---|---|
| 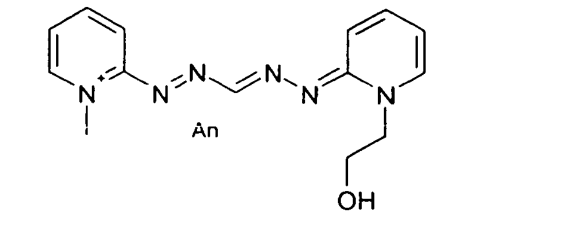 | 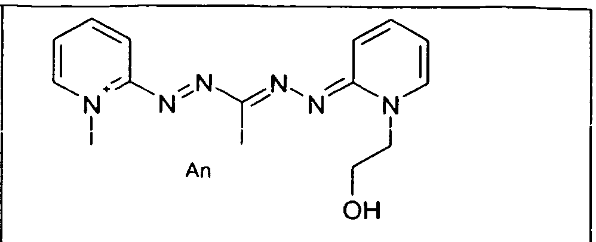 |
| 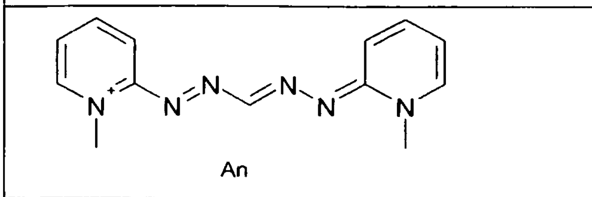 | 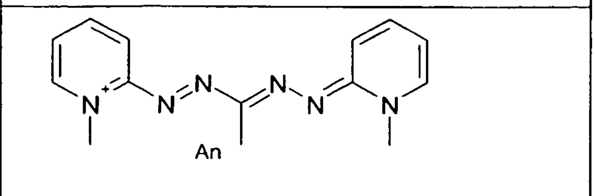 |
| 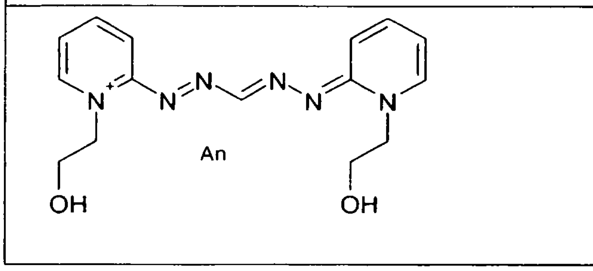 | 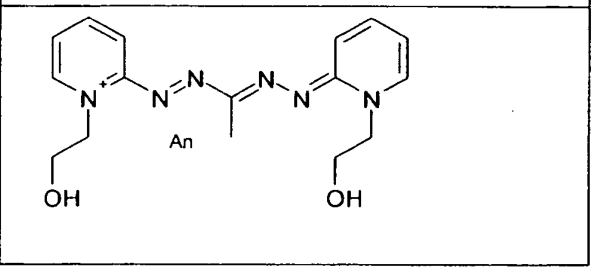 |

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre

de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

La composition peut aussi comprendre au moins un autre composé disulfure additionnel différent de celui correspondant aux formules (I) à (IV) détaillées ci-dessus. A titre indicatif, le disulfure peut être choisi parmi les composés comprenant au moins une chaîne grasse, plus particulièrement au moins une chaîne hydrocarbonée en $C_5$-$C_{30}$, linéaire ou ramifiée, saturée ou non, éventuellement substituée par un hétéroatome, éventuellement interrompue par un groupement carboxylique, neutralisé ou non. A titre d'exemple de composés de ce type, on peut citer les dimères de l'acide thioglycolique et ses dérivés du type $CH_3$-$(CH_2)_{17}$-S-S-$(CH_2)_{17}$-$CH_3$ ou $CH_3$-$(CH_2)$ -S-S-$(CH_2)_{10}$-$CH_3$

S'il est présent, la teneur en ce composé est comprise entre 0,001 et 10 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante :

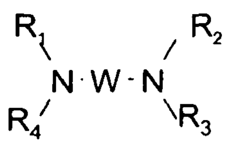
(A)

dans laquelle W est un reste propylène éventuellement substitué par un groupement hydroxyle ou un radical alkyle en $C_1$-$C_4$ ; $R_1$, $R_2$, $R_3$ et $R_4$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Selon un mode de réalisation particulier, le procédé de l'invention comprend un pré-traitement avec un agent

réducteur capable de réduire la liaison disulfure. L'agent réducteur est par exemple choisi parmi les thiols par exemple l'acide thioglycolique, la cystéine, l'homocystéine, l'acide thiolactique, les sels de ces thiols, les phosphines, le bisulfite, les sulfites.

Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tetraméthylammonium, tetraéthylammonium, tétra n-butylammonium, benzyltriéthylammonium); le catécholborane.

Ce prétraitement peut être de courte durée, de 0,1 seconde à 30 minutes, de préférence de 0,1 seconde à 5 minutes avec une agent réducteur tel que cité précédemment.

L'application de la composition tinctoriale est généralement effectuée à température ambiante. Elle peut cependant être réalisée à des températures variant de 20 à 180°C.

Selon une variante, l'agent réducteur est ajouté à la composition tinctoriale au moment de l'emploi.

Selon une autre variante, l'application de la composition tinctoriale peut être suivie par une étape courte de réduction de 0,1 seconde à 30 minutes de préférence de 0,1 seconde à 5 minutes, avec un agent réducteur de type thiol ou borohydrure tel que décrit précédemment.

Suivant une autre variante, la composition tinctoriale peut comporter un agent d'oxydation, on parle alors de composition prête à l'emploi.

Habituellement, ladite composition est obtenue en mélangeant la composition selon l'invention avec une composition oxydante avant l'application sur les matières kératiniques à traiter.

L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition tinctoriale exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

L'application de la composition tinctoriale peut être suivie d'une étape de post-traitement oxydant, ou d'une étape de post-traitement conditionneur éventuellement associé à une étape de post-traitement oxydant.

L'invention a aussi pour objet un dispositif à plusieurs compartiments ou "kit" de teinture dans lequel un premier compartiment renferme une composition tinctoriale comprenant au moins un colorant disulfure de formules (I), (II), (III) ou (IV) et un deuxième compartiment renferme un agent réducteur capable de réduire la liaison disulfure du colorant.

L'un de ces compartiments peut en outre contenir un ou plusieurs autres colorants de type colorant direct ou colorant d'oxydation à la condition que le colorant disulfure utile dans l'invention et les colorants de type colorant direct ou colorant d'oxydation ne soient pas présents dans le même compartiments du kit.

Elle concerne aussi un dispositif à plusieurs compartiments dans lequel un premier compartiment contient une composition tinctoriale comprenant au moins un colorant disulfure de formules (I), (II) ou (III) ou (IV); un deuxième compartiment contient un agent réducteur capable de réduire la liaison disulfure du colorant ; un troisième compartiment contient un agent oxydant.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif.

## EXEMPLES

## EXEMPLES DE SYNTHESE

### Exemple 1

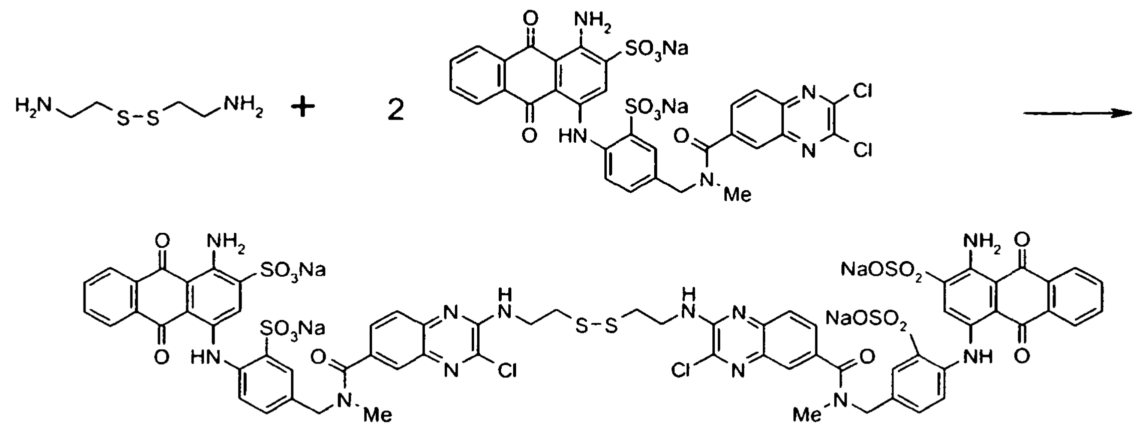

On prépare une solution composée de 500mg de dihydrochlorure de cystamine 2.22 mmol) et de 20ml de tampon borate 0,05M pH 9 que l'on ramène à pH 9 par addition de soude molaire. A cette solution, on ajoute 4,6 grammes de Reactive Blue 44 [Cas number 12225-56-8], à 60% de pureté soit 4,44mmol). le mélange est agité pendant 18 heures à température ambiante, le colorant précipité est filtré et séché. On recueille 1,67g de colorant bleu (I).

**Exemple 2**

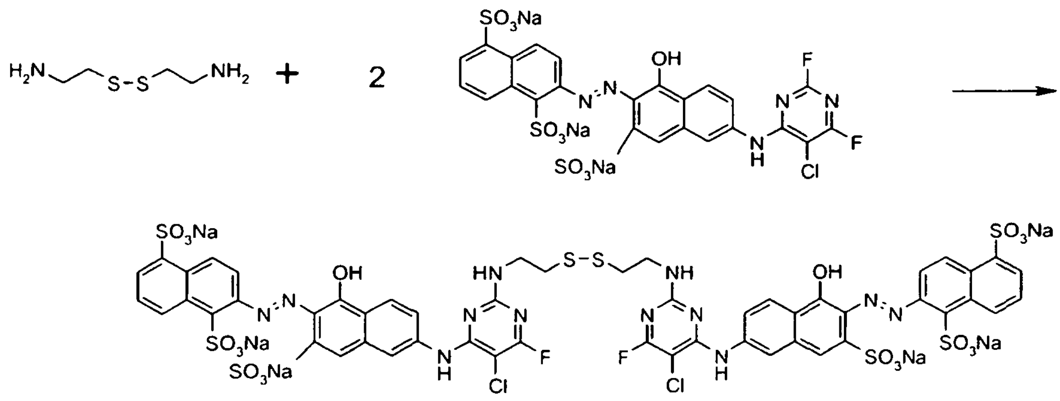

On prépare une solution composée de 500mg de dihydrochlorure de cystamine 2,22 mmol) et de 20mL de tampon borate 0,05M pH 9 que l'on ramène à pH 9 par addition de soude molaire. A cette solution, on ajoute 6,23 grammes de Reactive Orange 64 [Cas number 61901-80-2], à 50% de pureté soit 4,44mmol), le mélange est agité pendant 18 heures à température ambiante, le colorant précipité est filtré et séché. On recueille 2,03g de colorant orange (II).

**Exemple 3**

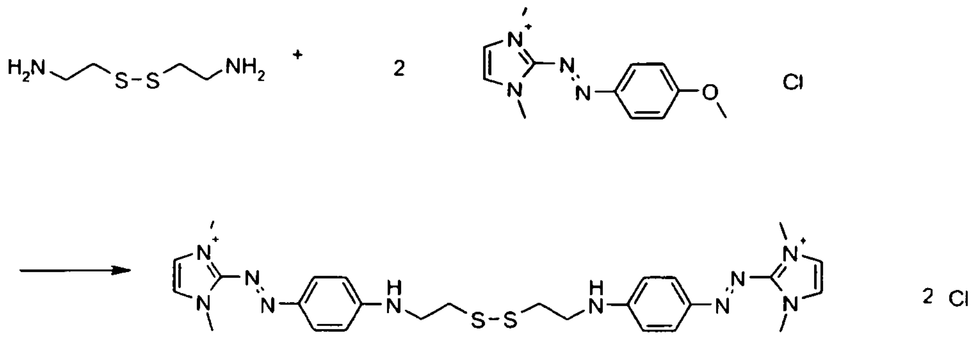

La cystamine base (552.2 mg ; 3.62 mmol) obtenue à partir de dichlorhydrate de cystamine par addtion de soude et extraction par l'acétate d'éthyle, est solubilisée dans 2 ml de pentanol. On ajoute le chlorure de 2-[(4-methoxyphenyl)diazenyl]-1,3-dimethyl-1H-imidazol-3-ium (2.42 m ; 9.1 mmol), en suspension dans 80 ml de dichlorométhane. Le mélange est porté à 50°C et maintenu agité 1 heure. Il est concentré sous vide (élimination du dichlorométhane), on ajoute 20 ml d'eau et le mélange réactionnel est maintenu une heure supplémentaire à 50°C. Il est ensuite refroidi et versé sur 50mL de pentanol ; un précipité rouge apparaît, qui est filtré et lavé par de l'acétone puis séché sous vide. On obtient ainsi 1.1 g de poudre rouge foncée conforme à la structure ci-dessus.

**Exemple 4**

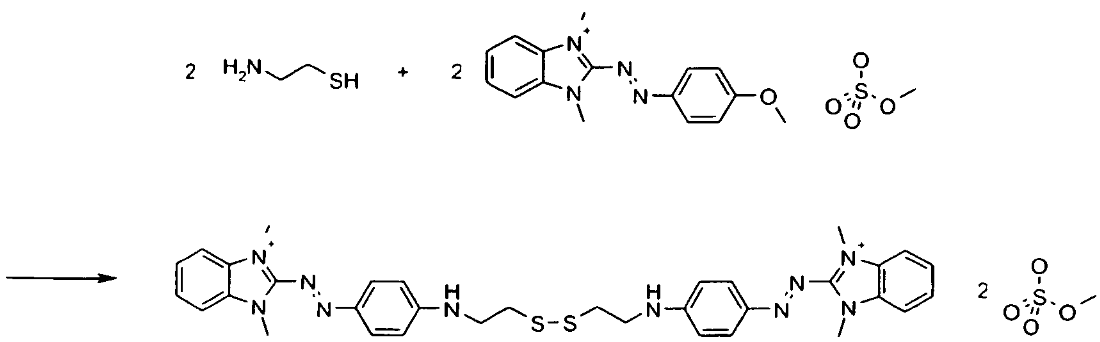

La cystéamine base (110.2 mg ; 0.73 mmol) est solubilisée dans 2 mL de méthanol. On ajoute le méthylsulfate de 2-[(4-methoxyphenyl)diazenyl]-1,3-dimethyl-1H-imidazol-3-ium (600 mg ; 1.53 mmol), en suspension dans 10 ml de dichlorométhane. Le mélange est porté à 35°C et maintenu agité 2 heures, en présence d'oxygène (air).
A la fin du procédé, on introduit 200 ml d'acétate d'éthyle.

Le précipité obtenu est filtré. On obtient une poudre noire (278 mg) qui contient majoritairement le produit attendu.

**Exemple 5 :**

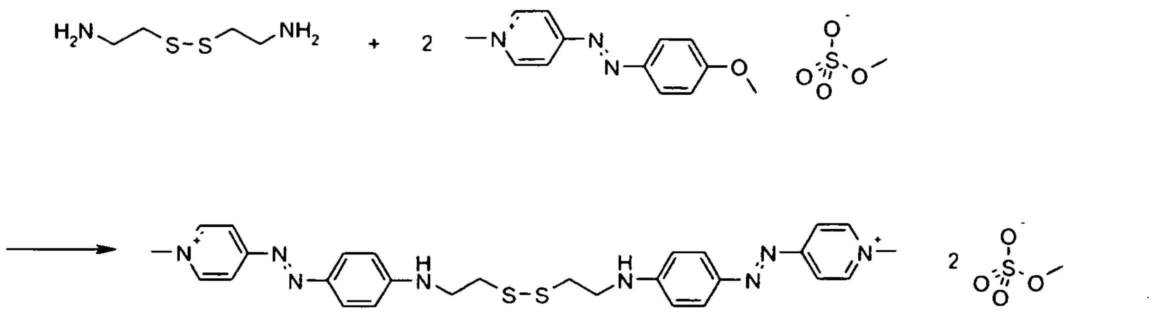

4.18 g de méthylsulfate de 4-[(E)-(4-methoxyphenyl)diazenyl]-1-methylpyridinium sont dissous dans 275 mL de dichlorométhane et ajoutés goutte à goutte sur une solution de 1.25 g de cystamine dans 2 mL de pentanol, à 50°C (le dichlorométhane distille ; le mélange réactionnel ainsi obtenu est homogène et concentré). Après 24h d'agitation le mélange est concentré sous vide. L'huile obtenue est mélangée avec de l'acétone et 100 mL de célite. La pâte obtenue est lavée par de l'acétone, du dichlorométhane et de l'acétate d'éthyle. Le produit attendu est ensuite désorbé de la célite par extraction à l'eau. La solution aqueuse obtenue est concentrée sous vide. On recueille ainsi 2g de solide rouge-noir. Les analyses montrent que ce solide contient majoritairement le produit attendu (m/z : 272 ; λ max 520).

**Exemple 6 :**

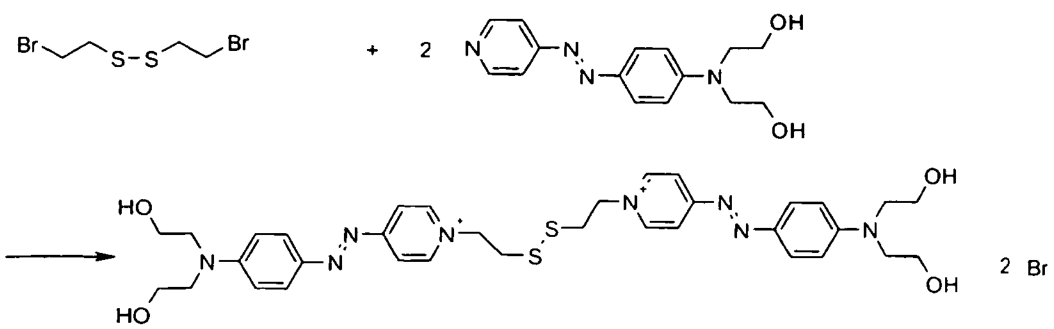

1g de 1-bromo-2-[(2-bromoethyl)disulfanyl]ethane et 2.14 g de 2-((2-hydroxyethyl){4-[(E)-pyridin-4-yldiazenyl] phenyl}amino)ethanol sont dissous dans 5 mL de diméthylformamide. Le mélange est agité et chauffé à 80°C pendant 4h. Après refroidissement du mélange, 200 mL d'acétone sont ajoutés. Une huile noire décante (1.94g). Cette huile est purifiée par extraction liquide liquide eau/ butanol et on recueille après évaporation des phases aqueuses 1.77 g de pâte noire. Les analyses montrent que cette huile contient majoritairement le produit attendu (m/z : 346 ; λ max 548 nm),

**Exemple 7 :**

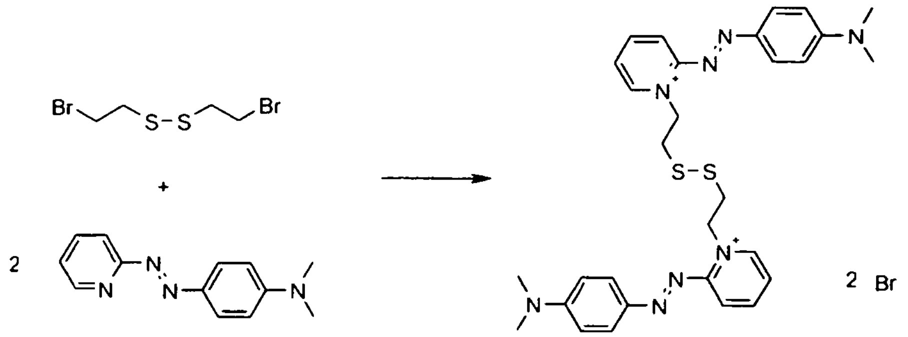

1 g de 1-bromo-2-[(2-bromoethyl)disulfanyl]ethane et 1.7 g de N,N-dimethyl-4-[(Z)-pyridin-2-yldiazenyl]aniline sont mélangés dans 5 mL de diméthylformamide. Le mélange est agité et chauffé à 80°C pendant 4 h. Après refroidissement du mélange réactionnel, 200 mL d'acétone sont ajoutés. Un précipité se forme, il est filtré, lavé avec trois fois 100 mL d'acétone puis séché. Le produit obtenu est solubilisé dans 50 mL de mélange eau / éthanol 1 :1 et reprécipité par addition d'acétone. Le précipité est filtré puis séché. On recueille ainsi 1.34 g de poudre violet-noir. Les analyses sont conformes à la structure du produit attendu (m/z 286 ; λ max : 534 nm).

**Exemple 8 :**

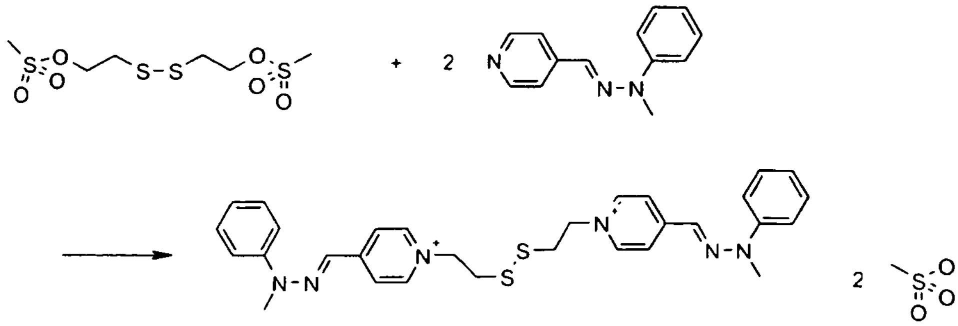

11.38 g de 2-({2-[(methylsulfonyl)oxy]ethyl}disulfanyl)ethyl methanesulfonate et 15.50 g 4-{(E)-[methyl(phenyl) hydrazono]methyl}pyridine sont mélangés dans 10 mL de diméthylformamide. Le mélange hétérogène ainsi obtenu est chauffé et agité à 80°C pendant 4 h. Le diméthylformamide est distillé sous vide. Le solide obtenu est purifié par chromatographie liquide liquide eau/ butanol. 7.64 g de solide jaune sont recueillis après évaporation du solvant. Les analyses sont conformes à la structure du produit attendu (m/z : 271 ; λ max 422 nm).

**Exemple 9 :**

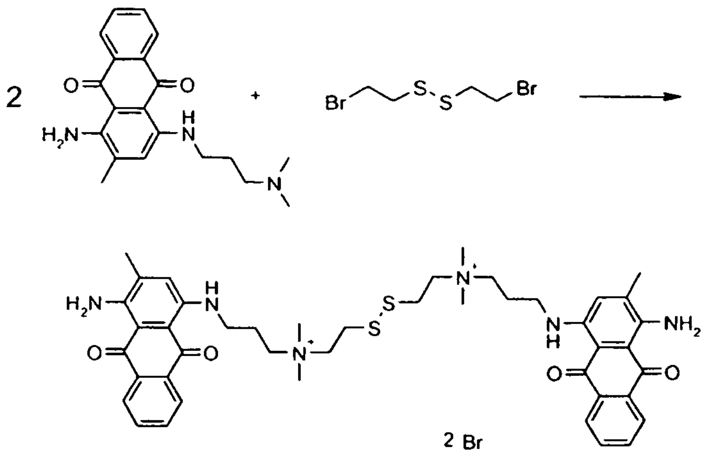

500 mg de 1-bromo-2-[(2-bromoethyl)disulfanyl]ethane et 1.405g de 1-amino-4-{[3-(dimethylamino)propyl]amino}-2-methylanthra-9,10-quinone sont dissous dans 5 mL de diméthylformamide. Le mélange ainsi obtenu est agité et chauffé à 80°C pendant 8h. Le mélange est refroidi et versé sur 200 mL d'acétone. Le précipité obtenu est filtré et lavé par 3 fois 100 mL d'acétone. Le solide obtenu est solubilisé dans 50 mL de mélange eau / éthanol 1 :1, puis reprécipité par addition d'acétone. Le précipité est filtré puis séché à l'étuve (66°C). On recueille 465mg de poudre noire. Les analyses sont conformes à la structure du produit attendu (m/z : 397 ; λ max 564 et 610 nm).

## EXEMPLES DE COLORATION

### Exemple 10

Etape 1 : prétraitement par une solution réductrice diluée

Des mèches de cheveux gris à 90% de cheveux blancs naturels, permanentés ou décolorés, sont imprégnées par une solution de réduction de permanente (Dulcia Vital N°2 commercialisé par L'Oréal Professionnel) diluée dans de l'eau permutée 1/2) pendant trois minutes puis rincées à l'eau courante pendant 30 secondes.

Etape 2 : application du colorant

Une solution du colorant disulfure de l'exemple 2 ci dessus à 1 % p/p dans un milieu tamponné à pH 9 (borate 0,05M) est appliquée sur les cheveux prétraités suivant l'étape 1 décrite ci-dessus, à raison de 5 g de solution par gramme de cheveu, pendant 10 minutes à température ambiante.
Les cheveux sont abondamment rincés à l'eau courante puis séchés.
Les mèches sont évaluées avant et après la teinture dans le système L*a*b*, au moyen d'un spectrophotomètre CM 2002 MINOLTA®, (Illuminant D65).
Dans le système L* a* b*, les trois paramètres désignent respectivement l'intensité (L*), la nuance (a*) et la saturation (b*). Selon ce système, plus la valeur de L est élevée, plus la couleur est claire ou peu intense. Inversement, plus la valeur de L est faible, plus la couleur est foncée ou très intense. a* et b* indiquent deux axes de couleurs, a* indique l'axe de couleur vert/rouge et b* l'axe de couleur bleu/jaune.
Les résultats sont regroupés dans le tableau ci-dessous :

| Type de cheveu | L | a | b | Couleur |
|---|---:|---:|---:|---|
| Cheveu gris naturel | 48,13 | 22,20 | 24,30 | Orange |
| Cheveu gris permanente | 43,22 | 27,05 | 28,72 | Orange |
| Cheveu décoloré | 40,49 | 43,09 | 41,00 | Orange |

**Exemple 11**

Etape 1 : prétraitement par une solution réductrice diluée

Des mèches de cheveux gris à 90% de cheveux blancs naturels, permanentés ou décolorés, sont immergées ou simplement imprégnées par une solution de réduction : acide thioglycolique à une concentration de 0.05 ; 0.2 ; 1 molaire (pH amené à8.5 ; immersion dans la solution pendant 10 min).

Etape 2 : application du colorant

Une formule aqueuse du colorant disulfure de l'exemple 3 ci dessus à $10^{-3}$ mol/100g en colorant dans un milieu tamponné à pH 9 (borate 0,05M) est appliquée sur les cheveux prétraités suivant l'étape 1 décrite ci-dessus, à raison de 5 g de solution par gramme de cheveu, pendant 20 minutes à température ambiante.

Les cheveux sont abondamment rincés à l'eau courante puis séchés.

Des mèches ont aussi été traitées par la formule aqueuse contenant le colorant disulfure de l'exemple 3 sans étape de pré-traitement par une solution réductrice.

Les mèches sont évaluées avant et après la teinture dans le système L*a*b*, selon la méthode de l'exemple 5.

Les mèches colorées sont soumises à 12 shampooings selon un cycle qui comprend le mouillage des mèches à l'eau, le lavage aux shampooings, un rinçage à l'eau suivi d'un séchage.

La couleur des mèches avant et après les 12 lavages a été évaluée dans le système L*a*b*. La variation de la couleur avant et après lavages a été mesurée par ΔE selon l'équation ci dessus à partir des valeurs de L0*a0*b0* des mèches colorées et des valeurs de L*a*b* obtenues après 12 shampooings.

$$\Delta E = \sqrt{(L^* - L_o{}^*)^2 + (a^* - a_o{}^*)^2 + (b^* - b_o{}^*)^2}$$

Plus la valeur de ΔE est importante, plus la différence de couleur avant et après lavages est importante, et dans le cas présent, moins la coloration est résistante aux shampooings.

Les résultats sont regroupés dans le tableau ci-dessous :

**Application sans traitement réducteur**

| Type de cheveu | * | L* | a* | b* | DE* |
|---|---|---|---|---|---|
| Cheveu gris naturel | 0 | 34,1 | 32,5 | 15,1 | |
| Cheveu gris naturel | 3 | 32,3 | 34,3 | 15,4 | 2,5 |
| Cheveu gris naturel | 12 | 41,9 | 34,0 | 13,2 | 8,2 |
| * nombre de shampooings effectués sur les mèches avant la mesure de couleur | | | | | |

**Applications avec traitement réducteur préalable (étape 1)**

| Type de cheveu | * | L* | a* | b* | DE* |
|---|---|---|---|---|---|
| Cheveu gris naturel | 0 | 28,826 | 26,217 | 17,448 | |
| Cheveu gris naturel | 3 | 30,576 | 24,596 | 15,692 | 2,9 |

(suite)

| Type de cheveu | * | L* | a* | b* | DE* |
|---|---|---|---|---|---|
| Cheveu gris naturel | 12 | 31,566 | 29,86 | 17,108 | 4,6 |
| * nombre de shampooings effectués sur les mèches avant la mesure de couleur | | | | | |

Ces résultats montrent que la composition de l'invention présente une bonne résistance aux lavages, en particuliers lorsque le procédé de coloration comprend un pré-traitement réducteur.

## Revendications

**1.** Procédé de coloration des fibres kératiniques humaines **caractérisé en ce qu'**on applique sur les fibres, une composition tinctoriale, comprenant dans un milieu cosmétique approprié, au moins un colorant disulfure choisi parmi les colorants de formules (I), (II), (III) ou (IV) suivantes :

$$A-(X)_p-C_{sat}-S\text{-}S-C_{sat}-(X)_p-A \qquad (I)$$

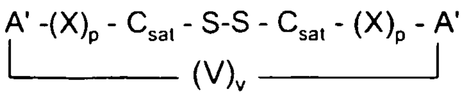

(II)

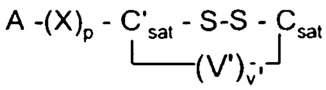

(III)

$$A-(X)_p-C_{sat}-S\text{-}S-C_{sat}-(X)_p-D \qquad (IV)$$

leurs sels, isomères solvates tels que hydrates,
formules dans lesquelles :

- A, et A', identiques ou différents, représentent un radical contenant au moins un chromophore cationique ou non;
- V et V', identiques ou différents représentent un groupe pontant
- v et v', identiques ou différents représentent 0 ou 1 ;
- X, identiques ou non, représentent une chaîne hydrocarbonée en $C_1$-$C_{30}$, linéaire ou ramifiée, saturée ou insaturée, éventuellement interrompue et/ ou éventuellement terminée à l'une ou deux de ses extrémités par un ou plusieurs groupes divalents ou leur combinaisons choisis parmi :

  - N(R)-, -$N^+$(R)(R)-, -O-, -S-, -CO-, -$SO_2$- avec R, identiques ou différents, choisi parmi un hydrogène, un radical alkyle en C1-C4, hydroxyalkyle, aminoalkyle
  - un radical (hétéro)cyclique aromatique ou non, saturé ou insaturé, condensé ou non comprenant éventuellement un ou plusieurs hétéroatomes identiques ou non, éventuellement substitué;

- le coefficient p est égal à 0 ou 1 ;
- $C_{sat}$ $C'_{sat}$, identiques ou différents représentent une chaîne alkylène en $C_1$-$C_{18}$, linéaire ou ramifiée, éventuellement substituée, éventuellement cyclique ;
- D correspond à un radical choisi parmi les radicaux hydroxy, hydroxyalkyle, alcoxy, carboxyliques, carboxylates,

amino, alkylamino, dialkylamino.

**2.** Procédé selon la revendication 1, **caractérisé en ce que** dans les formules (I), (II), (III) ou (IV), lorsque p est égal à1, X représente la séquence suivante :

$$-(T)_t-(Y)_y-(Z)_z-$$

ladite séquence étant reliée dans les formules (I), (II), (III) ou (IV), comme suit :

- $C_{sat}$ (ou $C'_{sat}$)-$(T)_t$-$(Y)_y$-$(Z)_z$-(A ou A') ; dans laquelle
T représente un ou plusieurs radicaux ou leurs combinaisons choisis parmi $-SO_2-$, -O-, -S-, -N(R)-, -N+(R)(R) -CO-, R représentant un atome d'hydrogène, un radical alkyle en $C_1$-$C_4$, ou hydroxyalkyle en C1-C4;
le coefficient **t** vaut 0 ou 1 ;
**Y** représente :

  - un radical choisi parmi $-(CH_2)_2-SO_2-$ ; $-CH_2-CHR-CO-NR'-$ avec R, R', identiques ou non, représentant un atome d'hydrogène, un radical alkyle en $C_1$-$C_4$.
  - un groupement de formule (a), (a') ou (a") :

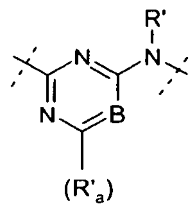
(a)
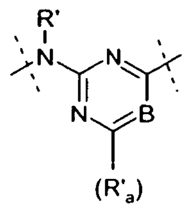
(a')
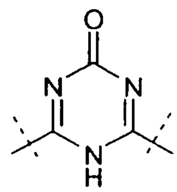
(a")

dans laquelle

• B représente -N-, $-CR_a$, avec $R_a$ représentant un atome d'hydrogène, un atome d'halogène choisi parmi le chlore ou le fluor, un groupement nitro, un groupement pyridinium éventuellement substitué;
• R' a la même définition que précédemment
• $R'_a$ représente :

- un atome d'hydrogène
- un atome de chlore ou un atome de fluor
- un groupement pyridinium éventuellement substitué par au moins un groupement $R_c$, $R_c$ prouvant être un groupement alkyle en $C_1$-$C_4$, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C1-C18, éventuellement porteurs d'au moins un hydroxyle) ; un groupement ester $-COOR_d$ avec $R_d$ représentant un radical alkyle en $C_1$-$C_4$ ; un groupement amide $-CON(R_d)_2$ avec $R_d$ identiques ou non, représentant un atome d'hydrogène ou un radical alkyle en $C_1$-$C_4$ ;
- un groupement hydroxyle
- un groupement amino, alkylamino ou dialkylamino, les groupements alkyles identiques ou différents, en C1-C18, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C1-C10

- un groupement de formule (b) suivante :

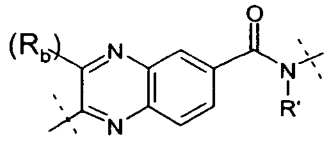 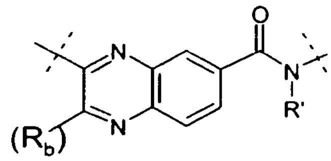

dans laquelle

- R' a la même définition que précédemment
- $R_b$ représente

  - un atome de chlore
  - un groupement amino, alkylamino ou dialkylamino, les groupements alkyles identiques ou différents, en $C_1$-$C_{18}$, étant linéaires ou ramifiés, éventuellement interrompus par un hétéroatome choisi parmi N, O, S, éventuellement substitués par un ou plusieurs hydroxyles
  - un hétérocycle azoté, saturé ou insaturé, pouvant être substitué
  - un groupement arylamino, dans lequel de préférence le radical aryle est en $C_6$ ;

**y** vaut 0 ou 1 ;
**Z** représente

- $-(CH_2)_m-$ avec m entier compris entre 1 et 8
- $-(CH_2CH_2O)_q-$ ou $-(OCH_2CH_2)_q-$ dans lesquelles q est un entier compris entre 1 et 15
- un radical aryle, alkylaryle ou arylalkyle dont le radical alkyle est en $C_1$-$C_4$ et le radical aryle est de préférence en $C_6$, étant éventuellement substitué par au moins un groupement $SO_3M$ avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle

**z** vaut 0 ou 1.

**3.** Procédé selon la revendication 1 ou 2, **caractérisé en ce que** Y représente :

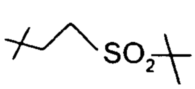 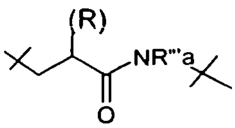 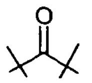 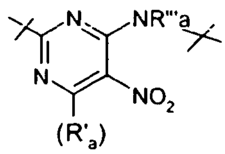 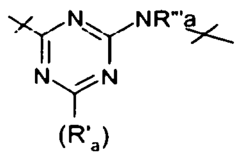

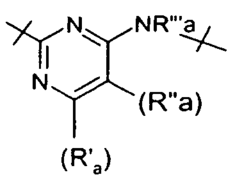 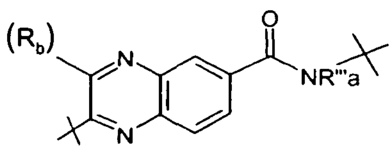 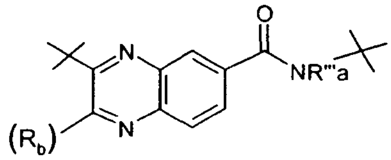

dans lesquels les radicaux R, $R'_a$ et $R_b$ sont définis comme précédemment ; $R''_a$, a la même définition que $R'_a$, indépendamment les uns des autres ; $R'''_a$ représente un atome d'hydrogène ou un radical alkyle.

**4.** Procédé selon l'une quelconque des revendications précédentes, **caractérisé en ce** Z représente :

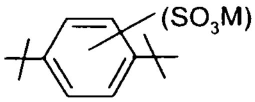 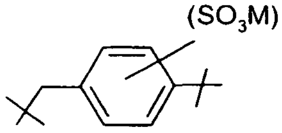 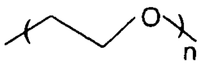 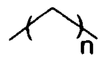

5. Procédé selon l'une quelconque des revendications précédentes, **caractérisé en ce que** le colorant disulfure est tel que v est égal à 0.

6. Procédé selon l'une quelconque des revendications précédentes, **caractérisé en ce que**, dans les formules (I), (II), (III) ou (IV), A et A' représentent un radical comprenant au moins un chromophore issu de colorants de type acridines, acridones, anthranthrones, anthrapyrimidines, anthraquinones, azines, azoïques, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines, diazines, dicétopyrrolopyrroles, dioxazines, diphénylamines, diphénylméthanes, dithiazines, flavonoïdes, fluorindines, formazans, hydrazones, hydroxycétones, indamines, indanthrones, indigoides et pseudo-indigoïdes, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes.

7. Procédé selon la revendication 6, **caractérisé en ce que**, dans les formules (I), (II), (III) ou (IV), A et A' représentent un radical comprenant un chromophore choisi parmi les chromophores de type azos, anthraquinones et hydrazones.

8. Procédé selon l'une quelconque des revendications 1 à 7, dans lequel le colorant disulfure est choisi parmi

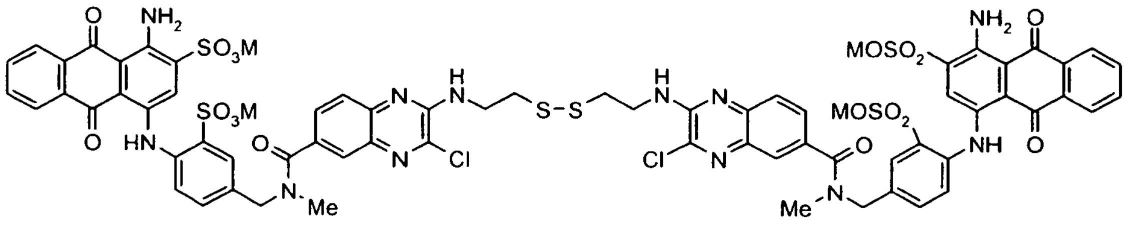

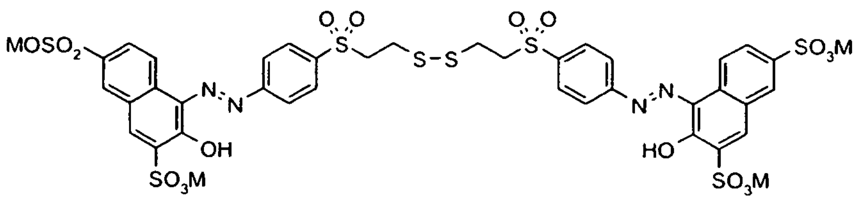

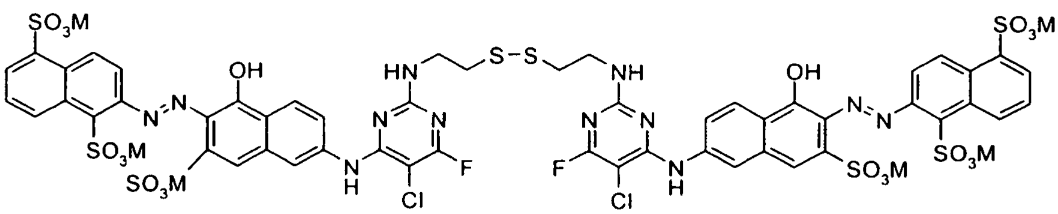

avec M représentant un atome d'hydrogène, un métal alcalin ou un groupement ammonium ou un groupement

ammonium substitué par un ou plusieurs radicaux alkyle, identiques ou non, linéaires ou ramifiés, en $C_1$-$C_{10}$ éventuellement porteurs d'au moins un hydroxyle, ainsi que les composés suivants, sous forme acide, basique ou neutralisée :

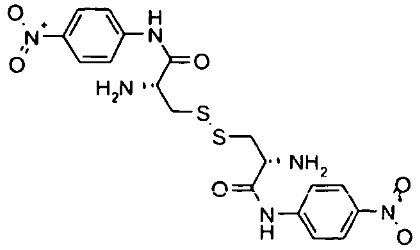
(1)

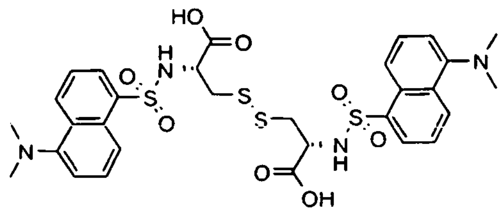
(2)

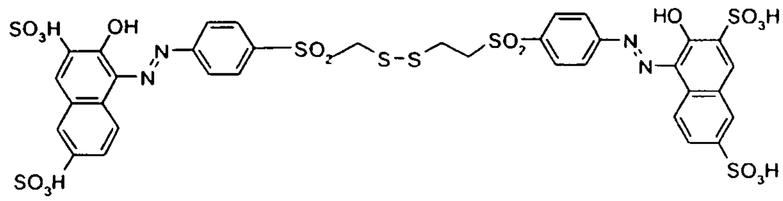
(3)

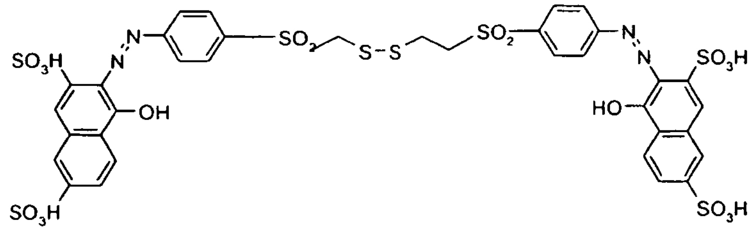
(4)

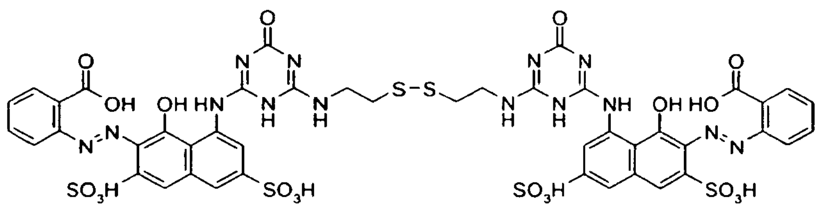
(5)

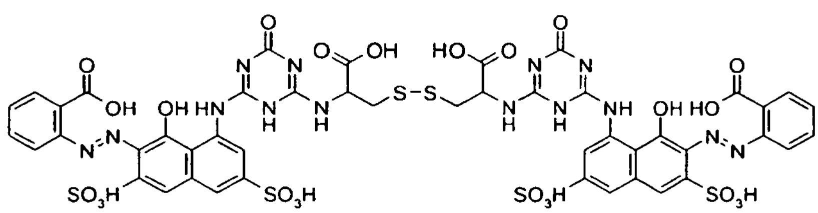
(6)

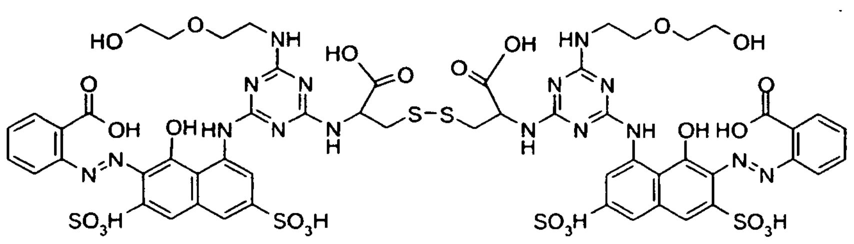

(7)

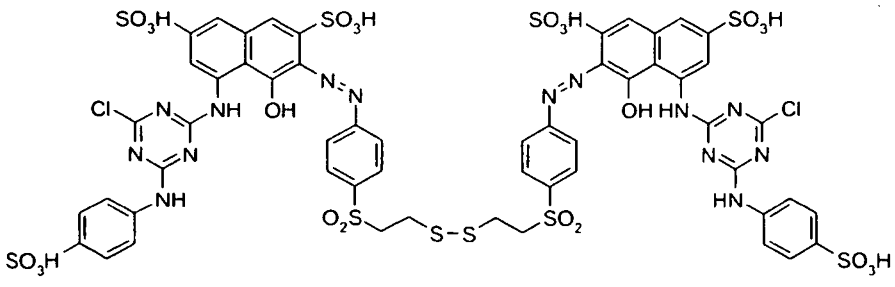

(8)

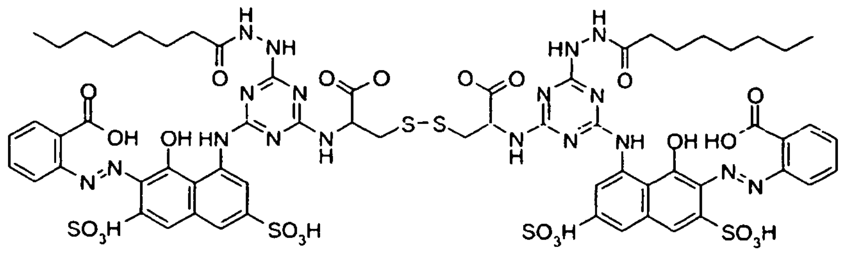

(9)

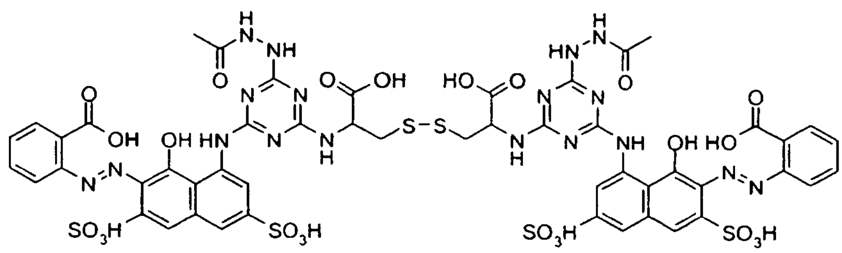

(10)

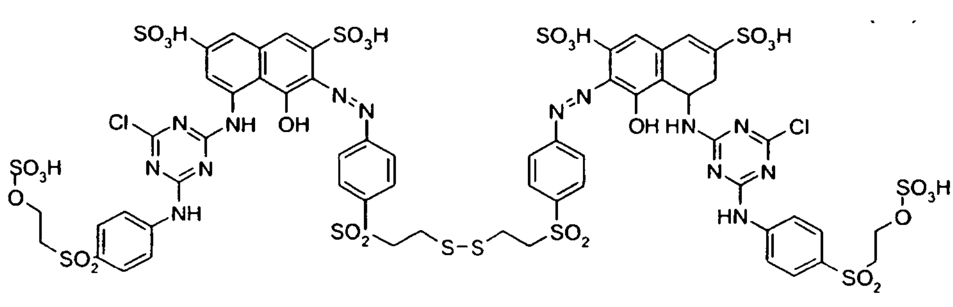

(11)

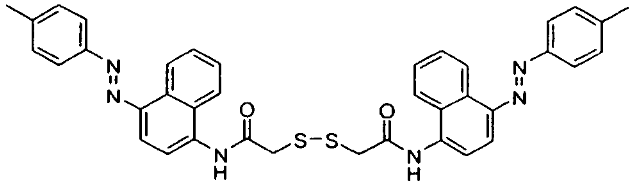

(12)

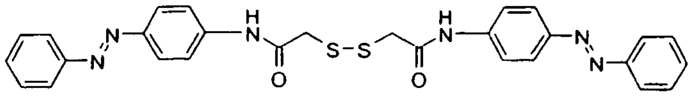

(13)

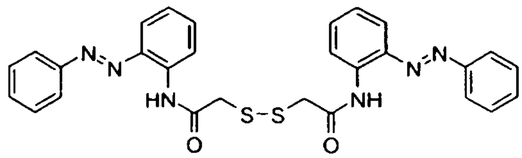

(14)

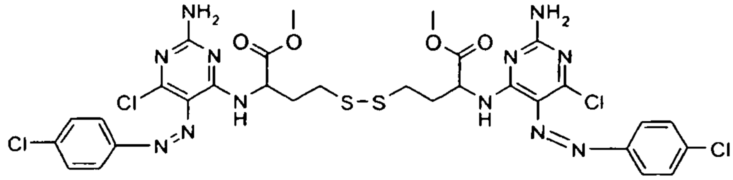

(15)

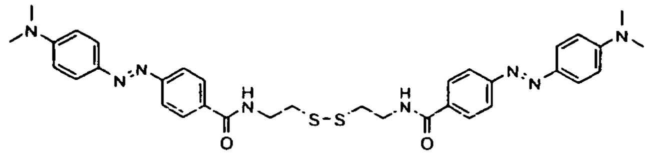

(16)

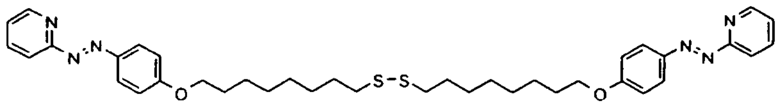

(17)

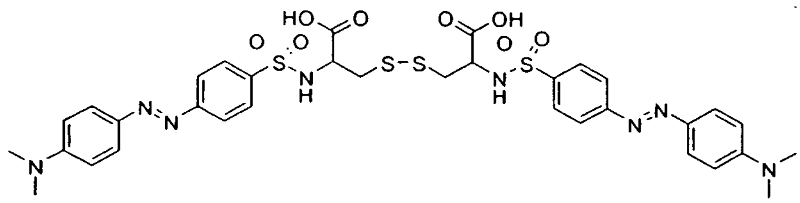

(18)

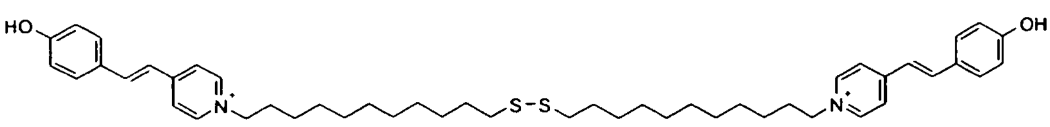

(21)

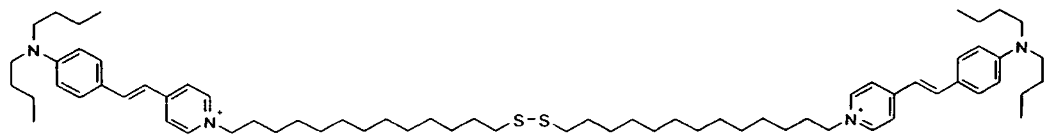

(22)

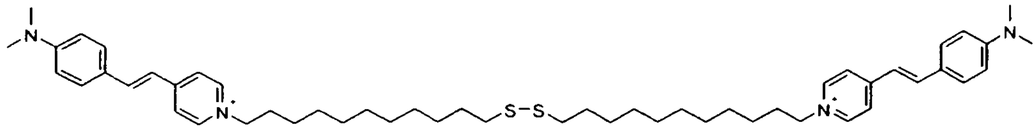

(23)

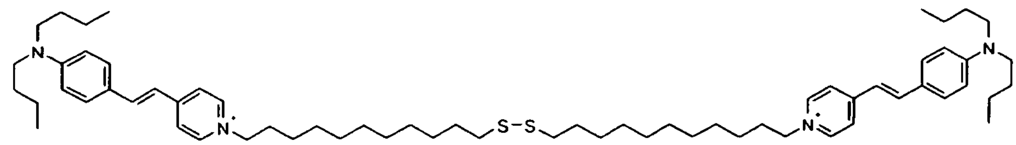

(24)

**9.** Procédé selon la revendication 7, **caractérisé en ce que**, dans les formules (I), (II), (III) ou (IV), A et A' comprennent un chromophore cationique.

**10.** Procédé selon la revendication 9, dans lequel le chromophore cationique comprend un radical cationique qui est un ammonium quaternaire.

**11.** Procédé selon l'une quelconque des revendications 9 ou 10, **caractérisé en ce que** le colorant disulfure est tel que **A** représente W-N=N-Ar- ou -W-N=N-Ar, avec W représentant un hétérocycle aromatique ou non, condensé ou non, comprenant un ammonium quaternaire ; Ar représente un radical aryle en $C_5$, $C_6$, ou un bicycle aromatique de type naphtyle, éventuellement substitués par un ou plusieurs atomes d'halogène, de préférence chlore, fluor ; par un ou plusieurs groupements alkyle, de préférence en $C_1$-$C_4$ ; par un ou plusieurs groupements hydroxyle ; par un ou plusieurs groupements alcoxy, par un ou plusieurs groupements hydroxyalkyle, par un ou plusieurs groupements amino ou (di)alkylamino, de préférence avec la partie alkyle en $C_1$-$C_4$

**12.** Procédé selon la revendication 11, **caractérisé en ce que** le colorant disulfure est tel que p est égal à 1, y et z sont égaux à zéro et T représente -N(R)- en position para sur Ar par rapport à la fonction azoïque.

**13.** Procédé selon l'une quelconque des revendications 11 ou 12, **caractérisé en ce que** W est un imidazolium, pyridinium, benzimidazolium, pyrazolium, benzothiazolium, éventuellement substitués par un ou plusieurs radicaux alkyles, identiques ou non, en $C_1$-$C_4$.

**14.** Procédé selon l'une quelconque des revendications précédentes **caractérisé en ce que** le colorant disulfure est choisi parmi :

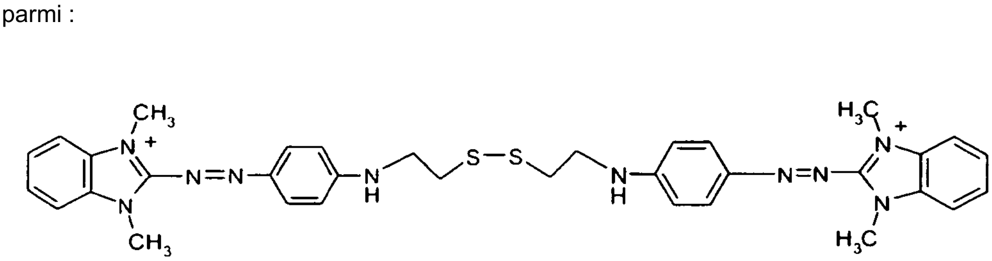

2M'

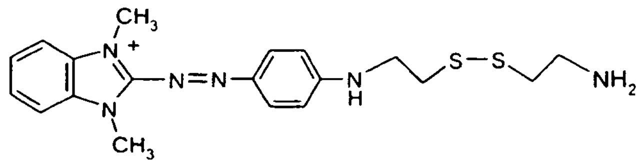

M'

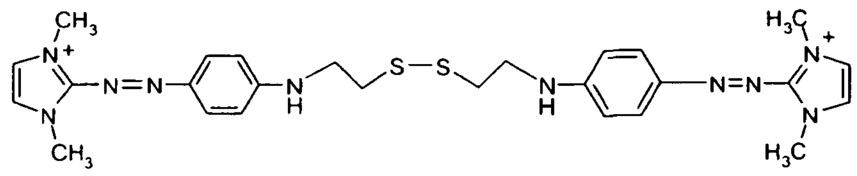

2M'

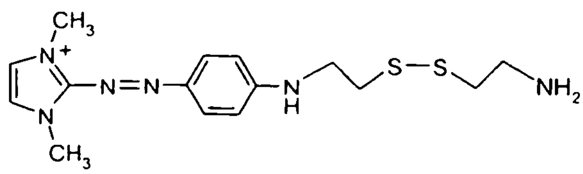

M'

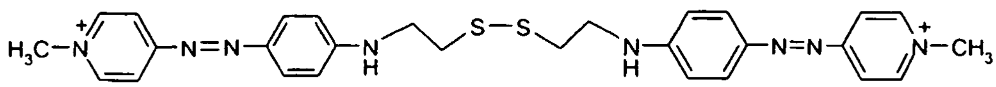

2M'

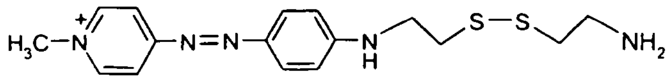

M'

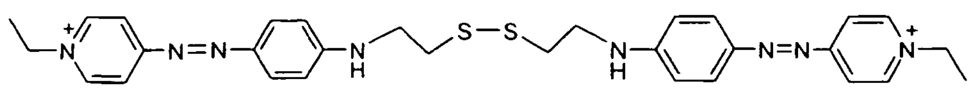

2M'

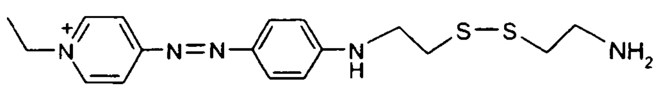

M'

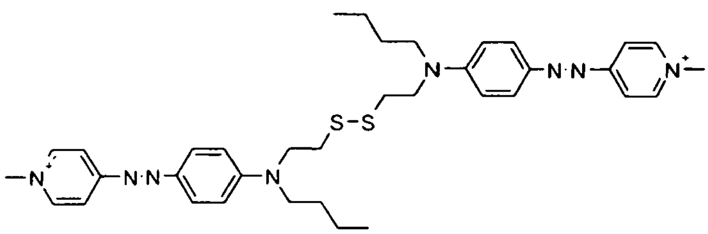

2M'

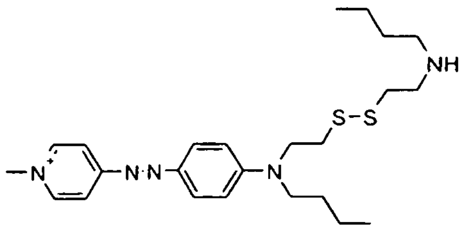

M'

**15.** Procédé selon l'une quelconque des revendications précédentes dans lequel la composition contient un agent réducteur.

**16.** Procédé selon l'une quelconque des revendications 1 à 14 comprenant un pré-traitement avec un agent réducteur.

**17.** Procédé selon l'une quelconque des revendications 1 à 14 comprenant un post-traitement avec un agent réducteur.

**18.** Procédé selon la revendication 15 à 17 dans lequel l'agent réducteur est choisi parmi les thiols, les phosphines, le bisulfite, les sulfites.

**19.** Procédé selon la revendication précédente , **caractérisé en ce que** l'agent réducteur est choisi l'acide thioglycolique, la cystéine, l'homocystéine, l'acide thiolactique, les sels de ces thiols.

**20.** Procédé selon la revendication 15 à 17 dans lequel l'agent réducteur est choisi parmi les borohydrures ou leurs dérivés, comme par exemple les sels du borohydrure, du cyanoborohydrure, du triacétoxyborohydrure, du trimethoxyborohydrure : sels de sodium, lithium, potassium, calcium, ammonium quaternaires comme tétraméthylammonium, tétraéthylammonium, tétra n-butylammonium, benzyltriethylammonium ; le catécholborane.

**21.** Procédé selon l'une quelconque des revendications 1 à 14, dans lequel la composition comprend un agent oxydant.

**22.** Procédé selon l'une quelconque des revendications 1 à 14 comprenant un post-traitement avec un agent oxydant.

**23.** Procédé selon l'une quelconque des revendications 1 à 14 comprenant une étape de post traitement conditionneur éventuellement associé à un post-traitement oxydant.

**24.** Procédé selon l'une quelconque des revendications 21 ou 22, dans lequel l'agent oxydant est choisi parmi le peroxyde d'hydrogène, le peroxyde d'urée, les bromates de métaux alcalins, les persels tels que les perborates et les persulfates, ainsi que les enzymes.

**25.** Procédé selon l'une quelconque des revendications précédentes, dans lequel le colorant disulfure est présent en quantité comprise entre 0,001 et 50% en poids, de préférence, entre 0,005 et 20% en poids et encore plus préférentiellement entre 0,01 et 5% en poids, par rapport au poids total de la composition.

**26.** Procédé selon l'une quelconque des revendications précédentes dans lequel la composition contient au moins un composé disulfure additionnel différent du composé de formule (I), (II), (III) ou (IV).

**27.** Procédé selon la revendication précédente, **caractérisé en ce que** le composé disulfure autre que les composés de formule (I), (II), (III) ou (IV) est choisi parmi les composés comprenant au moins une chaîne grasse.

**28.** Procédé selon l'une quelconque des revendications précédentes, dans lequel la composition comprend au moins une base d'oxydation, un coupleur et/ou un colorant direct autre qu'un colorant disulfure.

**29.** Composition tinctoriale comprenant dans un milieu cosmétique approprié, un colorant disulfure de formules (I), (II), (III) ou (IV) telle que définie précédemment aux revendications 1 à 14.

**30.** Composition selon la revendication 29 dans laquelle le colorant disulfure de formule (I) est différent du colorant suivant :

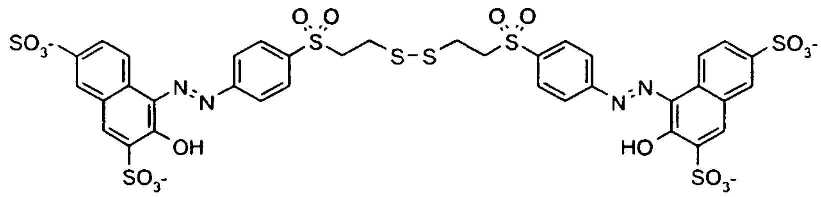

**31.** Composition selon l'une quelconque des revendications 29 et 30 dans laquelle le colorant disulfure est tel que A représente W-N=N-Ar- ou -W-N=N-Ar, avec W représentant un hétérocycle aromatique ou non, condensé ou non, comprenant un ammonium quaternaire ; Ar représente un radical aryle en $C_5$, $C_6$, ou un bicycle aromatique de type naphtyle, éventuellement substitués par un ou plusieurs atomes d'halogène, de préférence chlore, fluor ; par un ou plusieurs groupements alkyle, de préférence en $C_1$-$C_4$ ; par un ou plusieurs groupements hydroxyle ; par un ou plusieurs groupements alcoxy, par un ou plusieurs groupements hydroxyalkyle, par un ou plusieurs groupements amino ou (di)alkylamino, de préférence avec la partie alkyle en $C_1$-$C_4$

**32.** Composition selon la revendication précédente, **caractérisée en ce que** le colorant disulfure est tel que p est égal à 1, y et z sont égaux à zéro et T représente -N(R)-.

**33.** Composition selon l'une quelconque des revendications 31 ou 32, **caractérisé en ce que** W est un imidazolium, pyridinium, benzimidazolium, pyrazolium, benzothiazolium, éventuellement substitués par un ou plusieurs radicaux alkyles, identiques ou non, en $C_1$-$C_4$.

**34.** Composition selon l'une quelconque des revendications 29 à 33 comprenant au moins une base d'oxydation, un coupleur et/ou un colorant direct autre qu'un colorant disulfure.

**35.** Composition selon l'une quelconque des revendications 29 à 34 comprenant au moins un agent oxydant.

**36.** Composition selon l'une quelconque des revendications 29 à 35 comprenant un solvant organique et/ou un agent épaississant.

**37.** Dispositif à plusieurs compartiments dans lequel un premier compartiment contient une composition tinctoriale comprenant un colorant disulfure tel que défini aux revendications 1 à 13 et un deuxième compartiment contient un agent réducteur capable de réduire une liaison disulfure.

**38.** Dispositif selon la revendication 37 comprenant un troisième compartiment qui contient un agent oxydant.

**39.** Utilisation des colorants disulfure tels que définis aux revendications 1 à 14 pour la teinture de fibres kératiniques humaines, en particulier les cheveux.

**40.** Utilisation selon la revendication 39 pour améliorer la ténacité de la coloration.

**41.** Colorant disulfure cationique comprenant au moins un radical ammonium quaternaire, de formules (I), (II), (III) ou (IV) suivantes, :

$$A-(X)_p-C_{sat}-S\text{-}S-C_{sat}-(X)_p-A \qquad (I)$$

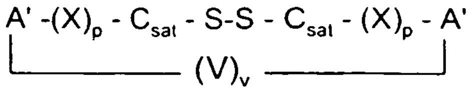

(II)

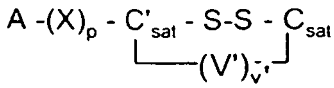

(III)

$$A-(X)_p-C_{sat}-S\text{-}S-C_{sat}-(X)_p-D \qquad (IV)$$

leurs sels, isomères solvates tels que hydrates,
formules dans lesquelles :

- A, et A', identiques ou différents, représentent un radical contenant au moins un chromophore cationique-; ce chromophore appartenant aux familles azoïques, anthraquinones, ou hydrazones ;
- V et V', identiques ou différents représentent un groupe pontant ;
- v et v', identiques ou différents représentent 0 ou 1 ;
- X représente la séquence suivante :

$$-(T)_t-(Y)_y-(Z)_z-$$

ladite séquence étant reliée dans les formules (I), (II), (III) ou (IV), comme suit :

- $C_{sat}$ (ou $C'_{sat}$)-$(T)_t$-$(Y)_y$-$(Z)_z$-(A ou A') ; dans laquelle
T représente un ou plusieurs radicaux ou leurs combinaisons choisis parmi $-SO_2-$, -O-, -S-, -N(R)-, -N+(R)(R) -CO-, R représentant un atome d'hydrogène, un radical alkyle en $C_1$-$C_4$, ou hydroxyalkyle en $C_1$-$C_4$; le coefficient t vaut 0 ou 1 ;
Y représente :
- un radical choisi parmi $-(CH_2)_2-SO_2-$ ; $-CH_2-CHR-CO-NR'-$ avec R, R', identiques ou non, représentant un atome d'hydrogène, un radical alkyle en $C_1$-$C_4$.
- un groupement de formule (a), (a') ou (a") :

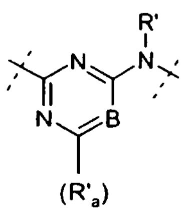
(a)

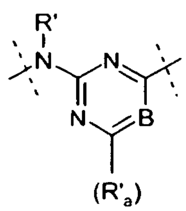
(a')

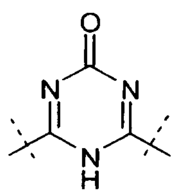
(a")

dans laquelle

- B représente -N-, $-CR_a$, avec $R_a$ représentant un atome d'hydrogène, un atome d'halogène choisi parmi le chlore ou le fluor, un groupement nitro, un groupement pyridinium éventuellement substitué;
- R' a la même définition que précédemment
- $R'_a$ représente :

- un atome d'hydrogène
- un atome de chlore ou un atome de fluor
- un groupement pyridinium éventuellement substitué par au moins un groupement $R_c$, $R_c$ prouvant être un groupement alkyle en $C_1$-$C_4$, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en $C_1$-$C_{18}$, éventuellement porteurs d'au moins un hydroxyle) ; un groupement ester $-COOR_d$ avec $R_d$ représentant un radical alkyle en $C_1$-$C_4$ ; un groupement amide $-CON(R_d)_2$ avec $R_d$ identiques ou non, représentant un atome d'hydrogène ou un radical alkyle en $C_1$-$C_4$ ;
- un groupement hydroxyle
- un groupement amino, alkylamino ou dialkylamino, les groupements alkyles identiques ou différents, en $C_1$-$C_{18}$, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en $C_1$-$C_{10}$
- un groupement de formule (b) suivante :

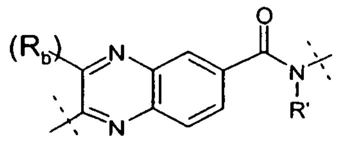 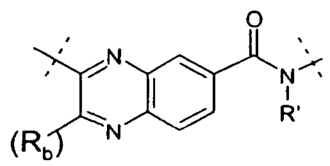

dans laquelle

- R' a la même définition que précédemment
- $R_b$ représente

  - un atome de chlore
  - un groupement amino, alkylamino ou dialkylamino, les groupements alkyles identiques ou différents, en $C_1$-$C_{18}$, étant linéaires ou ramifiés, éventuellement interrompus par un hétéroatome choisi parmi N, O, S, éventuellement substitués par un ou plusieurs hydroxyles
  - un hétérocycle azoté, saturé ou insaturé, pouvant être substitué

- un groupement arylamino, dans lequel de préférence le radical aryle est en $C_6$ ;
- le coefficient p est égal à 1 ;
- **y** vaut 0 ou 1 ;
- **Z** représente :

  - - -$(CH_2)_m$- avec m entier compris entre 1 et 8
  - - -$(CH_2CH_2O)_q$- ou -$(OCH_2CH_2)_q$- dans lesquelles q est un entier compris entre 1 et 15
  - un radical aryle, alkylaryle ou arylalkyle dont le radical alkyle est en $C_1$-$C_4$ et le radical aryle est de préférence en $C_6$, étant éventuellement substitué par au moins un groupement $SO_3M$ avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle

- **z** vaut 0 ou 1.
- $C_{sat}$, $C'_{sat}$, identiques ou différents représentent une chaîne alkylène en $C_1$-$C_{18}$, linéaire ou ramifiée, éventuellement substituée, éventuellement cyclique ;
- D correspond à un radical choisi parmi les radicaux hydroxy, hydroxyalkyle, alcoxy, carboxyliques, carboxylates, amino, alkylamino, dialkylamino.

**42.** Colorant selon la revendication 41 **caractérisé en ce que** A ou A' identiques ou différents contiennent un chromophore azoïque.

**43.** Colorant selon l'une quelconque des deux revendications précédentes, **caractérisé en ce que** le colorant disulfure est tel que y et z sont égaux à zéro et T représente -N(R)-.

**44.** Colorant selon l'une quelconque des revendications 41 à 43, **caractérisé en ce que** W est un imidazolium, pyridinium, benzimidazolium, pyrazolium, benzothiazolium, éventuellement substitués par un ou plusieurs radicaux alkyles, identiques ou non, en $C_1$-$C_4$.

**45.** Colorant selon l'une quelconque des revendications 41 à 44, **caractérisé en ce que** le colorant disulfure est choisi parmi :

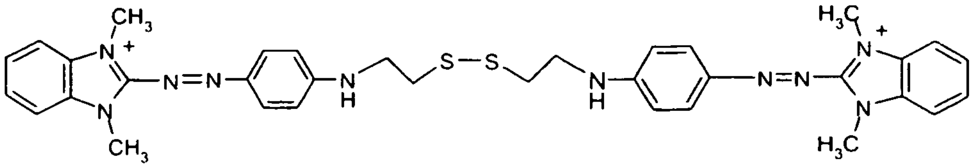

2M'

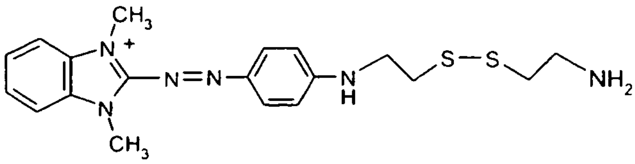

M'

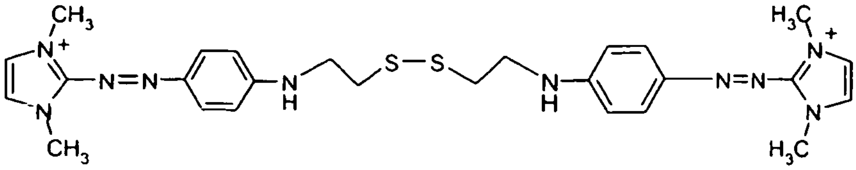

2M'

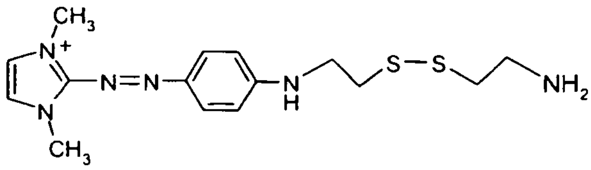

M'

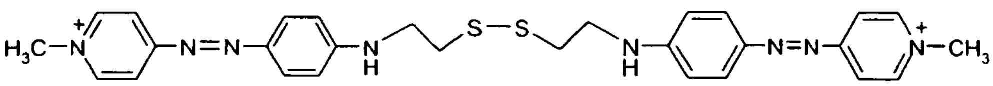

2M'

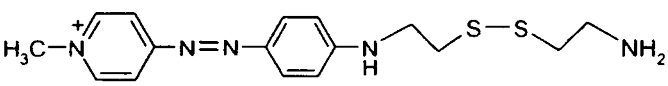

M'

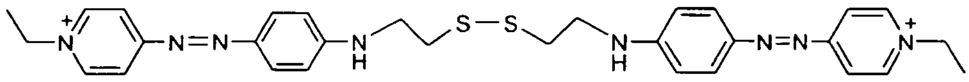

2M'

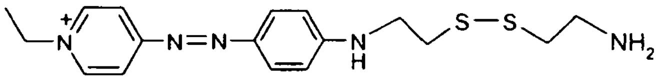

M'

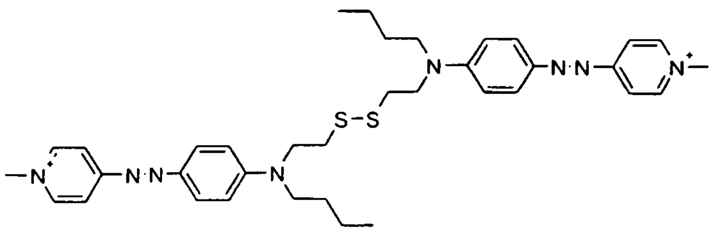

2M'

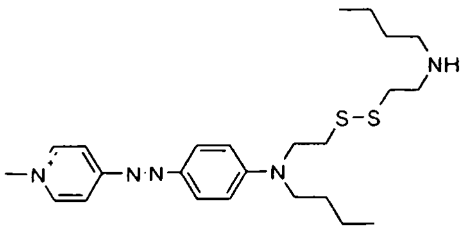

M'

**46.** Colorant de formules suivantes :

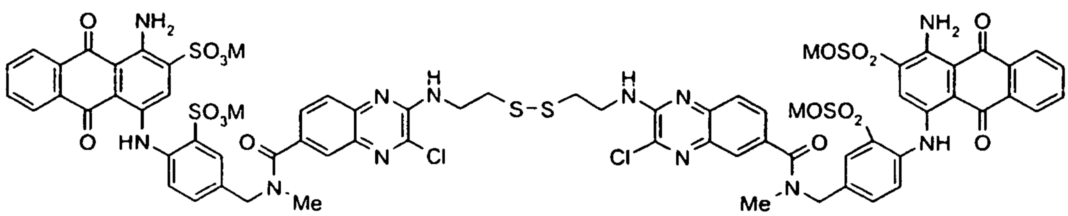

(17)

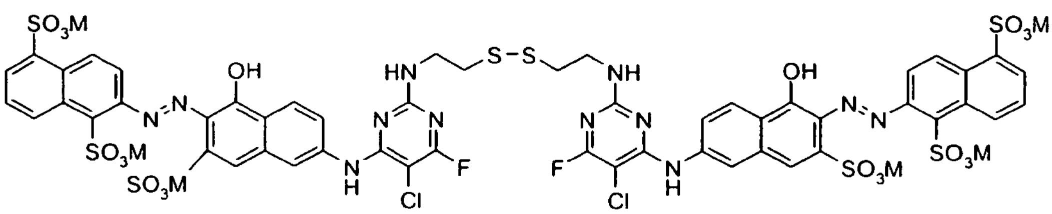

(18)

M représentant un atome d'hydrogène, un métal alcalin ou un groupement ammonium ou un groupement ammonium substitué par un ou plusieurs radicaux alkyle, identiques ou non, linéaires ou ramifiés, en $C_1$-$C_{10}$ éventuellement porteurs d'au moins un hydroxyle, ainsi que les composés suivants, sous forme acide, basique ou neutralisée.

**Claims**

1. Method for dyeing human keratin fibres, **characterized in that** one applies to the fibres a dyeing composition comprising, in an appropriate cosmetic medium, at least one disulphide dye chosen from the dyes of the following formulae (I), (II), (III) or (IV) :

$$A-(X)_p-C_{sat}-S\text{-}S-C_{sat}-(X)_p-A \qquad (I)$$

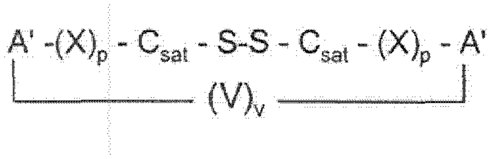

(II)

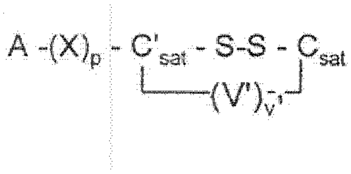

(III)

$$A-(X)_p-C_{sat}-S\text{-}S-C_{sat}-(X)_p-D \qquad (IV)$$

their salts, isomers and solvates such as hydrates, in which formulae:

- A and A', which identical or different, represent a radical containing at least one cationic or noncationic chromophore;
- V and V', which are identical or different, represent a bridging groups;
- v and v', which are identical or different, represents 0 or 1;
- X, which are identical or different, represents a saturated or unsaturated, linear or branched $C_1$-$C_{30}$ hydrocarbon chain optionally interrupted and/or optionally terminated at one or both of its ends by or more divalent groups or combinations thereof, chosen from:

  - -N(R)-, -$N^+$(R)(R)-, -O-, -S-, -CO-, -$SO_2$- with R, which are identical or different, being chosen from hydrogen, a $C_1$-$C_4$ alkyl, hydroxyalkyl or aminoalkyl radical;
  - an optionally substituted, saturated or unsaturated, fused or nonfused, aromatic or nonaromatic (hetero) cyclic radicals optionally comprising one or more identical or different heteroatoms;

- the coefficient p is equal to 0 or 1;
- $C_{sat}$, $C'_{sat}$, which are identical or different, represent an optionally substituted, optionally cyclic, linear or branched $C_1$-$C_{18}$ alkylene chain;
- D corresponds to a radical chosen from hydroxyl, hydroxyalkyl, alkoxy, carboxyl, carboxylate, amino, alkylamino and dialkylamino radicals.

2. Method according to claim 1, **characterized in that** in formulae (I), (II), (III) or (IV), when p is equal to 1, X represents the following sequence:

$$-(T)_t-(Y)_y-(Z)_z-$$

the said sequence being linked in formulae (I), (II), (III) or (IV) as follows:

- $C_{sat}$ (or $C'_{sat}$)-$(T)_t$-$(Y)_y$-$(Z)_z$-(A or A'); in which **T** represents one or more radicals or combinations thereof, chosen from -$SO_2$-, -O-, -S-, -N(R)-,

-N+(R)(R)-CO-, R representing a hydrogen atom, a $C_1$-$C_4$ alkyl radical or a $C_1$-$C_4$ hydroxyalkyl radical;
the coefficient **t** is equal to 0 or 1;
**Y** represents;

- a radical chosen from $-(CH_2)_2-SO_2-$;
- $CH_2$-CHR-CO-NR'- with R, R', which are identical or different, representing a hydrogen atom, a $C_1$-$C_4$ alkyl radical.
- a group of formula (a), (a') or (a") :

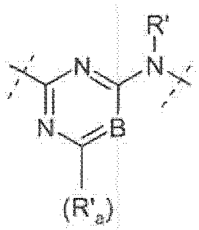
(a)

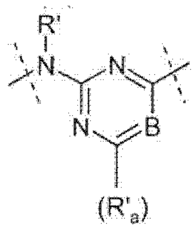
(a')

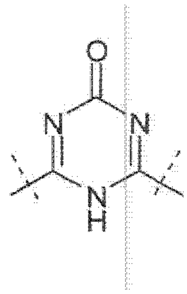
(a")

in which

- B represents -N-, $-CR_a$, with $R_a$ representing a hydrogen atom, a halogen atom from chlorine or fluorine, a nitro group, a pyridinium, which is optionally substituted;
- R' has the definition as above
- $R'_a$ represents:
    - a hydrogen atom
    - a chlorine atom or a fluorine atom
    - a pyridinium group which is optionally substituted with at least one group $R_c$, it being possibly for $R_c$ to be a $C_1$-$C_4$ alkyl groups, a halogen atom, a carboxyl group -COOM (with M representing a hydrogen atom, an alkali metal, an ammonium group or an ammonium groups substituted with one or more linear or branched, identical or different $C_1$-$C_{18}$ alkyl radicals, optionally bearing art least one hydroxyl); an ester group $-COOR_d$ with $R_d$ representing a $C_1$-$C_4$ alkyl radical; an amide group $-CON(R_d)_2$ with $R_d$, which are identical or different, representing a hydrogen atom or a $C_1$-$C_4$ alkyl radical;
    - a hydroxyl group
    - an amino, alkylamino or dialkylamino group, the identical or different $C_1$-$C_{18}$ alkyl groups being linear or branched, optionally interrupted by a heteroatom chosen from N, O, which are optionally substituted with one or more hydroxyl groups
    - a group NHNHCOR where R represents a linear or branched $C_1$-$C_{10}$ alkyl grout

- a group of the following formula (b):

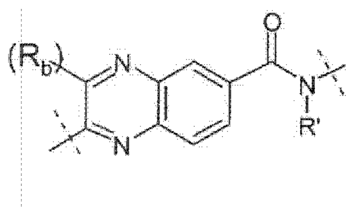

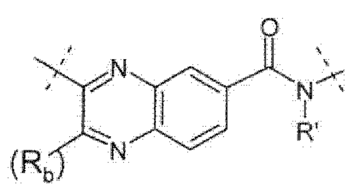

in which

- R' has the same definition as above
- $R_b$ represents
    - a chlorine atom

- an amino, alkylamino or dialkylamino group, the identical or different $C_1$-$C_{18}$ alkyl groups being linear or branched, optionally interrupted by a heteroatom chosen from N, O, S, which are optionally substituted with one or more hydroxyls
- a saturated or unsaturated nitrogen-containing heterocycle which may be substituted
- an arylamino group in which the aryl radical is preferable $C_6$;

**y** is equal to 0 or 1;
**z** represents
- $(CH_2)_m$- with m integer between 1 and 8
- -$(CH_2CH_2O)_q$- or -$(OCH_2CH_2)_q$- in which q is an integer between 1 and 15
- an aryl, alkylaryl or arylalkyl radicals whose alkyl radical is $C_1$-$C_4$ and the aryl radical is preferably $C_6$, being optionally substituted with at least one group $SO_3M$ with M representing a hydrogen atom, an alkali metal or an ammonium group or an ammonium group substituted with one or more identical or different, linear or branched $C_1$-$C_{18}$ alkyl radicals optionally bearing at least one hydroxyl

**z** is equal to 0 or 1.

**3.** Method according to Claim 1 or 2, **characterized in that** Y represents:

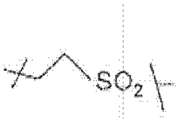
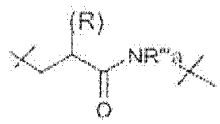
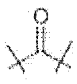
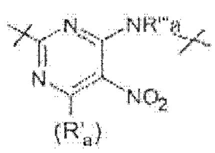
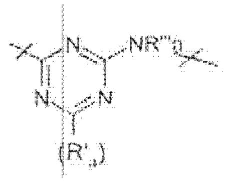
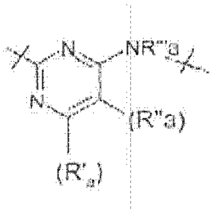
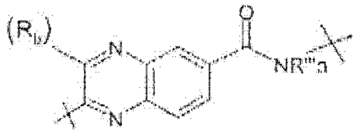
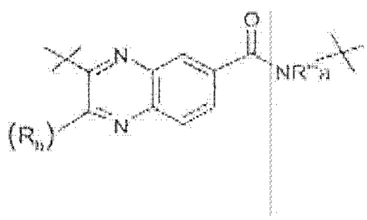

in which the radicals R, $R'_a$ and $R_b$ are as defined above; $R''_a$ has the same definition as $R'_a$, independently of each other; $R'''_a$ represents a hydrogen atom or an alkyl radical.

**4.** Method according to any one of the preceding claims, **characterized in that** Z represents:

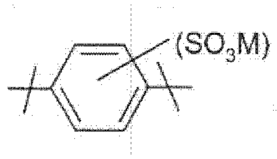
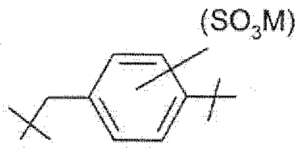
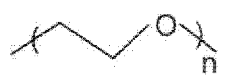
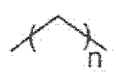

**5.** Method according to any one of the preceding claims, **characterized in that** the disulphide dye is such that v is equal to 0.

**6.** Method according to any one of the preceding claims, **characterized in that** in formulae (I), (II), (III) or (IV), A and A' represent a radicals comprising at least one chromophore derived from dyes of following type: acridines, acridones, anthranthrones, anthrapyrimidines, anthraquinones, azines, azos, azomethines, benzanthrones, benzimidazoles, benzimidazolones, benzindoles, benzoxazoles, benzopyrans, benzothiazoles, benzoquinones, bisazines, bisisoindolines, carboxanilides, coumarins, cyanins, diazines, diketopyrrolopyrroles, dioxazines, diphenylamines, diphenylmethanes, dithiazines, flavonoids, fluorindines, formazans, hydrazones, hydroxy ketones, indamines, indanthrones, indigoids and pseudo-indigoids, indophenols, indoanilines, isoindolines, isoindolinones, isoviolanthrones, lactones, methines, naphthalimides, naphthanilides, naphtholactams, naphthoquinones, nitro dyes, oxadiazoles, oxazines, perilones, perinones, perylenes, phenazines, phenothiazines, phthalocyanin, polyenes/carotenoids, porphyrins, pyranthrones, pyrazolanthrones, pyrazolones, pyrimidinoanthrones, pyronines, quinacridones, quinolines, quinophthalones, squaranes, stilbenes, tetrazoliums, thiazines, thioindigo, thiopyronines, triarylmethanes, xanthenes.

7. Method according to Claim 6, **characterized in that** in formulae (I), (II), (III) or (IV), A and A' represent a radical comprising a chromophore chosen from chromophores of the azo, anthraquinone and hydrazone type.

8. Method according to any one of Claims 1 to 7, in which the disulphide dye is chosen from

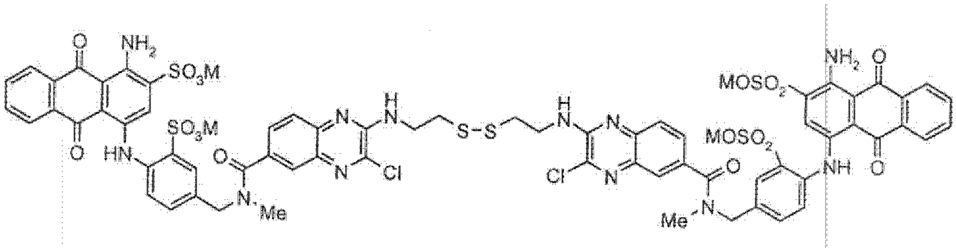

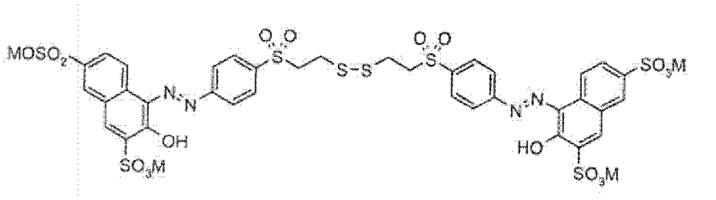

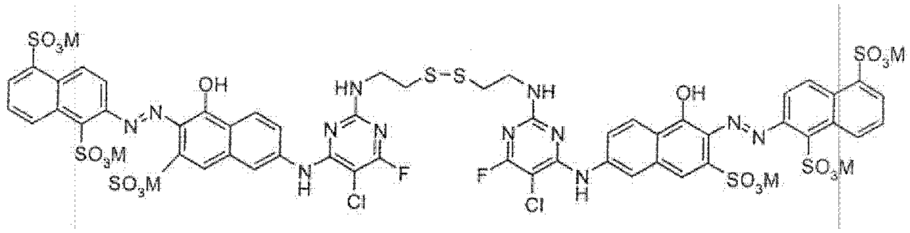

with M representing a hydrogen atom, an alkali metal or an group or an ammonium group substituted with one or more identical or different, linear or blanched $C_1$-$C_{10}$ alkyl radicals optionally bearing at least one hydroxyl, and the following compounds, in acidic, basic or neutralized form:

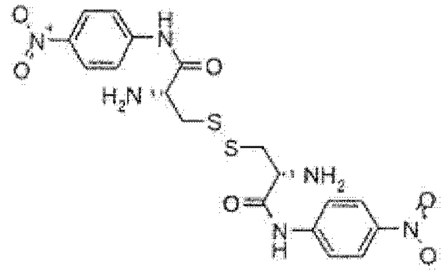

(1)

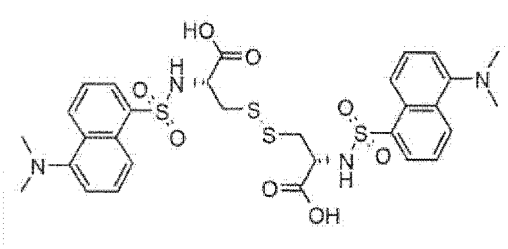

(2)

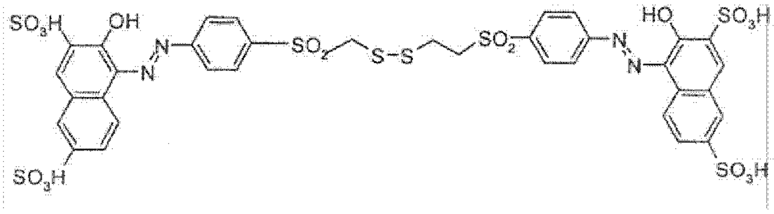

(3)

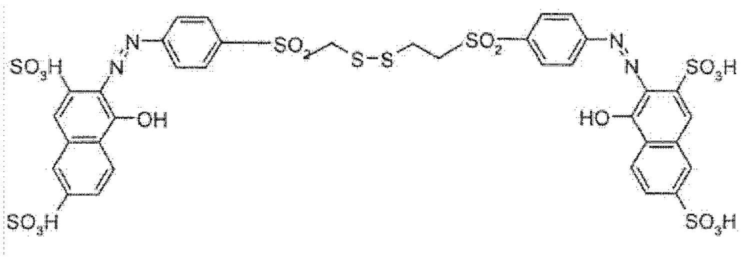

(4)

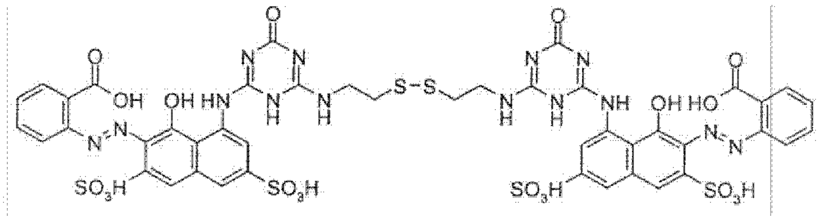

(5)

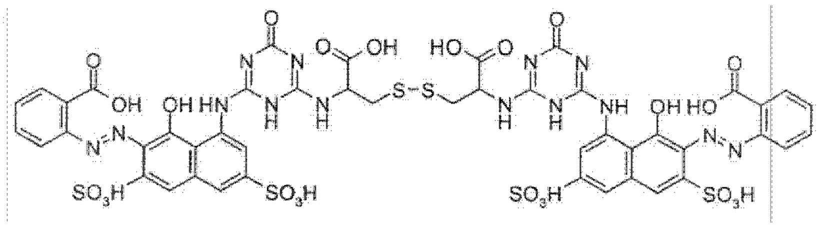

(6)

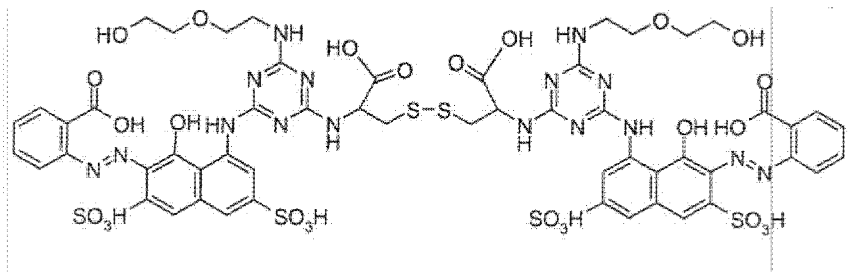

(7)

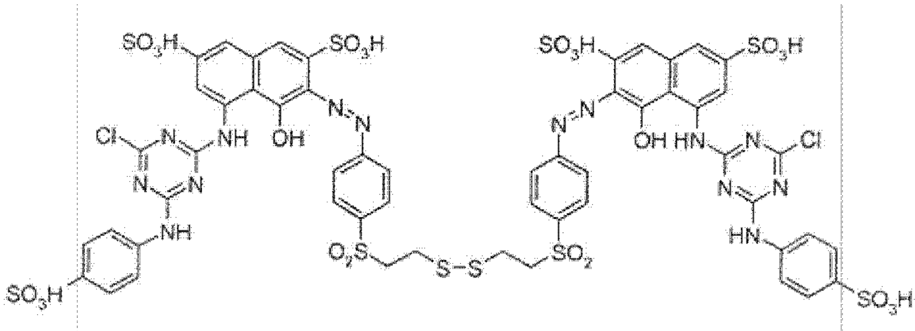
(8)

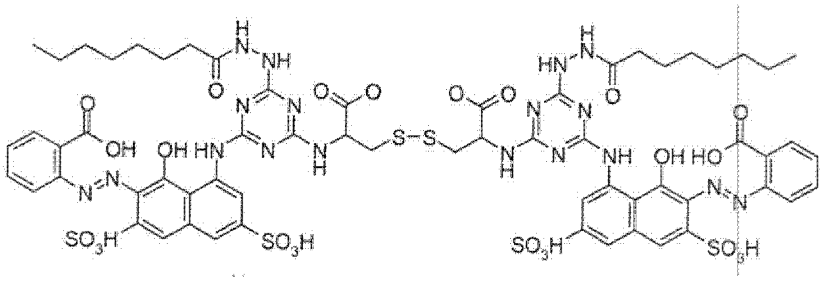
(9)

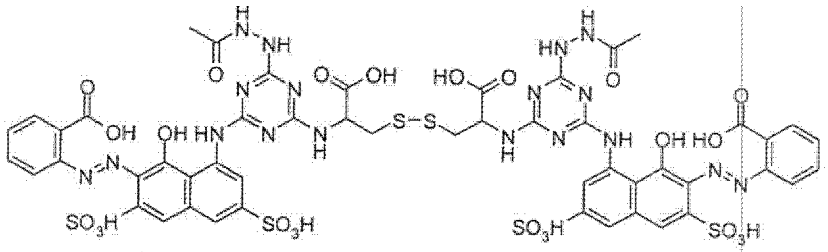
(10)

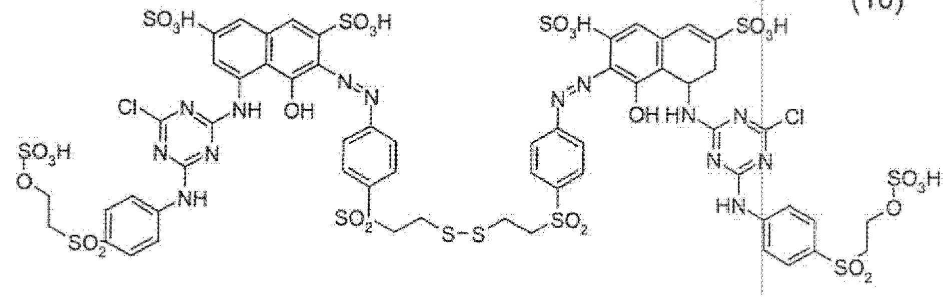
(11)

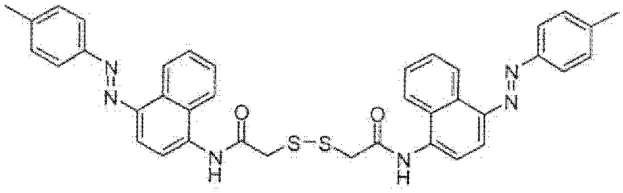
(12)

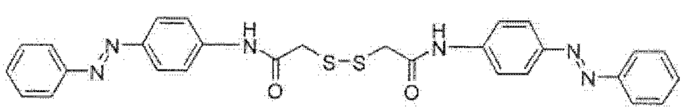
(13)

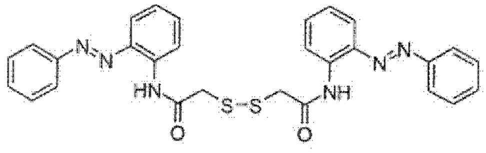

(14)

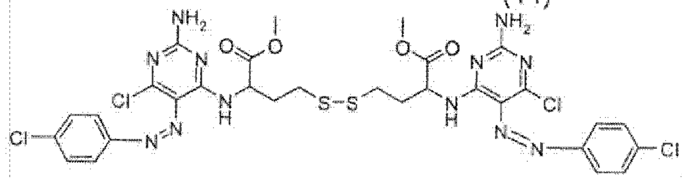

(15)

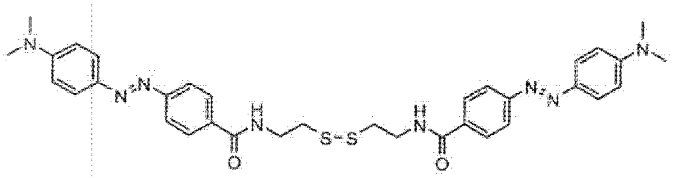

(16)

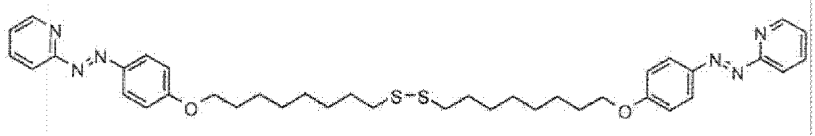

(17)

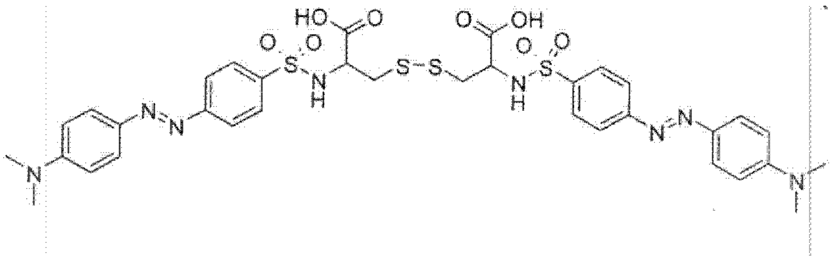

(18)

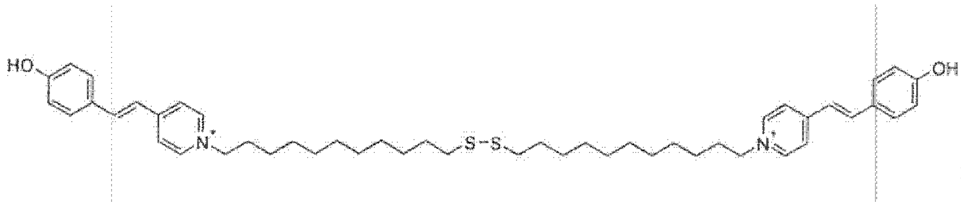

(21)

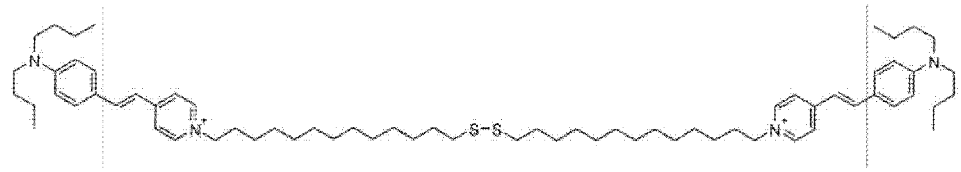

(22)

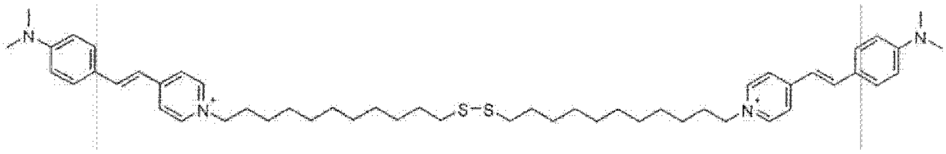

(23)

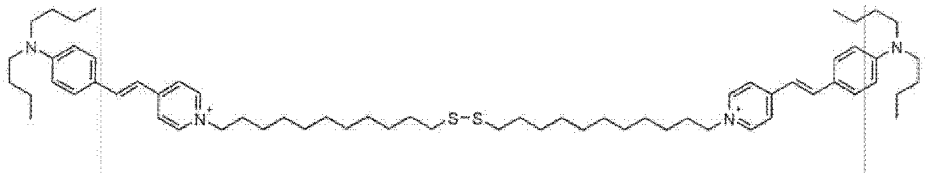

(24)

**9.** Method according to Claim 7, **characterized in that** in formulae (I), (II), (III) or (IV), A and A' comprise a cationic chromophore.

**10.** Method according to Claim 9, in which the cationic chromophore comprises a cationic radical which is a quaternary ammonium.

**11.** Method according to either of Claims 9 and 10, **characterized in that** the disulphide dye is such that A represents W-N=N-Ar- or -W-N=N-Ar, with W representing a fused or nonfused, aromatic or nonaromatic heterocycle comprising a quaternary ammonium; Ar represents a $C_5$ or $C_6$ aryl radical or an aromatic bicycle of the naphthyl type, which are optionally substituted with one or more halogen atoms, preferably chlorine or fluorine atoms; with one or more alkyl, preferably $C_1$-$C_4$ alkyl, groups; with one or more hydroxyl groups; with one or more alkoxy groups, with one or more hydroxyalkyl groups, with one or more amino or (di)alkylamino groups, preferably with the alkyl part being $C_1$-$C_4$.

**12.** Method according to Claim 11, **characterized in that** the disulphide dye is such that p is equal to 1, y and z are equal to zero and T represents -N(R)- at the para-position on Ar with respect to the azo functional group.

**13.** Method according to either of Claims 11 and 12, **characterized in that** W is an imidazolium, pyridinium, benzimidazolium, pyrazolium, benzothiazolium, which are optionally substituted with one or more identical or different $C_1$-$C_4$ alkyl radicals.

**14.** Method according to any one of the preceding claims, **characterized in that** the disulphide dye is chosen from:

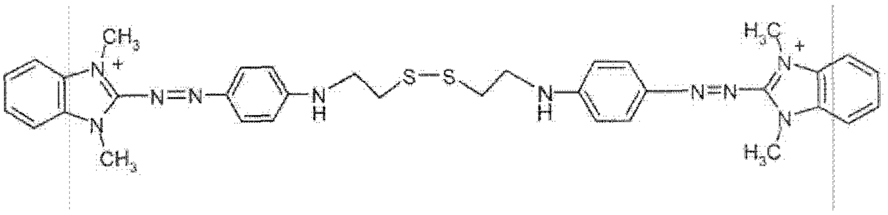

2M'

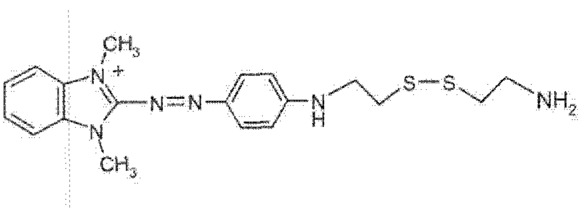

M'

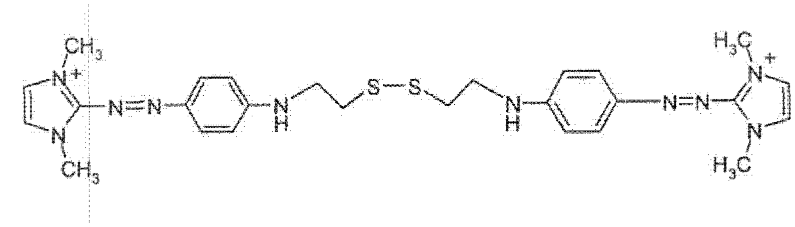

2M'

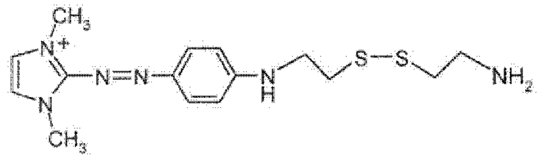

M'

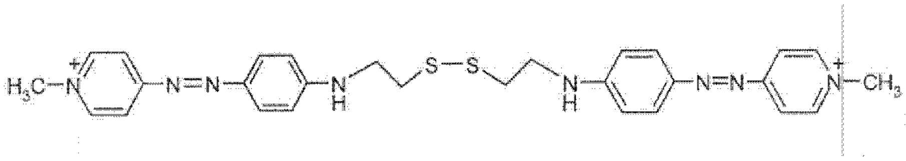

2M'

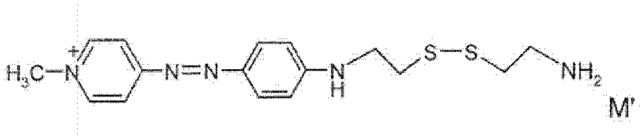

M'

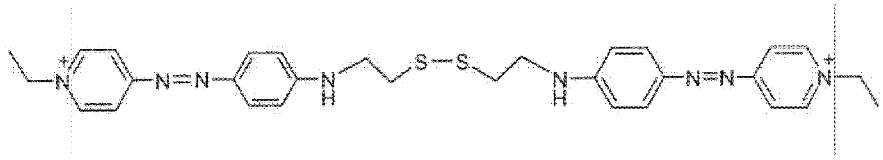

2M'

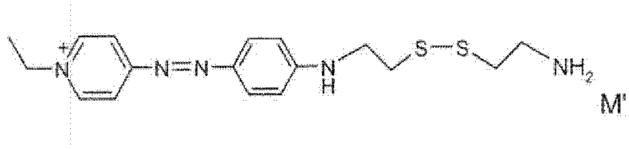

M'

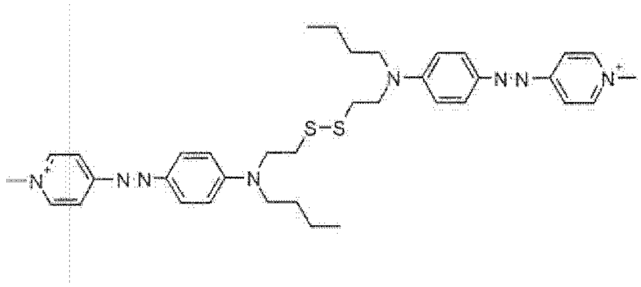

2M'

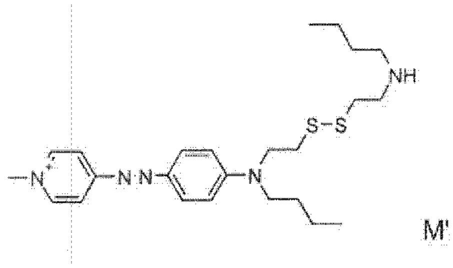

M'

**15.** Method according to any one of the preceding claims, in which the composition contains a reducing agent.

**16.** Method according to any one of Claims 1 to 14, comprising a pretreatment with a reducing agent.

**17.** Method according to any one of Claims 1 to 14, comprising a post-treatment with a reducing agent.

**18.** Method according to Claims 15 to 17, in which the reducing agent is chosen from thiols, phosphines, bisulphite and sulphites.

**19.** Method according to the preceding claim, **characterized in that** the reducing agent is chosen from thioglycolic acid, cysteine, homocysteine, thiolactic acid and the salts of these thiols.

**20.** Method according to Claims 15 to 17, in which the reducing agent is chosen from borohydrides or derivatives thereof, such as for example the borohydride, cyanoborohydride, triacetoxyborohydride and trimethoxyborohydride salts: sodium, lithium, potassium, calcium and quaternary ammonium, such as tetramethylammonium, tetraethylammonium, tetra-n-butylammonium and benzyltriethylammonium, salts; catecholborane.

**21.** Method according to any one of Claims 1 to 14, in which the composition comprises an oxidizing agent.

**22.** Method according to any one of Claims 1 to 14, comprising a post-treatment with an oxidizing agent.

**23.** Method according to any one of Claims 1 to 14, comprising a step of conditioning post-treatment optionally combined with an oxidizing post-treatment.

**24.** Method according to either of Claims 21 and 22, in which the oxidizing agent is chosen from hydrogen peroxide, urea peroxide, alkali metal bromates, persalts such as perborates and persulphates, and enzymes.

**25.** Method according to any one of the preceding claims, in which the disulphide dye is present in a quantity of between 0.001 and 50% by weight, preferably between 0.005 and 20% by weight and more preferably still between 0.01 and 5% by weight relative to the total weight of the composition.

**26.** Method according to any one of the preceding claims, in which the composition contains at least one additional disulphide compound different from the compound of formula (I), (II), (III) or (IV).

**27.** Method according to the preceding claim, **characterized in that** the disulphide compound other than the compounds of formula (I), (II), (III) or (IV) is chosen from compounds comprising at least one fatty chain.

**28.** Method according to any one of the preceding claims, in which the composition comprises at least one oxidation base, one coupler and/or one direct dye other than a disulphide dye.

**29.** Dyeing composition comprising, in an appropriate cosmetic medium, a disulphide dye of formulae (I), (II), (III) or (IV) as defined above in Claims 1 to 14.

**30.** Composition according to Claim 29, in which the disulphide dye of formula (I) is different from the following dye:

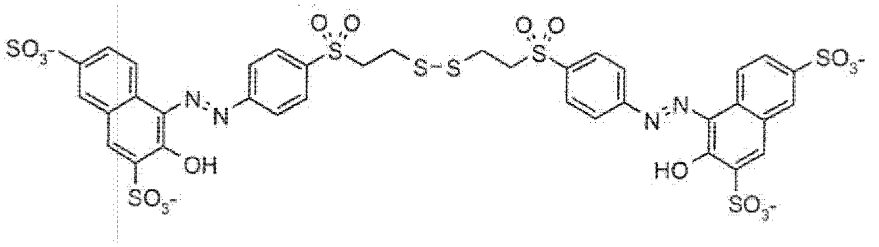

31. Composition according to either of Claims 29 and 30, in which the disulphide is such that A represents W-N=N-Ar- or -W-N=N-Ar, with W representing a fused or nonfused, aromatic or nonaromatic heterocycle comprising a quaternary ammonium; Ar represents a $C_5$ or $C_6$ aryl radical or an aromatic bicycle of the naphthyl type, which are optionally substituted with one or more halogen atoms, preferably chlorine or fluorine atoms; with one or more alkyl, preferably $C_1$-$C_4$ alkyl, groups; with one or more hydroxyl groups; with one or more alkoxy groups, with one or more hydroxyalkyl groups, with one or more amino or (di)alkylamino groups, preferably with the alkyl part being $C_1$-$C_4$.

32. Composition according to the preceding claim, **characterized in that** the disulphide dye is such that p is equal to 1, y and z are equal to zero and T represents -N(R)-.

33. Composition according to either of Claims 31 or 32, **characterized in that** W is an imidazolium, pyridinium, benzimidazolium, pyrazolium, benzothiazolium which are optionally substituted with one or more identical or different $C_1$-$C_4$ alkyl radicals.

34. Composition according to any one of Claims 29 to 33, comprising at least one oxidation base, one coupler and/or one direct dye other than a disulphide dye.

35. Composition according to any one of Claims 29 to 34, comprising at least one oxidizing agent.

36. Composition according to any one of Claims 29 to 35, comprising an organic solvent and/or a thickening agent.

37. Multicompartment device in which a first compartment contains a dyeing composition comprising a disulphide dye as defined in Claims 1 to 13, and a second compartment contains a reducing agent capable of reducing a disulphide bond.

38. Device according to Claim 37, comprising a third compartment which contains an oxidizing agent.

39. Use of the disulphide dyes as defined in Claims 1 to 14 for dyeing human keratin fibres, in particular hair.

40. Use according to Claim 39, for improving the fastness of the coloration.

41. Cationic disulphide dye comprising at least one quaternary ammonium radical of the following formulae (I), (II), (III) or (IV):

$$A-(X)_p-C_{sat}-S\text{-}S-C_{sat}-(X)_p-A \qquad (I)$$

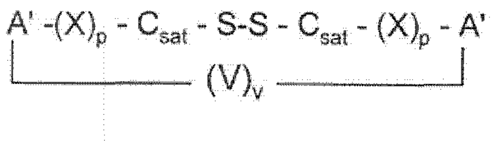

(II)

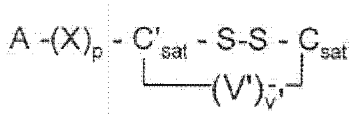

(III)

$$A-(X)_p-C_{sat}-S\text{-}S-C_{sat}-(X)_p-D \qquad (IV)$$

their salts, isomers and solvates such as hydrates, in which formulae:

- A and A', which are identical or different, represent a radical containing at least one cationic chromophore-; this chromophore belonging to the azo, anthraquinone or hydrazone families;
- V and V', which are identical or different, represent a bridging group;
- v and v', which are identical or different, represent 0 or 1;
- X represents the following sequence:

$$-(T)_t-(Y)_y-(Z)_z-$$

the said sequence being linked in formulae (I), (II), (III) or (IV) as follows:

- $C_{sat}$(or $C'_{sat}$)-$(T)_t$-$(Y)_y$-$(Z)_z$-(A or A'); in which T represents one or more radicals or combinations thereof, chosen from $-SO_2-$, -O-, -S-, -N(R)-,
- N+(R)(R)-CO-, R representing a hydrogen atom, a $C_1$-$C_4$ alkyl radical or a $C_1$-$C_4$ hydroxyalkyl radical; the coefficient t is equal to 0 or 1;

Y represents:

- a radical chosen from $-(CH_2)_2-SO_2-$;
- $CH_2$-CHR-CO-NR'- with R, R', which are identical or different, representing a hydrogen atom, a $C_1$-$C_4$ alkyl radical.
- a group of formula (a), (a') or (a"):

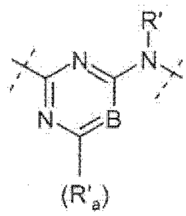

(a)

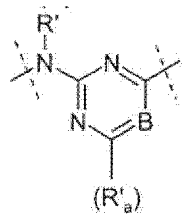

(a')

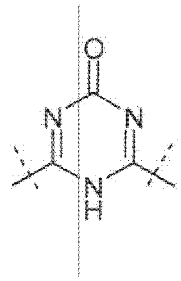

(a")

in which

- B represents -N-, $-CR_a$, with $R_a$ representing a hydrogen atom, a halogen atom chosen from chlorine or fluorine, a nitro group, a pyridinium group which is optionally substituted;
- R' has the same definition as above
- $R'_a$ represents:
  - a hydrogen atom
  - a chlorine atom or a fluorine atom
  - a pyridinium, group which is optionally substituted with at least one group $R_c$, it being possible for $R_c$ to be a $C_1$-$C_4$ alkyl group, a halogen atom, a carboxyl group -COOM (with M representing a hydrogen atom, an alkali mental, an ammonium group or an ammonium group substituted with one or more linear or branched, identical or different $C_1$-$C_{18}$ alkyl radicals, optionally bearing at

least one hydroxyl); an ester group -$COOR_d$ with $R_d$ representing a $C_1$-$C_4$ alkyl radical; an amide group -$CON(R_d)_2$ with $R_d$, which are identical or different, representing a hydrogen atom or a $C_1$-$C_4$ alkyl radical;
- a hydroxyl group
- an amino, alkylamino or dialkylamino group, the identical or different $C_1$-$C_{18}$ alkyl groups being linear or branched, optionally interrupted by a heteroatom chosen from N, O, which are optionally substituted with one or more hydroxyl groups
- a group NHNHCOR where R represents a linear or branched $C_1$-$C_{10}$ alkyl group

- a group of the following formula (b):

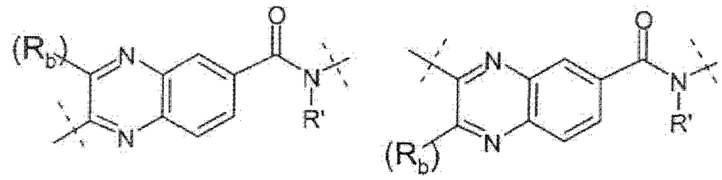

in which

• R' has the same definition as above
• $R_b$ represents

- a chlorine atom
- an amino, alkylamino or dialkylamino group, the identical or different $C_1$-$C_{18}$ alkyl groups being linear or branched, optionally interrupted by a heteroatom chosen from N, O, S, which are optionally substituted with one or more hydroxyls
- a saturated or unsaturated nitrogen-containing heterocycle which may be substituted

• an arylamino group in which the aryl radical is preferably $C_6$;
• the coefficient p is equal to 1;
• **Y** is equal to 0 or 1;
• **Z** represents:

- -$(CH_2)_m$- with m integer between 1 and 8
- -$(CH_2CH_2O)_q$ or $(OCH_2CH_2)_q$- in which q is an integer between 1 and 15
- an aryl, alkylaryl or arylalkyl radical whose alkyl radical is $C_1$-$C_4$ and the aryl radical is preferably $C_6$, being optionally substituted with at least one group $SO_3M$ with M representing a hydrogen atom, an alkali metal or an ammonium group or an ammonium group substituted with one or more identical or different, linear or branched $C_1$-$C_{18}$ alkyl radicals optionally bearing at least one hydroxyl

• **z** is equal to 0 or 1.
• $C_{sat}$, $C'_{sat}$, which are identical or different, represent an optionally substituted, optionally cyclic, linear or branched $C_1$-$C_{18}$ alkylene chain;
• D corresponds to a radical chosen from hydroxyl, hydroxyalkyl, alkoxy, carboxyl, carboxylate, amino, alkylamino and dialkylamino radicals.

**42.** Dye according to Claim 41, **characterized in that** A or A', which are identical or different, contain an azo chromophore.

**43.** Dye according to any one of the two preceding claims, **characterized in that** the disulphide dye is such that y and z are equal to zero and T represents -N(R)-.

**44.** Dye according to any one of Claims 41 to 43, **characterized in that** W is an imidazolium, pyridinium, benzimidazolium, pyrazolium, benzothiazolium which are optionally substituted with one or more identical or different $C_1$-$C_4$ alkyl radicals.

**45.** Dye according to any one of Claims 41 to 44, **characterized in that** the disulphide dye is chosen from:

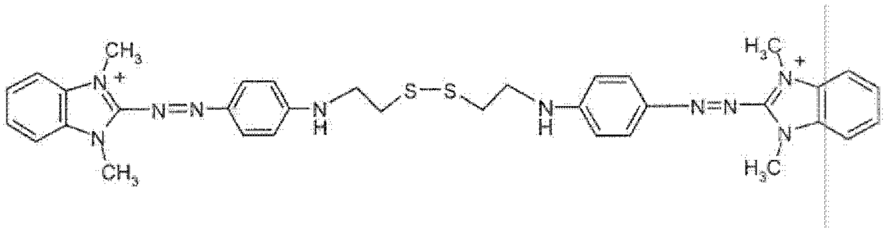

2M'

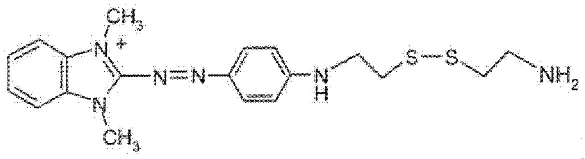

M'

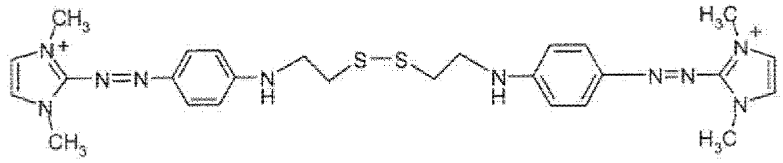

2M'

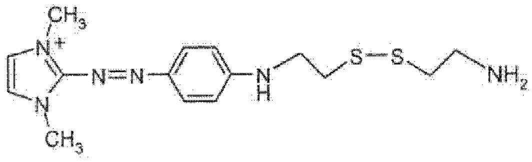

M'

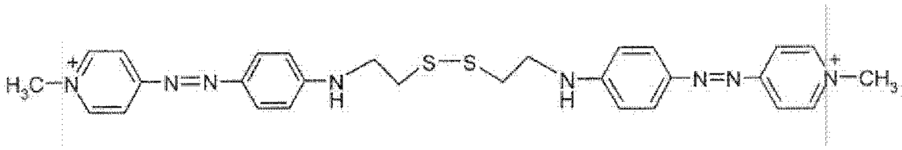

2M'

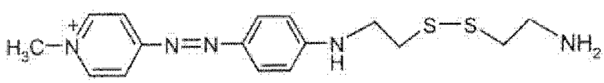

M'

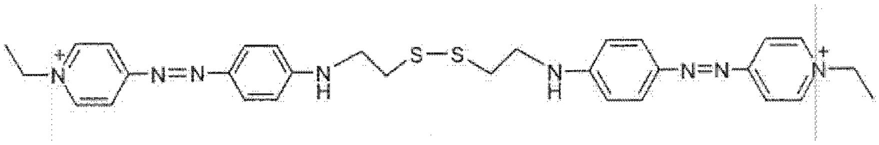

2M'

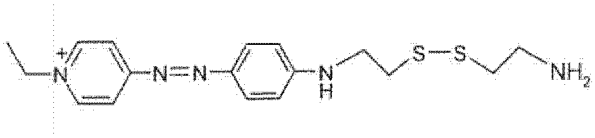

M'

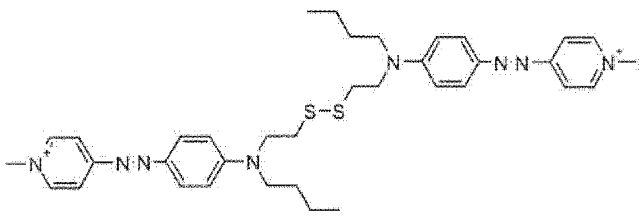

2M'

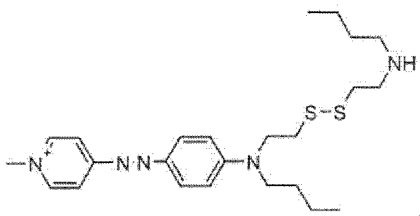

M'

**46.** Dye of the following formulae:

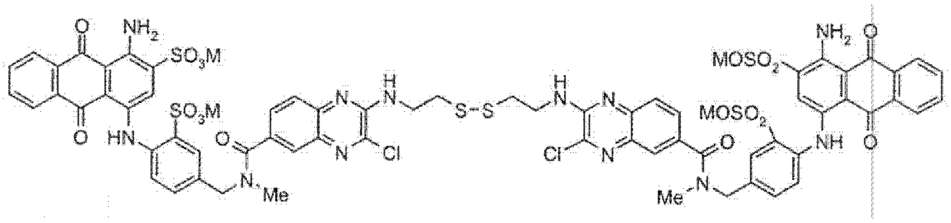

(17)

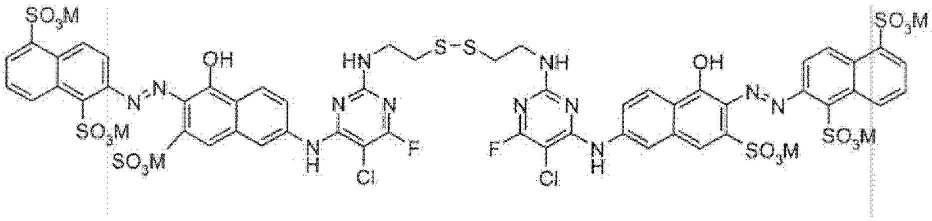

(18)

with M representing a hydrogen atom, an alkali metal or an ammonium group or an ammonium group substituted with one or more identical or different, linear or blanched $C_1$-$C_{10}$ alkyl radicals optionally bearing at least one hydroxyle, and the following compounds, in acidic, basic or neutralized form.

## Patentansprüche

**1.** Verfahren zum Färben von menschlichen Keratinfasern, **dadurch gekennzeichnet, dass** auf die Fasern eine Farbmittelzusammensetzung aufgetragen wird, die in einem geeigneten kosmetischen Medium mindestens einen Disulfidfarbstoff enthält, der ausgewählt ist unter den Farbstoffen der folgenden Formeln (I), (II), (III) oder (IV):

$$A - (X)_p - C_{sat} - S\text{-}S - C_{sat} - (X)_p - A \qquad (I)$$

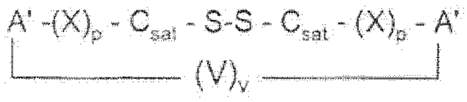

(II)

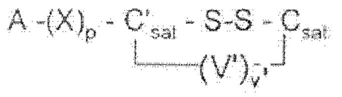

(III)

$$A\text{-}(X)_p\text{-}C_{sat}\text{-}S\text{-}S\text{-}C_{sat}\text{-}(X)_p\text{-}D \qquad (IV)$$

in den Formeln:

- A und A', die gleich oder verschieden sind, bedeuten eine Gruppe, die mindestens ein kationisches oder nicht kationisches Chromophor enthält;
- V und V', die gleich oder verschieden sind, bedeuten eine Brückengruppe;
- v und v', die gleich oder verschieden sind, bedeuten 0 oder 1;
- X, die gleich oder verschieden sind, bedeuten eine geradkettige oder verzweigte, gesättigte oder ungesättigte Kohlenwasserstoffkette mit 1 bis 30 Kohlenstoffatomen, die gegebenenfalls unterbrochen und/oder gegebenenfalls an einem oder an beiden Enden abgeschlossen werden kann durch eine oder mehrere Gruppen, die unter den folgenden Gruppen oder ihren Kombinationen ausgewählt sind:

  - -N(R)-, $-N^+(R)(P-)-$, -O-, -S-, -CO-, $-SO_2-$, wobei die Gruppen R, die gleich oder verschieden sind, ausgewählt sind unter Wasserstoff, eine $C_{1-4}$-Alkylgruppe, Hydroxyalkl, Aminoalkyl,
  - eine aromatische oder nicht aromatische, gesättigte oder ungesättigte, kondensierte oder nicht kondensierte (hetero)cyclische die gegebenenfalls ein oder mehrere, gleiche oder verschiedene, gegebenenfalls substituierte Heteroatome enthält;

- der Koeffizient p bedeuten 0 der 1;
- Csat und C'sat, die gleich oder verschieden sind, bedeuten eine lineare oder verzweigte, gegebenenfalls substituierte, gegebenenfalls cyclische $C_{1-18-}$-Alkylenkette;
- D entspricht einer Gruppe, die unter den Gruppen Hydroxy, Hydroxyalkyl, Alkoxy, Carboxy, Carboxyat, Amino, Alkylamino, Dialkylamino ausgewählt ist.

**2.** Verfahren nach Anspruch 1, **dadurch gekennzeichnet, dass** in den Formeln (I), (II), (III) oder (IV), wenn p gleich 1 ist, X die folgende Sequenz bedeutet:

$$-(T)_t-(Y)_y-(Z)_z-$$

wobei diese Sequenz in den Formeln (I), (II), (III) oder (IV) folgendermaßen gebunden ist;

- Csat (oder C'sat)-(T)t-(Y)y-(Z)z-(A oder A'); wobei in der Sequenz:
T bedeutet eine oder mehrere Gruppen oder deren Kombinationen, die ausgewählt sind unter: $-SO_2-$, -O-, -S-, -N(R)-, $-N^+(R)(R)-CO-$, wobei R ein Wasserstoffatom, eine $C_{1-4}$-Alkylgruppe oder eine $C_{1-4}$-Hydroxyalkylgruppe bedeutet;
der koeffizient t beträgt 0 oder 1;
Y bedeutet:
- eine Gruppe, die ausgewählt ist unter $-(CH_2)_2-SO_2-$; $-CH_2-CHR-CO-NR'-$, wobei R und R', die gleich oder verschieden sind, ein Wasserstoffatom, eine $C_{1-4}$-Alkylgruppe bedeuten;
- eine Gruppe der Formel (a), (a') oder (a") :

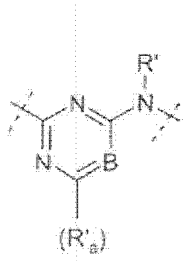
(a)
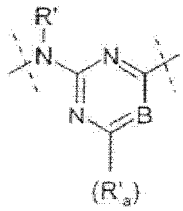
(a')
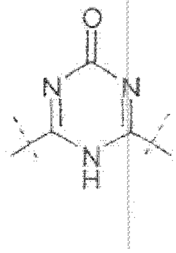
(a'')

.

in der Formel:

· B bedeutet -N-, $-CR_a$, wobei $R_a$ ein Wasserstoffatom, ein Halogenatom, das unter Chlor oder Fluor ausgewählt ist, eine Nitrogruppe, eine Kridiniumgruppe, die gegebenenfalls substituiert ist, bedeutet;
· R' weist die oben angegebenen Bedeutungen auf;
· R' bedeutet:

- ein Wasserstoffatom
- ein Chloratom oder ein Fluoratom
- eine Pyridiniumgruppe, die mit mindestens einer Gruppe $R_c$ substituiert ist, wobei es sich bei $R_c$ um $C_{1-4}$-Alkylgruppe, ein Halogenatom, eine Carboxygruppe -COOM (wobei M ein Wasserstoffatom, ein Alkalimetall, eine Ammoniumgruppe oder eine Ammoniumgruppe die mit einer oder mehreren, gleichen oder verschiedenen, linearen oder verzweigten $C_{1-18}$-Alkylgruppen substituiert ist, die gegebenenfalls mindestens eine hydroxygruppe tragen); eine Estergruppe $-COOR_d$, wobei es sich bei $R_d$ um eine $C_{1-4}$-Alkylgruppe handelt; eine Amidgruppe $-CON(R^d)_2$, wobei die Gruppen $R_d$, die gleich oder verschieden sind, ein Wasserstoffatom oder eine $C_{1-4}$-Alkylgruppe bedeuten;
- eine Hydroxygruppe
- eine Amino-, Alkylamino- oder Dialkylaminogruppe, wobei die Alkylgruppen, die gleich oder verschieden sind, 1 bis 18 Kohlenstoffatome aufweisen und linear oder verzweigt vorliegen und gegebenenfalls durch ein Heteroatom unterbrochen sind, das unter N,O ausgewählt ist, und gegebenenfalls mit einer oder mehreren Hydroxygruppen substituiert sind,
- eine Gruppe NHNHCOR, wobei R eine lineare oder verzweigte $C_{1-10}$-Alkylgruppe bedeutet;

- eine Gruppe der folgenden Formel (b):

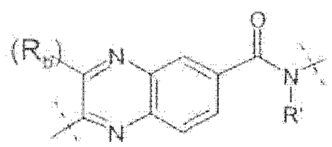
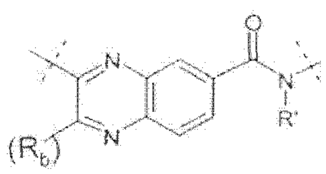

worin bedeuten:

• R' hat die oben angegebenen Bedeutungen
• $R_b$ bedeutet:

- ein Chloratom
- eine Amino-, Alkylamino- oder Dialkylaminogruppe, wobei die Alkylgruppen, die gleich oder verschieden sind, 1 bis 18 Kohlenstoffatomen aufweisen und linear oder verzweigt vorliegen und gegebenenfalls durch ein Heteroatom unterbrochen sind, das unter N, O, S ausgewählt ist, und gegebenenfalls mit einer oder mehreren Hydroxygruppen substituiert sind,
- eine gesättigten oder -Lingesättigten Stickstoffheterocyclus, der substituiert sein kann

- eine Arylaminogruppe, bei der die Arylgruppe vorzugsweise 6 Kohle-nstoffatome aufweist;

y bedeuten 0 oder 1;
Z bedeutet:

- $-(CH_2)_m-$, m eine ganze Zahl im Bereich von 1 bis 8 bedeutet
- $-(CH_2CH_2O)_q-$ oder $-(OCH_2CH_2)_q-$, worin q eine ganze Zahl im Bereich von 1 bis 15 bedeutet
- eine Aryl-, Alkylaryl- oder Arylalkylgruppe, wobei die Alkylgruppen 1 bis 4 Kohlenstoffatome aufweisen und die Arylgruppe vorzugsweise C6 ist, wobei sie gegebenenfalls mit mindestens einer Gruppe $SO_3M$ substituiert ist, wobei M ein Wasserstoffatom, ein Alkalimetall, eine Ammoniumgruppe oder eine Ammoniumgruppe bedeutet, die mit einer oder mehreren, gleichen oder verschiedenen, linearen oder verzweigten $C_{1-18}$-Alkylgruppen substituiert ist, die gegebenenfalls mindestens eine Hydroxygruppe tragen

z bedeuten 0 oder 1.

3. Verfahren nach Anspruch 1 oder 2, **dadurch gekennzeichnet, dass** Y bedeutet;

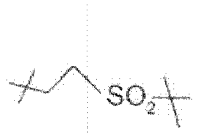
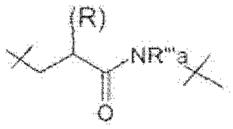
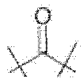
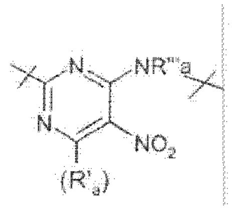
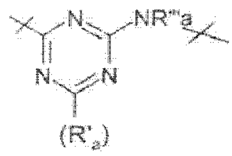

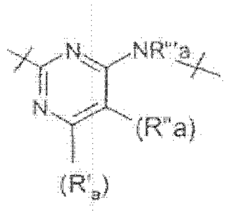
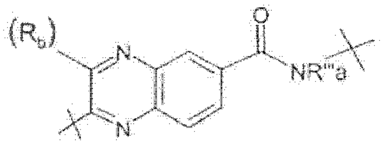
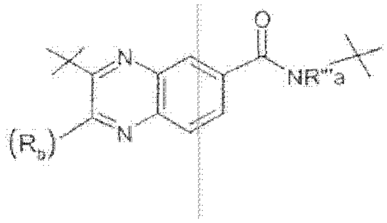

wobei die Gruppen R, $R'_a$ und $R_b$ die oben angegebenen Bedeutungen aufweisen; die Gruppen $R''_a$, weisen unabhängig voneinander die für $R'_a$ angegebenen Bedeutungen auf; $R'''_a$ bedeutet ein Wasserstoffatom oder eine Alkylgruppe.

4. Verfahren nach einem der vorhergehenden Ansprüche, **dadurch gekennzeichnet, dass** Z bedeutet:

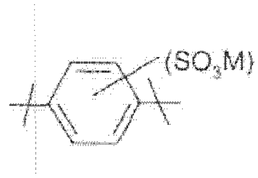
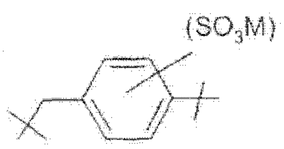
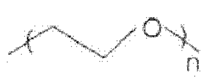
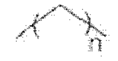

5. Verfahren nach einem der vorhergehenden Ansprüche, **dadurch gekennzeichnet, dass** der Disulfidfarbstoff so vorliegt, dass v gleich 0 ist

6. Verfahren nach einem der vorhergehenden Ansprüche, **dadurch gekennzeichnet, dass** in den Formeln (I), (II), (III) oder (IV) A und A' eine Gruppe bedeuten, die mindestens ein Chromophor umfasst, das von einem Farbstoff vom Typ der Acridine, Acridone, Anthranthrone, Anthrapyrimidine, Anthrachinone, Azine, Azofarbstoffe, Azomethine, Benzanthrone, Benzimidazole,
Benzimidazolone, Benzindole, Benzoxazole, Benzopyrane, Benzothiazole, Benzochinone, Bisazine, Bisisoindoline, Carboxanilide, Cumarine, Cyanine, Diazine, Dicetopyrrolopyrrole, Dioxazine, Diphenylamine, Diphenylmethane, Dithilazine, Flavonoide, Fluoriridine, Formazane, Hydrazone, Hydroxyketone, Indamine. Indanthrone, Indigoide und Pseudoindigoide, Indophenole, Indoaniline, Isoindoline, Isoindolinone, Isoviolanthrone, Lactone, Methine, Naphthalimide, Naphthanilide, Naphtholactame, Naphthochinone, Nitro, Oxadiazole, Oxazine, Perilone, Perinone, Perylene, Phenazine, Phenothiazine, Phthalocyanine, Polyene/Carotinoide, Porphyrine, Pyranthrone, Pyrazolanthrone, Pyrazolone, Pyrimidinoanthrone, Pyronine, Chinacridone, Chinoline, Chinophthalone, Squarane, Stilbene, Tetrazoliume, Thiazine, Thioindigo, Thiopyronine, Triarylmethane, Xanthene stammt.

**7.** Verfahren nach Anspruch 6, **dadurch gekennzeichnet, dass** in den formeln (I), (II), (III) oder (IV) A und A' eine Gruppe bedeuten, die mindestens ein Chromophor umfasst, das unter den Chromophoren vom Typ der Azofarbstoffe, Anthrachinone und Hydrazone ausgewählt ist.

**8.** Verfahren nach einem der Ansprüche 1 bis 7, wobei der Disulfidfarbstoff ausgewählt ist unter:

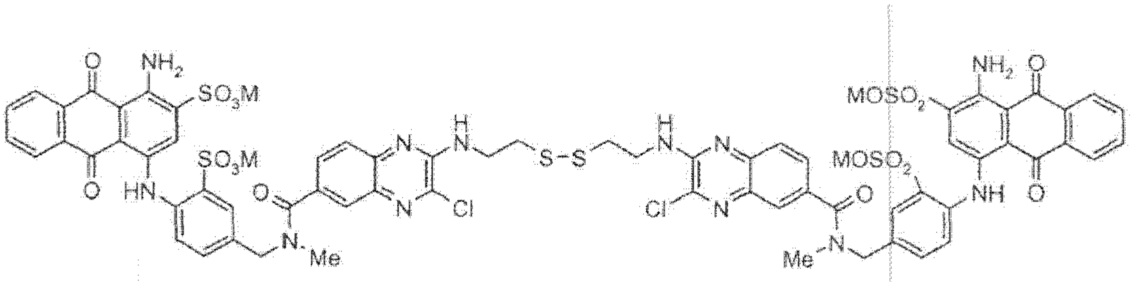

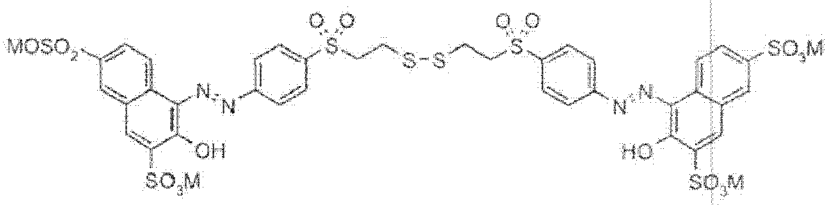

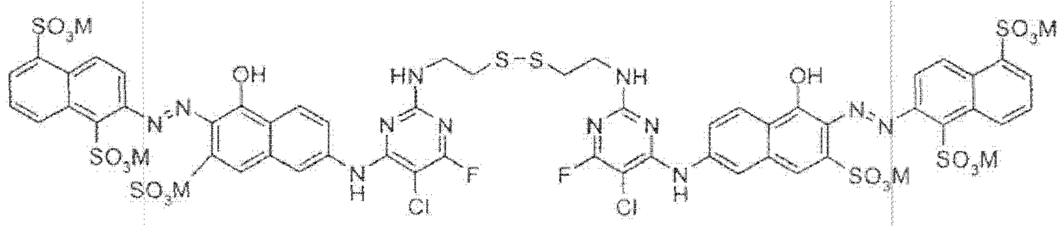

wobei M ein Wasserstoffatom, ein Alkalimetall, eine Ammoniumgruppe oder eine Ammoniumgruppe bedeutet, die mit einer oder mehreren, gleichen oder verschiedenen, linearen oder verzweigten $C_{1-10}$-Alkylgruppen substituiert ist, die gegebenenfalls mindestens eine Hyroxygruppe tragen, sowie den folgenden Verbindungen in saurer, basischer oder neutralisierter Form:

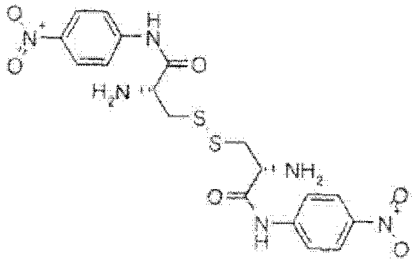

(1)

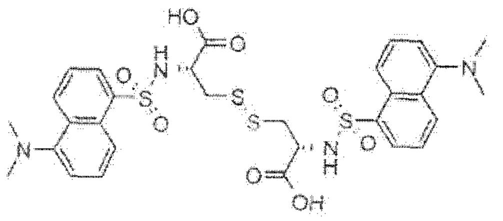

(2)

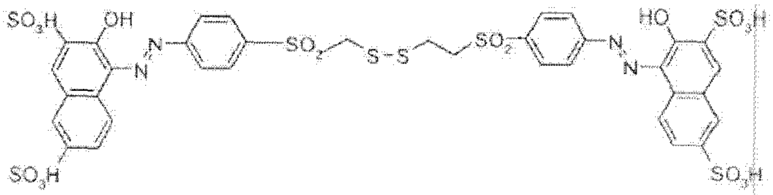

(3)

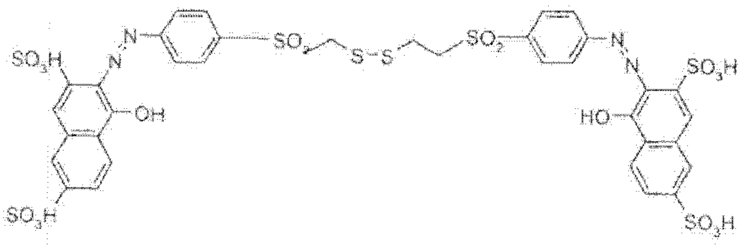

(4)

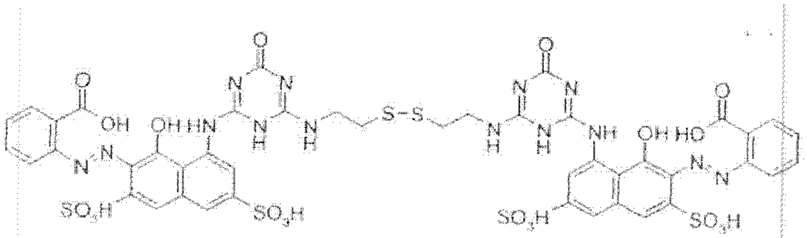

(5)

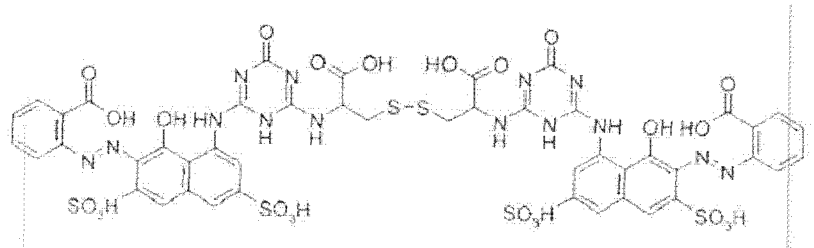

(6)

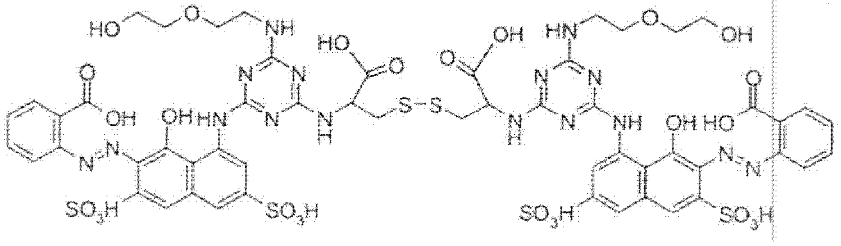

(7)

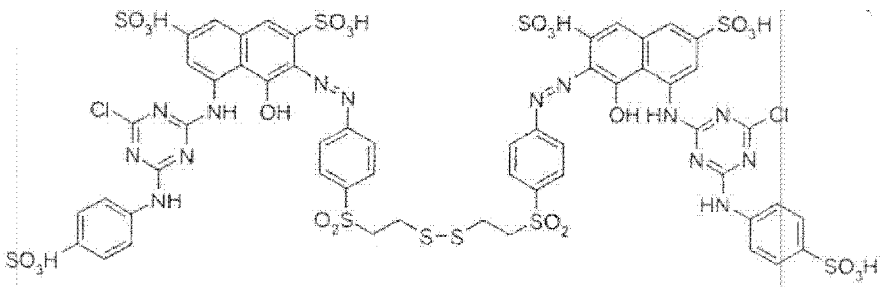

(8)

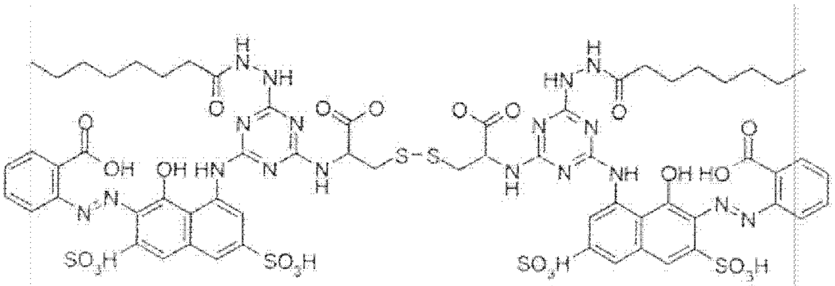

(9)

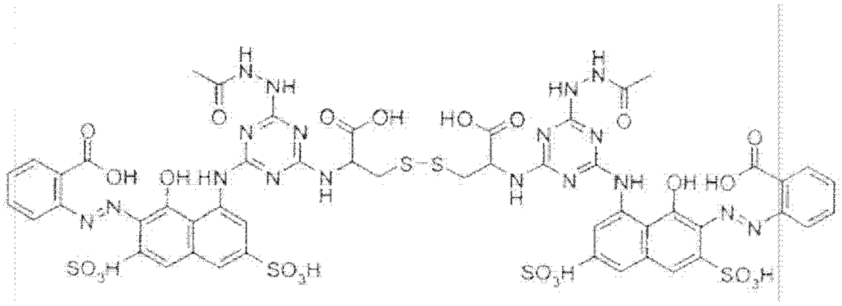

(10)

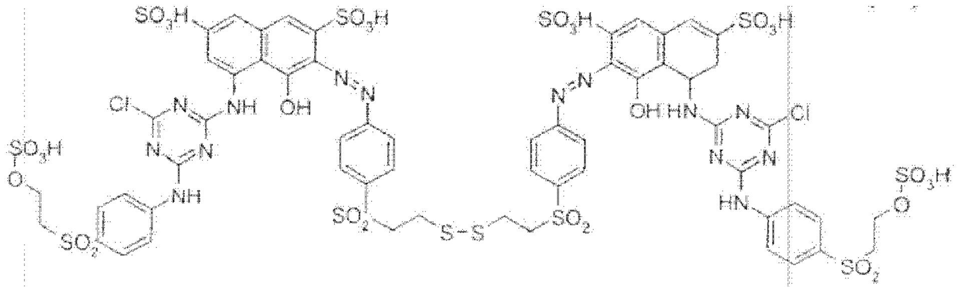

(11)

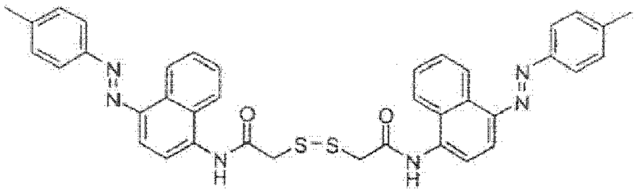

(12)

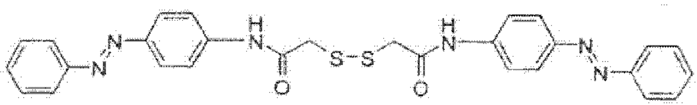

(13)

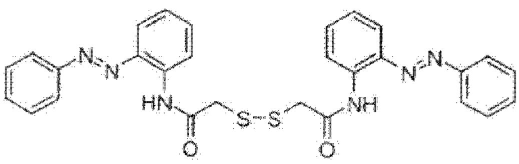

(14)

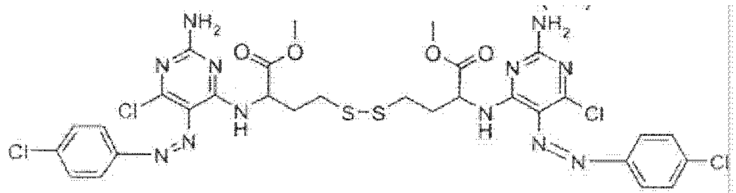

(15)

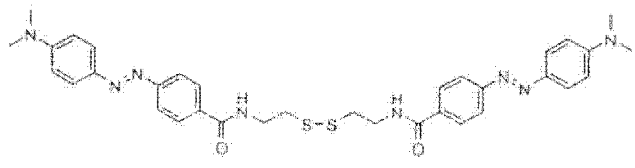

(16)

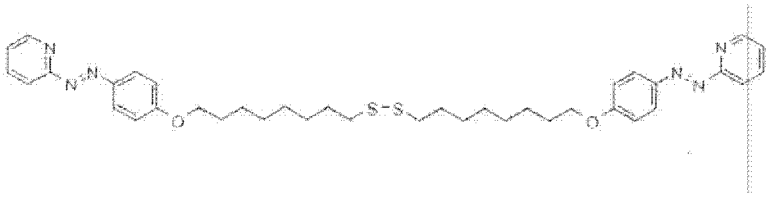

(17)

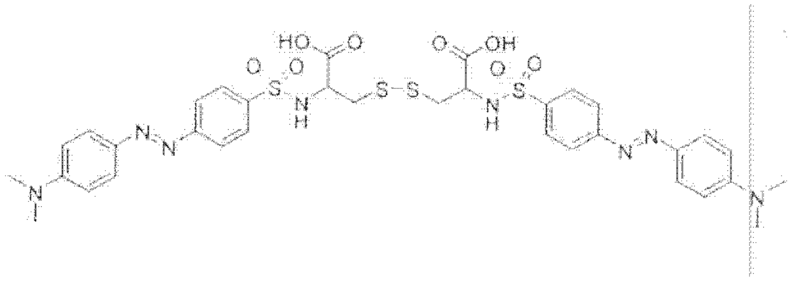

(18)

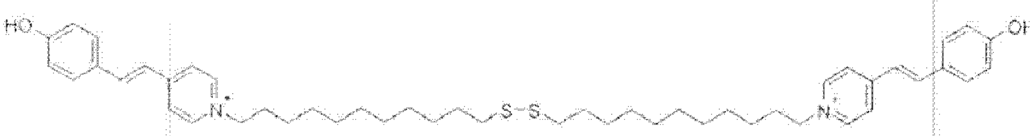

(21)

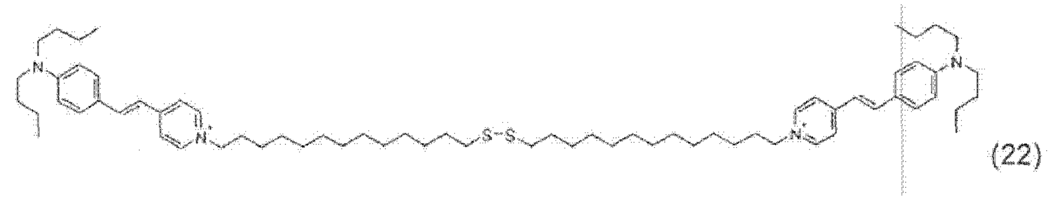

(22)

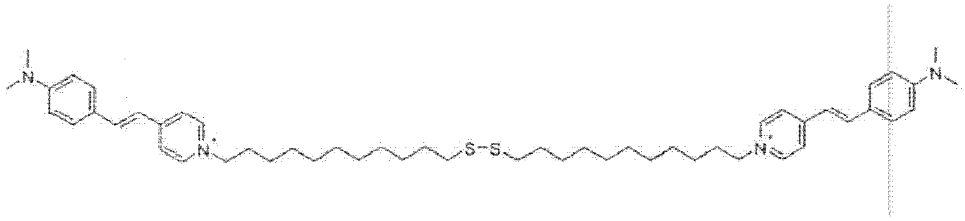

(23)

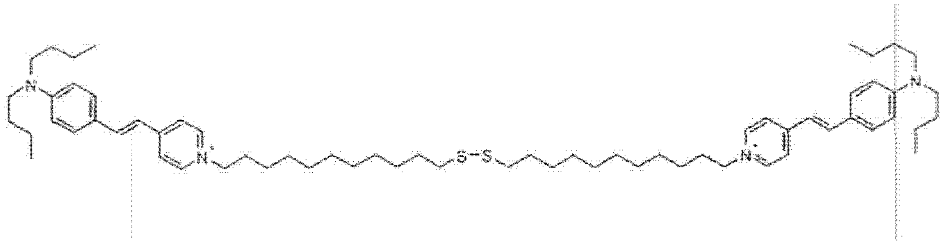

(24)

9. Verfahren nach Anspruch 7, **dadurch gekennzeichnet, dass** in den Formeln (I), (II), (III) oder (IV) A und A' ein kationisches Chromophor umfassen.

10. Verfahren nach Anspruch 9, wobei das kationische Chromophor eine kationische Gruppe aufweist, bei der es sich um eine quartäre Ammoniumgruppe handelt.

11. Verfahren nach einem der ansprüche 9 oder 10, **dadurch gekennzeichnet, dass** der Disulfidfarbstoff so vorliegt, dass A W-N=N-Ar- oder -W-N=N-AR bedeutet, wobei W einen aromatischen oder nicht aromatischen, kondensierten oder nicht kondensierten Heterocyclus bedeutet, der eine quartäre Ammoniumgruppe umfasst; Ar bedeutet eine $C_5$-, $C_6$-Arylgruppe oder einen aromatischen Bicyclus vom Naphthyltyp, wobei diese gegebenenfalls substituiert sind mit einem oder mehreren Halogenatomen, vorzugsweise Chlor, Fluor; mit einer oder mehreren Alkylgruppen, die vorzugsweise 1 bis 4 Kohlenstoffatome aufweisen; mit einer oder mehreren Hydroxygruppen; mit einer oder mehreren Alkoxygruppen, mit einer oder mehreren Hydroxyalkylgruppen, mit einer oder mehreren Amino- oder (Di) alkylaminogruppen, deren Alkylteil vorzugsweise 1 bis 4 Kohlenstoffatome aufweist.

12. Verfahren nach Anspruch 11, **dadurch gekennzeichnet, dass** der Disulfidfarbstoff so vorliegt, dass p 1 ist, y und z Null bedeuten und T in Bezug auf die Azofunktion in para-Stellung am Ar -N(R)- bedeutet.

13. Verfahren nach einem der Ansprüche 11 oder 12, **dadurch gekennzeichnet, dass** W bedeutet: Imidazolium, Pyridinium, Benzimidazolium, Pyrazolium, Benzothiazolium, die gegebenenfalls mit einer oder mehreren, gleichen oder verschiedenen $C_{1-4}$-Alkylgruppen substituiert sind.

14. Verfahren nach einem der vorhergehenden Ansprüche, **dadurch gekennzeichnet, dass** der Disulfidfarbstoff ausgewählt ist unter:

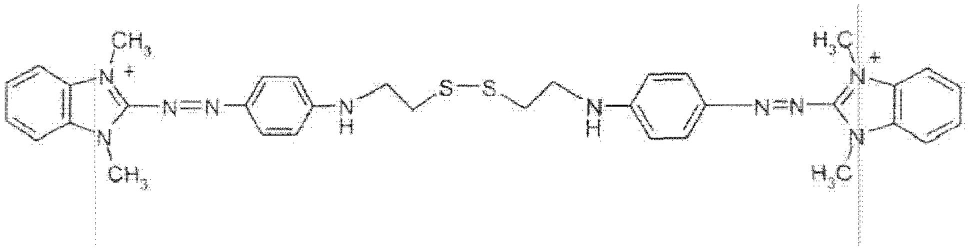

2M'

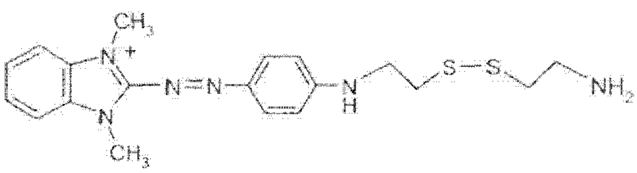

M'

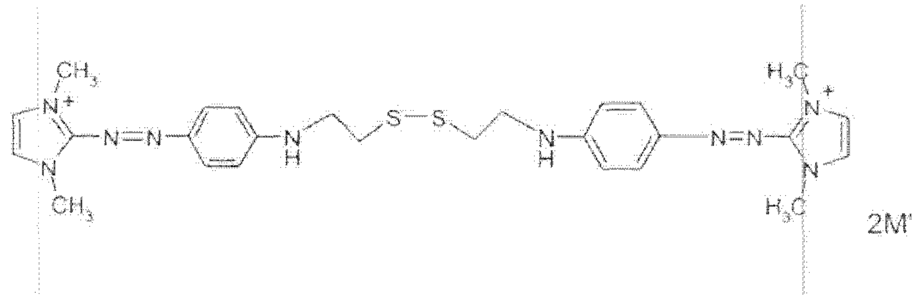

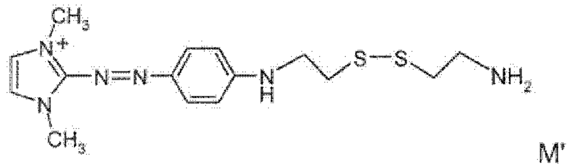

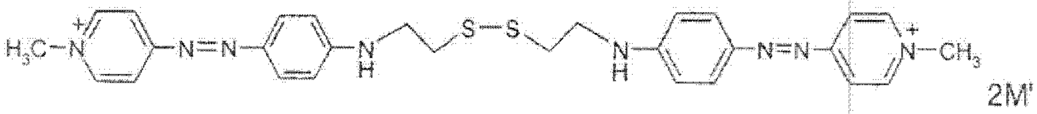

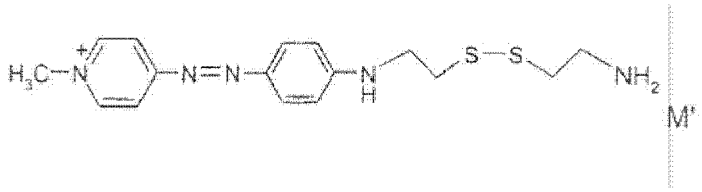

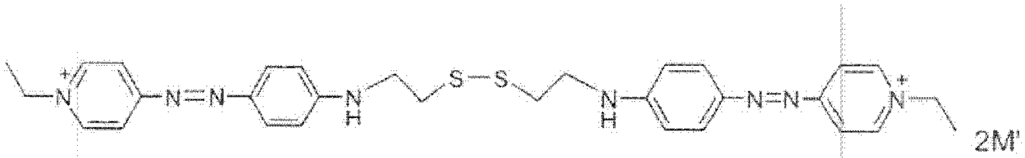

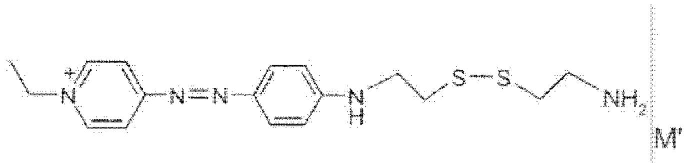

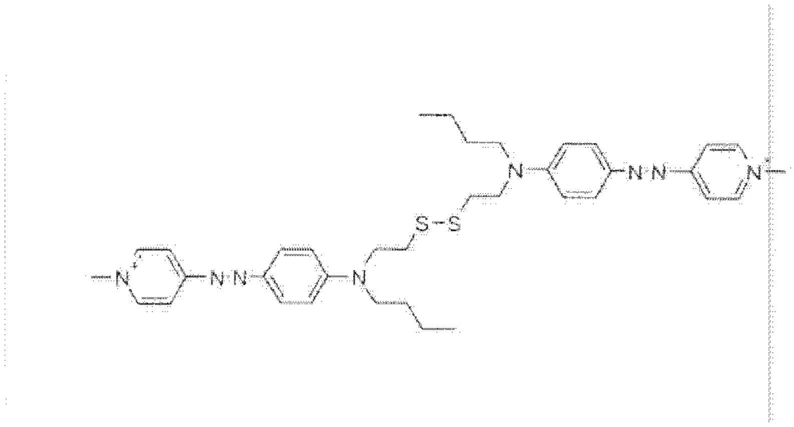

2M'

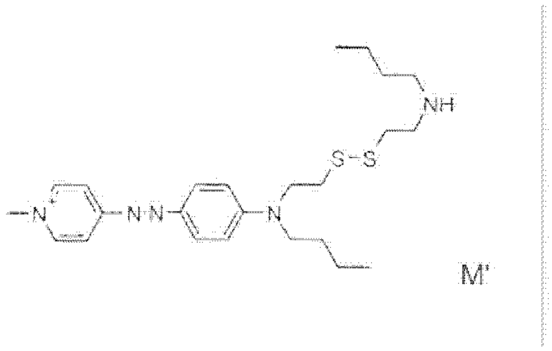

M'

**15.** Verfahren nach einem der vorhergehenden Ansprüche, **dadurch gekennzeichnet, dass** die Zusammensetzung ein Reduktionsmittel enthält.

**16.** Verfahren nach einem der Ansprüche 1 bis 14, **dadurch gekennzeichnet, dass** es eine Vorbehandlung mit einem Reduktionsmittel umfasst.

**17.** Verfahren nach einem der Ansprüche 1 bis 14, **dadurch gekennzeichnet, dass** es eine Nachbehandlung mit einem Reduktionsmittel umfasst.

**18.** Verfahren nach einem der Ansprüche 15 bis 17, wobei das Reduktionsmittel unter den Thiolen, Phosphinen, Bisulfit und Sulfiten ausgewählt ist.

**19.** Verfahren nach dem vorhergehenden Anspruch, wobei das Reduktionsmittel unter Thioglycolsäure, Cystein, Homocystein, Thiomilchsäure und den Salzen dieser Thiole ausgewählt ist.

**20.** Verfahren nach Anspruch 15 bis 17, wobei das Reduktionsmittel unter den Borhydriden und ihren Derivaten, wie beispielsweise den Salzen von Borhydrid, Cyanoborhydrid, Triacetoxyborhydrid, Trimethoxyborhydrid: Natriumsalzen, Lithiumsalzen, Kaliumsalzen, Calcium salzen, quartären Ammoniumsalzen, wie Tetramethylammonium, Tetraethylammonium, Tetra-n-butylammonium, Benzyltriethylammonium; Catecholboran ausgewählt ist.

**21.** Verfahren nach einem der Ansprüche 1 bis 14, wobei die Zusammensetzung ein Oxidationsmittel enthält.

**22.** Verfahren nach einem der Ansprüche 1 bis 14, das eine Nachbehandlung mit einem Oxidationsmittel umfasst.

**23.** Verfahren nach einem der Ansprüche 1 bis 14, das einen Schritt der konditionierenden Nachbehandlung gegebenenfalls in Kombination mit einer oxidierenden Nachbehandlung umfasst.

**24.** Verfahren nach einem der Ansprüche 21 oder 22, wobei das Oxidationsmittel unter Wasserstoffperoxid, Harnstoffperoxid, Alkalimetallbromaten, Salzen von Persäuren, wie Perboraten und Persulfaten, sowie Enzymen ausgewählt ist.

**25.** Verfahren nach einem der vorhergehenden Ansprüche, wobei der Disulfidfarbstoff in einem Mengenanteil im Bereich von 0,001 bis 50 Gew.-%, vorzugsweise im Bereich von 0,005 bis 20 Gew.-% und besonders bevorzugt im Bereich von 0,01 bis 5 Gew.-%, bezogen auf das Gesamtgewicht der Zusammensetzung, vorliegt.

**26.** Verfahren nach einem der vorhergehenden Ansprüche, wobei die Zusammensetzung mindestens einen ergänzenden Disulfidfarbstoff enthält, der von der Verbindung der Formel (I), (II), (III) oder (IV) verschieden ist.

**27.** Verfahren nach dem vorhergehenden Anspruch, wobei der Disulfidfarbstoff, der von der Verbindung der Formel (I), (II), (III) oder (IV) verschieden ist, unter den Verbindungen ausgewählt ist, die mindestens eine Fettkette enthalten.

**28.** Verfahren nach einem der vorhergehenden Ansprüche, wobei die Zusammensetzung mindestens eine Oxidationsbase, eine Kuppler und/oder einen Direktfarbstoff enthält, der von einem Disulfidfarbstoff verschieden ist.

**29.** Farbmittelzusammensetzung, die in einem kosmetisch geeigneten Medium einen Disulfidfarbstoff der Formeln (I), (II), (III) oder (IV) enthält, wie er in einem der Ansprüche 1 bis 14 definiert ist.

**30.** Zusammensetzung nach Anspruch 29, wobei der Disulfidfarbstoff der Formel (I) von dem folgenden Farbstoff verschieden ist:

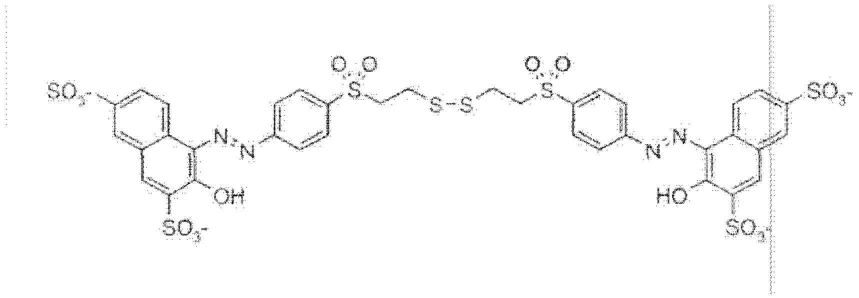

**31.** Zusammensetzung nach einem der Ansprüche 29 oder 30, der Disulfidfarbstoff so vorliegt, dass A-W-N=N-Ar oder W-N=N-Ar-bedeutet, wobei W eine aromatischen oder nicht aromatischen, kondensierten oder nicht kondensierten Heterocyclus bedeutet, der eine quartäre Ammoniumgruppe umfasst; Ar bedeutet eine $C_5$-, $C_6$-Arylgruppe oder einen aromatischen Bicyclus vom Naphthyltyp, wobei diese gegebenenfalls substituiert sind mit einem oder mehreren Halogenatomen, vorzugsweise Chlor, Fluor; mit einer oder mehreren Alkylgruppen, die vorzugsweise 1 bis 4 Kohlenstoffatome aufweisen; mit einer oder mehreren Hydroxygruppen; mit einer oder mehreren Alkoxygruppen, mit einer oder mehreren Hydroxyalkylgruppen, mit einer oder mehreren Amino- oder (Di)alkylaminogruppen, deren Alkylteil vorzugsweise 1 bis 4 Kohlenstoffatome aufweist.

**32.** Zusammensetzung nach dem vorhergehenden Anspruch, **dadurch gekennzeichnet, dass** der Disulfidfarbstoff so vorliegt, dass p 1. ist, y und z Null bedeuten und T -N(R)- bedeutet.

**33.** Zusammensetzung nach einem der Ansprüche 31 oder 32, **dadurch gekennzeichnet, dass** W bedeutet: Imidazolium, Pyridinium, Benzimidazolium, Pyrazolim, Benzothiazolium, die gegebenenfalls mit einer oder mehreren, gleichen oder verschiedenen $C_{1-4}$-Alkylgruppen substituiert sind.

**34.** Zusammensetzung nach einem der Ansprüche 29 bis 33, die mindestens eine Oxidationsbase, eine Kuppler und/ oder einem Direktfarbstoff enthält, der von einem Disulfidfarbstoff verschieden ist.

**35.** Zusamensetzung nach einem der Ansprüche 29 bis 34, die mindestens ein Oxidationsmittel enthält.

**36.** Zusammensetzung nach einem der Ansprüche 29 bis 35, die ein Lösungsmittel und/oder ein Verdickungsmittel enthält.

**37.** Vorrichtung mit mehrere Abteilungen, bei der eine erst Abteilung eine Farbmittelzusammensetzung enthält, die einen Disulfidfarbstoff enthält, wie er in einem der Ansprüche 1 bis 13 definiert ist, und eine zweite Abteilung ein Reduktionsmittel enthält, das befähigt ist, eine Disulfidbindung zu reduzieren.

**38.** Vorrichtung nach Anspruch 37, die eine dritte Abteilung aufweist, die ein Oxidationsmittel enthält.

**39.** Verwendung von Disulfidfarbstoffen, wie sie in einem der Ansprüche 1 bis 14 definiert sind, zum. Färben von menschlichen Keratinfasern und insbesondere Haaren.

**40.** Verwendung nach Anspruch 39 zur Verbesserung der Beständigkeit der Färbung.

**41.** Kationischer Disulfidfarbstoff, der mindestens eine quartäre Ammoniumgruppe enthält, der folgenden Formeln (I), (II), (III) oder (IV) :

$$A-(X)_p-C_{sat}-S-S-C_{sat}-(X)_p-A \qquad (I)$$

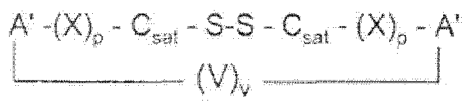

(II)

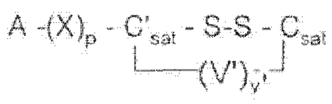

(III)

$$A-(X)_p-C_{sat}-S-S-C_{sat}-(X)_p-D \qquad (IV)$$

deren Salze, Isomere, Solvate, wie Hydrate,
in den Formeln:

- A und A', die gleich oder verschieden sind, bedeuten eine Gruppe, die mindestens ein kationisches oder nicht kationisches Chromophor enthält, wobei das Chromophor aus der Gruppe der Azofarbstoffe, Anthrachinone oder Hydrazone stammt;
- V und V', die gleich oder verschieden sind, bedeuten eine Brückengruppe;
- v und v', die gleich oder verschieden sind, bedeuten 0 oder 1 ;
- X bedeutet die folgende Sequenz:

$$-(T)_t-(Y)_y-(Z)_z-$$

wobei diese Sequenz in den Formeln (I), (II), (III) oder (IV) folgendermaßen gebunden ist;

- Csat (oder C'sat)-$(T)_t$-$(Y)_y$-$(Z)_z$-(A oder A'); wobei in der Sequenz:
T bedeutet eine oder mehrere Gruppen oder deren Kombinationen, die ausgewählt sind unter: -$SO_2$-, -O-, -S-, -N(R)-, -N+(R)(R)-CO-, wobei R ein Wasserstoffatom, eine $C_{1-4}$-Alkygruppe oder eine $C_{1-4}$-Hydroxyalkylgruppe bedeutet;
der koeffizient beträge 0 oder 1;
Y bedeutet:
- eine Gruppe, die ausgewählt ist unter -$(CH_2)_2$-$SO_2$- ; -$CH_2$-CHR-CO-NR'-, wobei R und R', die gleich oder verschieden ein Wasserstoffatom, eine $C_{1-4}$-Alkylgruppe bedeuten;
- eine Gruppe der Formel (a), (a') oder (a") :

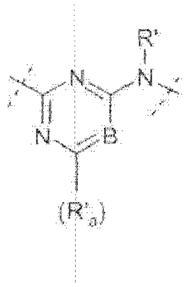 (a) 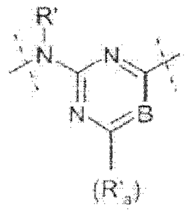 (a') 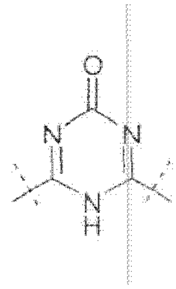 (a'')

in der Formel:

- B bedeutet -N-, $-CR_a$, wobei $R_a$ ein Wasserstoffatom,
- ein Halogenatom, das unter Chlor oder Fluor ausgewählt ist, eine Nitrogruppe, eine Pyridiniumgruppe , die gegebenenfalls substituiert ist, bedeutet;
- R' weist die oben angegebenen auf;
- $R'_a$ bedeutet:

  - ein Wasserstoffatom
  - ein Chloratom oder ein Fluoratom
  - eine Pyridiniumgruppe, die gegebenenfalls mit mindestens einer Gruppe $R_c$ substituiert ist, wobei es sich bei $R_c$ um eine $C_{1-4}$-Alkylgruppe, ein Halogenatom, eine Carboxygruppe -COOM (wobei M ein Wasserstoffatom, ein Alkalimetall, eine Ammoniumgruppe oder eine Ammoniumgruppe bedeutet, die mit einer oder meheren, gleichen oder verschiedenen, linearen oder verzweigten $C_{1-18}$-Alkylgruppen substituiert ist, die gegebenenfalls mindestens eine Hydroxygruppe tragen); eine Estergruppe $-COOR_d$, wobei es sich bei $R_d$ um eine $C_{1-4}$-Alkylgyruppe handelt; eine Amidgruppe $-CON(R_d)_2$, wobei die Gruppen $R_d$, die gleich oder verschieden sind, ein Wasserstoffatom oder eine $C_{1-4}$-Alkylgruppe bedeuten;
  - eine Hydroxygruppe
  - eine Amino-, Alkylamino- oder Dialkylaminogruppe; wobei die Alkylgruppen, die gleich oder verschieden sind, 1 bis 18 Kohlenstoffatome aufweisen und linear oder verzweigt vorliegen und gegebenenfalls durch ein Heteroatom unterbrochen sind, das unter N,O ausgewählt ist, und gegebenenfalls mit einer oder mehreren Hydroxygruppen substituiert sind, eine Gruppe NHNHCOR, wobei, R eine lineare oder verzweigte $C_{1-10}$-Alkylgruppe bedeutet;

- eine Gruppe der folgenden Formel (b):

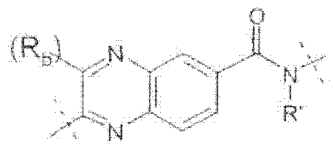 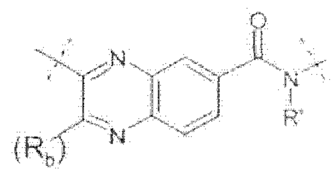

worin bedeuten:

- R' hat die oben angegebenen Bedeutungen
- $R_b$ bedeutet:

  - ein Chloratom
  - eine Amino-, Alklamino- oder Dialkylaminogruppe, wobei die Alkylgruppen, die gleich oder verschieden sind, 1 bis 18 Kohlenstoffatomen aufweisen und linear oder verzweigt vorliegen und gegebenenfalls durch ein Heteroatom unterbrochen sind, das unter N, O, S ausgewählt ist, und gegebenenfalls mit einer oder mehreren Hydroxygruppen substituiert sind,
  - einen gesättigten oder ungesättigten Stickstoffheterocyclus, der substituiert sein kann
  - eine Arylaminogruppe, bei der die Arylgruppe vorzugsweise 6 Kohlenstoffatome aufweist;

der Koeffizient p ist 1;
y bedeutet 0 oder 1;
Z bedeutet:

- $(CH_2)_m$-, wobei m eine ganze Zahl im Bereich von 1 bis 8 bedeutet
- $(CH_2CH_2O)_q$- oder -$(OCH_2CH_2)_q$-, worin q eine ganze Zahl im Bereich von 1 bis 15 bedeutet
- eine Aryl-, Alkylaryl- oder Arylalkylgruppe, wobei die Alkylgruppen 1 bis 4 Kohlenstoffatomen aufweisen und die Arylgruppe vorzugsweise C6 ist, wobei sie gegebenenffalls mit mindestens einer Gruppe $SO_3M$ substituiert ist, wobei M ein Wasserstoffatom, ein Alkalimetall, eine Ammoniumgruppe oder eine Ammoniumgruppe bedeutet, die mit einer oder mehreren, gleichen oder verschiedenem, linearen oder verzweigten $C_{1-18}$-Alkylgruppen substituiert ist, die gegebenenfalls mindestens eine Hydroxygruppe tragen
- z bedeutet 0 oder 1;
- $C_{sat}$, $C'_{sat}$, die gleich oder verschieden sind, bedeuten eine lineare oder verzweigte, gegebenenfalls substituierte, gegebenenfalls cyclische $C_{1-18}$-Alkylengruppe;
- D entspricht einer Gruppe, die unter den Gruppen Hydroxy, Hydroxyalkyl, Alkoxy, Carboxy, Carboxylat Amin, Alkylamino, Dialkylamino ausgewählt ist.

**42.** Farbstoff nach Anspruch 41, **dadurch gekennzeichnet, dass** die Gruppen A oder A', die gleich oder verschieden sind, ein Azochromophor enthalten.

**43.** Farbstoff nach einem der beiden vorhergehenden Ansprüchen, **dadurch gekennzeichnet, dass** der Disulfidfarbstoff so vorliegt, dass y und z Null bedeuten und T -N(R)- bedeutet.

**44.** Farbstoff nach einem der Ansprüche 41 bis 43, **dadurch gekennzeichnet, dass** W bedeutet: Imidazolium, Pyridinium, Benzimidazolium, Pyrazolium, Benzothiazolium, die gegebenenfalls mit einer oder mehreren, gleichen oder verschiedenen $C_{1-4}$-Alkylgruppen substituiert sind.

**45.** Farbstoff nach einem der Ansprüche 41 bis 44, **dadurch gekennzeichnet, dass** der Disulfidfarbstoff ausgewählt ist unter:

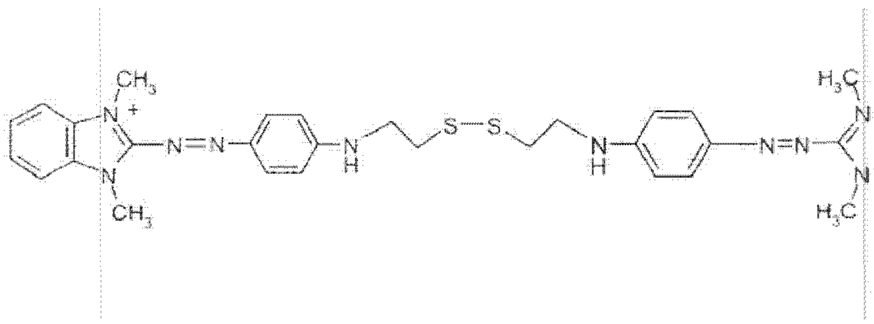

2M'

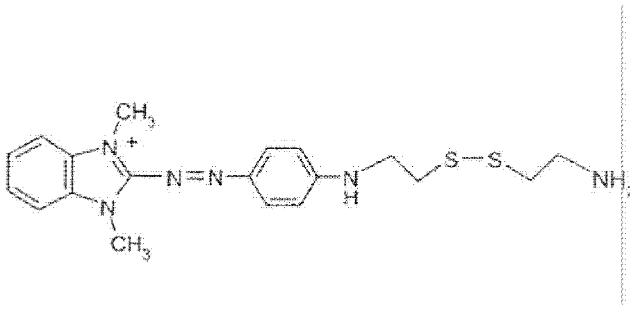

M'

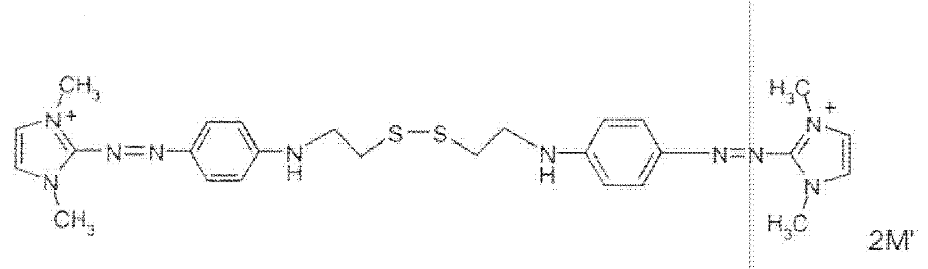

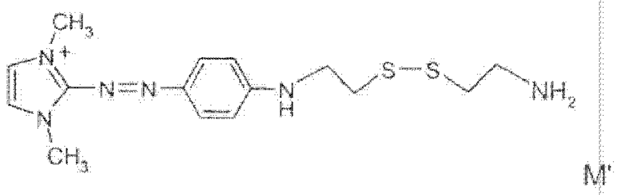

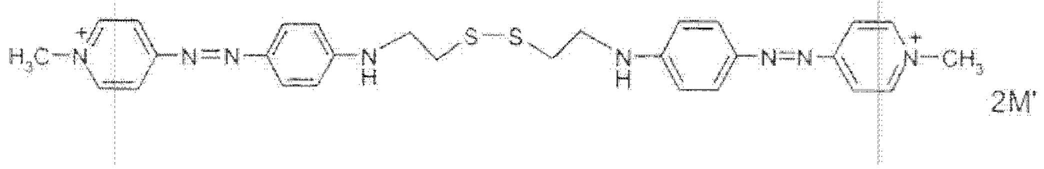

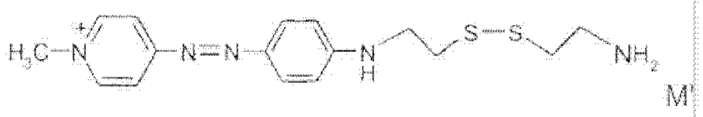

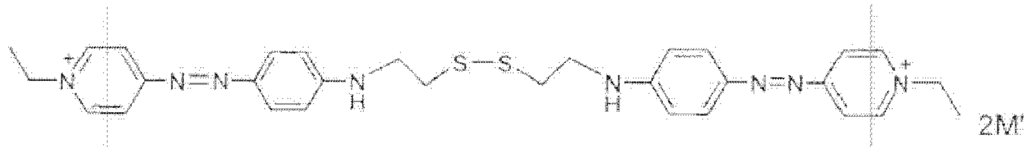

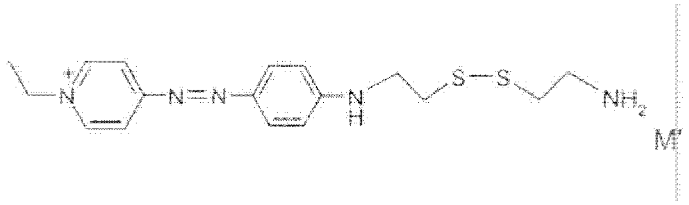

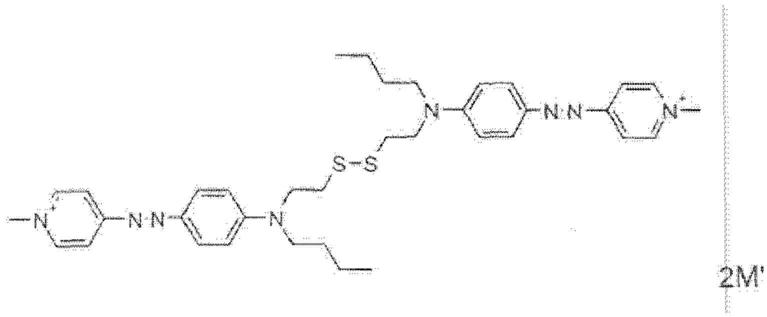

2M'

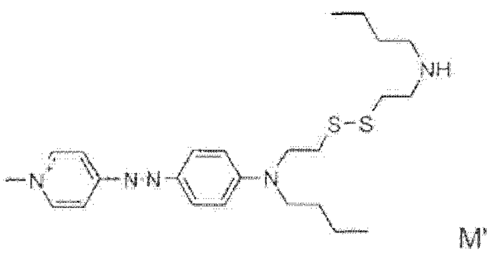

M'

**46.** Farbstoff der folgenden Formeln:

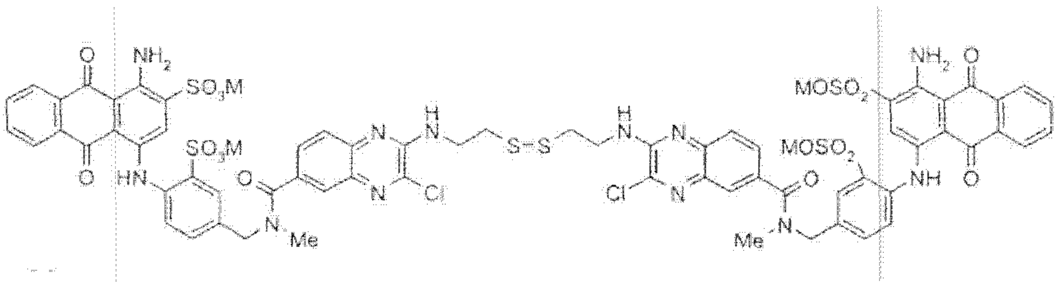

(17)

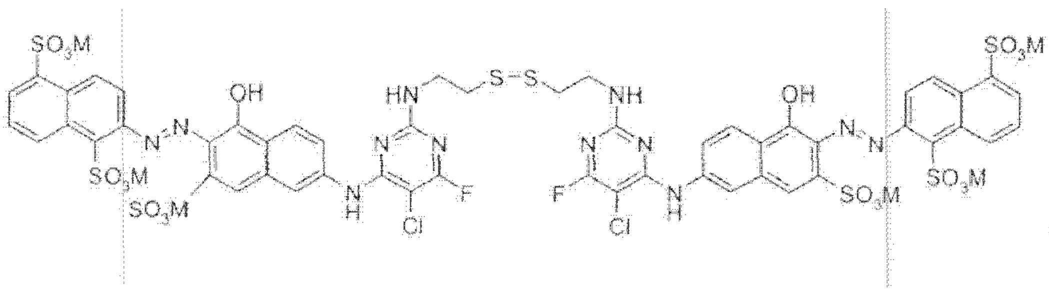

(18)

wobei M ein Wasserstoffatom, ein Alkalimetall, eine Ammoniumgruppe order eine Ammoniumgruppe bedeutet, die mit einer oder mehreren, gleichen oder verschiedenen, linearen oder verzweigten $C_{1-10}$-Alkylgruppen substituiert ist, die gegebenenfalls mindestens eine Hydroxygruppe tragen, sowie den folgenden, Verbindungen in saurer, basischer order neutralisierter Form.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 1156407
- WO 9515144 A
- WO 9501772 A
- EP 714954 A
- US 5888252 A
- EP 1133975 A
- WO 03029359 A
- EP 860636 A
- EP 318294 A
- EP 850636 A
- FR 2788433
- EP 920856 A
- WO 9948465 A
- FR 2757385
- EP 850637 A
- EP 918053 A
- WO 9744004 A
- FR 2570946
- FR 2285851
- DE 2538363
- FR 2189006
- FR 1560664
- FR 1540423
- FR 1567219
- FR 1516943
- FR 1221122
- DE 4220388
- DE 4137005
- WO 0166646 A
- US 5708151 A
- WO 515144 A
- GB 1195386 A
- US 3524842 A
- US 5879413 A
- EP 1062940 A
- EP 1133976 A
- GB 738585 A
- DE 2527638
- FR 2275462
- GB 197427645 A
- FR 2586913


### Littérature non-brevet citée dans la description


- The role of the vinylsulphonyl reactive dyes in prevention of wool damage. *J. Soc. Dyers Colourists,* Octobre 1991, vol. 107, 357-362
- **K. VENKATARAMAN.** The chemistry of synthetic dye. Academic press, 1952, vol. 1-7
- dyes and Dye intermediate. **Kirk Othmer.** Chemical technology. Wiley and sons, 1993
- ULLMANN's ENCYCLOPEDIA of Industrial chemistry. Wiley and sons
- *Histochem.,* 1978, vol. 61 (1), 48-52
- *Tsitologiya,* 1968, vol. 10 (3), 403-5
- *Zh. Obshch. Khim.,* 1970, vol. 40 (1), 195-202
- *Ann. Chim. (Rome),* 1975, vol. 65 (5-6), 305-14
- *Journal of the Chinese Chemical Society (Taipei),* 1998, vol. 45 (1), 209-211
- *Rev. Roum. Chim.,* 1988, vol. 33 (4), 377-83
- *Text. Res. J.,* 1984, vol. 54 (2), 105-7
- *Chim. Ind. (Milan),* 1974, vol. 56 (9), 600-3
- *Khim. Tekhnol.,* 1979, vol. 22 (5), 548-53
- *Ger. Monatsh. Chem.,* 1975, vol. 106 (3), 643-8
- *MRL Bull. Res. Dev.,* 1992, vol. 6 (2), 21-7
- *Lihua Jianyan, Huaxue Fence,* 1993, vol. 29 (4), 233-4
- *Dyes Pigm.,* 1992, vol. 19 (1), 69-79
- *Dyes Pigm.,* 1989, vol. 11 (3), 163-72
- Advanced Organic Chemistry